(19) Europäisches Patentamt
European Patent Office
Office européen des brevets

(11) EP 3 909 566 A1

(12) # EUROPEAN PATENT APPLICATION

(43) Date of publication:
**17.11.2021 Bulletin 2021/46**

(21) Application number: **21167699.4**

(22) Date of filing: **08.10.2016**

(51) Int Cl.:
*A61K 9/00* (2006.01) *A61K 38/00* (2006.01)
*A61K 47/02* (2006.01) *A61K 9/70* (2006.01)
*A61K 33/06* (2006.01) *A61K 33/30* (2006.01)
*A61K 38/29* (2006.01) *A61P 19/02* (2006.01)
*A61P 19/10* (2006.01) *A61K 47/10* (2017.01)
*A61K 47/40* (2006.01) *A61P 29/00* (2006.01)
*A61P 43/00* (2006.01) *C07K 14/635* (2006.01)
*A61P 19/00* (2006.01) *A61K 9/08* (2006.01)
*A61P 19/08* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**09.10.2015 US 201562239773 P**
**09.10.2015 US 201562239801 P**
**09.10.2015 US 201562239774 P**
**18.04.2016 US 201662324336 P**
**22.06.2016 US 201662353249 P**
**18.09.2016 US 201662396196 P**

(62) Document number(s) of the earlier application(s) in accordance with Art. 76 EPC:
**16854524.2 / 3 359 241**

(71) Applicants:
- **Radius Health, Inc.**
  **Waltham, MA 02451 (US)**
- **Kindeva Drug Delivery L.P.**
  **St. Paul, MN 55170 (US)**

(72) Inventors:
- **HATTERSLEY, Gary**
  **Stow, 01775 (US)**
- **HARRIS, Alan**
  **New York City, 10028 (US)**
- **SAEH, Jamal**
  **Belmont, 02478 (US)**
- **HAMED, Ehab**
  **Lexington, 02421 (US)**
- **BROWN, Kenneth**
  **St. Paul, 55133-3427 (US)**
- **DOHMEIER, Daniel**
  **St. Paul, 55133-3427 (US)**
- **ZHANG, Ying**
  **Woodbury, 55125 (US)**
- **DICK, Lisa**
  **St. Paul, 55133-3427 (US)**
- **MOSEMAN, Joan**
  **St. Paul, 55133-3427 (US)**

(74) Representative: **Carpmaels & Ransford LLP**
**One Southampton Row**
**London WC1B 5HA (GB)**

Remarks:
•This application was filed on 09-04-2021 as a divisional application to the application mentioned under INID code 62.
•Claims filed after the date of filing of the application (Rule 68(4) EPC).

(54) **FORMULATIONS OF PTHRP ANALOGUES, TRANSDERMAL PATCHES THEREOF, AND USES THEREOF**

(57) Disclosed are PTHrP analogue formulations for transdermal delivery of a therapeutically effective amount of a PTHrP analogue, as well as transdermal patches prepared using these formulations, methods of preparing the disclosed formulations and patches, and methods of using these formulations and patches to treat osteoporosis, osteopenia, osteoarthritis, and/or bone fracture, improve bone mineral density (BMD), improve trabecular bone score (TBS), and treat, prevent, and/or reduce bone fractures.

EP 3 909 566 A1

## Description

### PRIORITY CLAIM

The present application claims priority to U.S. Provisional Application No. 62/239,773, filed October 9, 2015, U.S. Provisional Application No. 62/239,774, filed October 9, 2015, U.S. Provisional Application No. 62/239,801, filed October 9, 2015, U.S. Provisional Application No. 62/324,336, filed April 18, 2016, U.S. Provisional Application No. 62/353,249, filed June 22, 2016, and U.S. Provisional Application No. 62/396,196, filed September 18, 2016, all of which are incorporated herein by reference in their entirety, including drawings.

### INTRODUCTION

Conventionally, osteoporosis is treated by administration of antiresorptive agents to suppress bone resorption. The most common of these treatments is oral or intravenous administration of bisphosphonates. However, an undesirable side effect of bisphosphonate administration is reduced bone formation (MacLean 2008). Anabolic agents provide an alternative to antiresorptives. The only anabolic agent currently available for treatment of osteoporosis is teriparatide (PTH (1-34), Forteo®), a recombinant form of parathyroid hormone (PTH) that acts by a mechanism that involves stimulating new bone formation (along with resorption) and reconstituting internal bone microarchitecture (Recker 2009; Dempster 2012; Ma 2011). The effects of teriparatide on bone mineral density (BMD) are superior to antiresorptive agents at the spine, but its effects at the hip are more modest, and often delayed until the second year of a two-year course of therapy (Leder 2014; Neer 2001).

Parathyroid hormone-related protein (PTHrP; UniProt Accession No. P12272) shares some homology with parathyroid hormone (PTH) at their N-terminal ends, and both proteins bind to the same G-protein coupled receptor, PTH receptor type-1 (PTH1R). Despite a common receptor, PTH primarily acts as an endocrine regulator of calcium homeostasis whereas PTHrP plays a fundamental paracrine role in the mediation of endochondral bone development (Kronenberg 2006). The differential effects of these proteins may be related not only to differential tissue expression, but also to distinct receptor binding properties (Pioszak 2009; Okazaki 2008; Dean 2008). Over the past several years, PTHrP and its secretory forms (PTHrP(1-36), PTHrP(38-94), and osteostatin), as well as analogues thereof, have been investigated as potential treatments for osteoporosis. Subcutaneous injection of PTHrP and its derivatives and analogues has been reported to be effective for treating osteoporosis and/or improving bone healing (Horwitz 2010; Horwitz 2006; Bostrom 2000; Augustine 2013).

Therefore, it is desirable to have an alternative delivery route that is both effective for treatment (e.g., a substantial bioequivalence of the subcutaneous delivery of PTHrP and/or derivatives and analogues thereof) and easy for administration to improve patients' satisfaction and compliance.

### SUMMARY OF THE INVENTION

Provided herein in certain embodiments are preparation formulations for use in transdermal delivery of PTHrP analogues such as abaloparatide comprising a PTHrP analogue (e.g., abaloparatide) and one or more excipients selected from the group consisting of $Zn2^+$salts (e.g., $ZnCl_2$, $Zn(OAc)_2$, $Zn_3(PO_4)_2$, ZnCitrate, ZnOxalate, etc., or combinations thereof), $Mg^{2+}$ salts (e.g., MgO, MgCitrate, $MgSO_4$, MgOrotate, MgLactate, $MgCO_3$, $MgCl_2$, $Mg(OAc)_2$, etc., or combinations thereof) $Ca^{2+}$ salts (e.g., CaSorbate, CaCitrate, CaAscorbate, $Ca_3(PO_a)_2$, $CaCl_2$, $CaCO_3$, $CaSO_4$, $Ca(OAc)_2$, etc., or combinations thereof), PEG (polyethylene glycol), PVP (polyvinylpyrrolidone), cyclodextrin (CD, e.g., 2-hydroxypropyl-β-cyclodextrin (HPβCD)), salts of carboxylic acids including fatty acids, NaCl and histidine and various combinations thereof. In certain embodiments, the preparation formulation further comprises water for injection, saline or phosphate buffered saline (PBS). In certain embodiments, the PTHrP analogue comprises, consists of, or consists essentially of abaloparatide ([$Glu^{22,25}$, $Leu^{23,28,31}$, $Aib^{29}$, $Lys^{26,30}$]hPTHrP(1-34)$NH_2$), which has the amino acid sequence set forth in SEQ ID NO:1. In certain embodiments, the PTHrP analogue is delivered by a transdermal patch comprising at least one microprojection (e.g., microneedle) prepared using the preparation formulation.

Provided herein in certain embodiments are patches for transdermal administration of a PTHrP analogue comprising one or more microprojections prepared using a preparation formulation as disclosed herein.

Provided herein in certain embodiments are methods of preparing a transdermal patch for administration of a PTHrP analogue comprising preparing at least a microprojection on a blank transdermal patch with a preparation formulation disclosed herein. In certain embodiments, the microprojections are microneedles.

Provided herein in certain embodiments are methods for treating osteoporosis, treating osteopenia, treating osteoarthritis, improving bone mineral density (BMD), improving trabecular bone score (TBS), and treating, preventing, and reducing bone fractures in a subject comprising transdermally administering a therapeutically effective amount of a PTHrP analogue via a transdermal patch comprising at least one microprojections prepared using PTHrP analog preparation formulation as disclosed herein. In some embodiments the osteoporosis being treated is postmenopausal osteoporosis. In some embodiments the osteoporosis being treated is glucocorticoid induced osteoporosis. In certain of these embodiments, the preparation formulation is administered via a transdermal patch as disclosed herein. The bone fractures being treated, prevented, or reduced and the bone with improved BMD or TBS may be vertebral or non-vertebral.

BRIEF DESCRIPTION OF DRAWINGS

Figures 1A-1C: Pharmacokinetic profile of various formulations of abaloparatide administered by transdermal versus subcutaneous routes. Figure 1A: One possible bioequivalence "window" of the abaloparatide-SC treatment, % scale on the vertical axis indicates the plasma abaloparatide concentration represented by % of its own maximum ($C_{max}$), i.e. 100 = $C_{max}$, hereinafter referred to as the "normalized plasma concentration."

Figure 1B: transdermal delivery in monkeys using a preparation formulation of abaloparatide comprising $ZnCl_2$, the vertical axis indicates normalized peptide plasma concentration.

Figure 1C: transdermal delivery using a preparation formulation of abaloparatide comprising PEG.

Figure 2: Pharmacokinetic profile of formulations of abaloparatide administered by transdermal versus subcutaneous routes (SC). The abaloparatide preparation formulation of the abaloparatide for transdermal delivery did not comprise $ZnCl_2$ or PEG, note the very quick $C_{max}$ of the transdermal delivery compare to SC and the increasing pulsatile nature of the delivery. Square: transdermal delivery (TD); and diamond: the abaloparatide-SC treatment. Administration in healthy postmenopausal women, % scale on vertical axis indicates the normalized plasma concentration of abaloparatide represented by % of $C_{max}$ for each group.

Figure 3: Pharmacokinetic profile of formulations of abaloparatide containing $ZnCl_2$ administered by transdermal (TD) versus subcutaneous (SC) routes, longitude of median plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined. Note the more prolonged release afforded by the $ZnCl_2$ addition.

Figure 4: Pharmacokinetic profile of formulations of abaloparatide administered by transdermal versus subcutaneous routes, median plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 5: Pharmacokinetic profile of formulations of abaloparatide administered by transdermal versus subcutaneous routes, longitude of mean plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 6: Pharmacokinetic profile of formulations of abaloparatide administered by transdermal versus subcutaneous routes, mean plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 7: Pharmacokinetic profile of formulations of abaloparatide administered by transdermal versus subcutaneous routes, longitude of median of dose normalized plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 8: Pharmacokinetic profile of formulations of abaloparatide administered by transdermal versus subcutaneous routes, median of dose normalized plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 9: Pharmacokinetic profile of formulations of abaloparatide administered by transdermal versus subcutaneous routes, longitude of mean of dose normalized plasma abaloparatide concentration v. time post administration. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 10: Pharmacokinetic profile of formulations of abaloparatide administered by transdermal versus subcutaneous routes, mean of dose normalized plasma abaloparatide concentration v. time post administration in healthy postmenopausal women. Transdermal administration was to the abdomen with a formulation including $ZnCl_2$. Historic TD used abaloparatide formulations in PBS buffer (no $ZnCl_2$) and represents all 150 $\mu$g TD studies combined.

Figure 11: Comparison of $C_{max}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 12: Comparison of $C_{max}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 13: Comparison of $AUC_{last}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 14: Comparison of $AUC_{last}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 15: Comparison of $AUC_{inf}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 16: Comparison of $AUC_{inf}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 17: Comparison of $C_{max}$/D ($C_{max}$ per dosage) of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD combined.

Figure 18: Comparison of $C_{max}$/D ($C_{max}$ per dosage) of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 19: Comparison of CL/F of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 20: Comparison of CL/F of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 21: Comparison of HL_Lambda_z of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 22: Comparison of HL_Lambda_z of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 23: Comparison of $T_{max}$ of formulations of abaloparatide administered by transdermal versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 24: Comparison of $T_{max}$ of formulations of abaloparatide administered by transdermal (abdomen) versus subcutaneous routes in healthy postmenopausal women. Transdermal administration was to the abdomen with a formulation including $ZnCl_2$. Historic TD used abaloparatide formulations in PBS buffer (no $ZnCl_2$) and represents all 150 μg TD studies combined.

Figure 25: Pharmacokinetic profile of formulations of abaloparatide administered by transdermal route (abdomen) in a selected patient versus historical subcutaneous data in healthy postmenopausal women. TD used abaloparatide formulations PBS buffer with no Zn salt added.

Figure 26: Percent BMD changes from baseline at lumber spine of the subjects treated with abaloparatide via transdermal delivery or SC injection in postmenopausal women with osteoporosis. Transdermal delivery used abaloparatide formulations with PBS buffer (no Zn salt).

Figure 27: Percent BMD changes from baseline at total hip of the subjects treated with abaloparatide via transdermal delivery or SC injection in postmenopausal women with osteoporosis. Transdermal delivery used abaloparatide formulations with PBS buffer (no Zn salt).

Figure 28: Local tolerance data of the subjects treated with abaloparatide via transdermal delivery in postmenopausal women with osteoporosis. Transdermal delivery used abaloparatide formulations with PBS buffer (no Zn salt).

Figure 29: Pharmacokinetic profile of formulations of abaloparatide administered by transdermal delivery used abaloparatide formulations with PBS only (no Zn salt) in healthy postmenopausal women (diamond) versus subcutaneous (square) routes. Note the very rapid and pulsatile release in the transdermal delivery compared to the SC administration.

Figures 30A-30B: PK/PD relationship of formulations of abaloparatide administered by transdermal and by subcutaneous routes all in postmenopausal women with osteoporosis. Figure 30A: $C_{max}$ v. BMD improvement (%) for formulations of abaloparatide administered by transdermal and by subcutaneous routes. Figure 30B: AUC v. BMD improvement (%) for formulations of abaloparatide administered by transdermal and by subcutaneous routes.

Figure 31: Comparison of pK curves from a sc combination cohort (80 $\mu$g), 1st generation transdermal (200 $\mu$g abaloparatide + PBS buffer only) and 2nd generation transdermal (200 $\mu$g abaloparatide + $ZnCl_2$). Values are the geometric means.

Figure 32: Comparison of pK curves of selected individual patients being treated with the 2nd generation transdermal (200 $\mu$g abaloparatide plus $ZnCl_2$) and compared to a reference set of sc treated patients. Values are the geometric means.

Figure 33: Concentration-time graph after administration of patch formulated with PEG 3350 NF and 100 $\mu$g of abaloparatide to abdomen of healthy post-menopausal women (N=12) where plasma concentration time points are the arithmetic means.

Figure 34: Concentration-time graph after administration of patch formulated with PEG 3350 NF and 150 $\mu$g of abaloparatide to abdomen of healthy post-menopausal women (N=13) where plasma concentration time points are the arithmetic means.

Figure 35: Concentration-time graph after administration of patch formulated with PEG 3350 NF and 200 $\mu$g of abaloparatide to abdomen of healthy post-menopausal women (N=14) where plasma concentration time points are the arithmetic means.

Figure 36: Concentration-time graph after administration of patch formulated with PEG 3350 NF and $ZnCl_2$ and 100 $\mu$g of abaloparatide to abdomen of healthy post-menopausal women (N=8) where plasma concentration time points are the arithmetic means.

Figure 37: Concentration-time graph after administration of patch formulated with PEG 3350 NF and $ZnCl_2$ and 150 $\mu$g of abaloparatide to abdomen of healthy post-menopausal women (N=7) where plasma concentration time points are the arithmetic means.

Figure 38: Concentration-time graph after administration of patch formulated with PEG 3350 NF and $ZnCl_2$ and 200 $\mu$g of abaloparatide to abdomen of healthy post-menopausal women (N=8) where plasma concentration time points are the arithmetic means.

Figure 39: Transdermal administration utilized patches having different microneedle lengths with abaloparatide formulations (no Zn) in monkeys. Square: subcutaneous delivery; triangle: transdermal delivery (short microneedles - 250 $\mu$m); diamond: transdermal delivery (regular microneedles - 500 $\mu$m); and star: transdermal delivery (long microneedles - 700 $\mu$m).

Figure 40: Comparison of Cmax (peak plasma concentration (pg/mL)) of various formulations of the PTHrP analogue of SEQ ID NO:1 in monkeys.

Figure 41: Comparison of AUC (area under the curve) of various formulations of the PTHrP analogue of SEQ ID NO:1 administered by transdermal versus subcutaneous route.

Figure 42: Comparison of plasma concentration (pg/mL) of formulation of abaloparatide (ABL, SEQ ID NO:1) administered by subcutaneous route (SC) or transdermal administration (TD), wherein the transdermal administration utilized a transdermal patch coated using different transdermal formulations in monkeys.

Table 1. Modeling of TD-A32 data for bioequivalence for the abaloparatide-SC treatment.

Table 2. PK Results of Abaloparatide 100 $\mu$g TD, Abaloparatide 150 $\mu$g TD, Abaloparatide 200 $\mu$g TD, Abaloparatide 80 $\mu$g SC, and Historical 150 $\mu$g TD.

Table 3. Comparisons of Abaloparatide 100 $\mu$g TD, Abaloparatide 150 $\mu$g TD, Abaloparatide 200 $\mu$g TD, Abaloparatide 80 $\mu$g SC, and Historical 150 $\mu$g TD to UnoPen 80 $\mu$g SC, respectively.

Table 4. Comparisons of Abaloparatide 100 $\mu$g TD, Abaloparatide 150 $\mu$g TD, Abaloparatide 200 $\mu$g TD, and

Abaloparatide 80 μg SC to Historical 150 μg TD, respectively.

Table 5. Design for a Phase 2 study of transdermal delivery of abaloparatide using a transdermal patch prepared by a first general abaloparatide formulation (with PBS).

Table 6. $C_{max}$, AUC, and BMD improvement of a Phase 2 study of transdermal delivery of abaloparatide (TD-50 mcg, TD-100 mcg, and TD-150 mcg) using a transdermal patch prepared by a first general abaloparatide formulation (with PBS), and subcutaneous delivery of abaloparatide (SC-80 mcg).

## DETAILED DESCRIPTION

Abaloparatide is a synthetic PTHrP analogue having the sequence set forth in SEQ ID NO:1. Abaloparatide has shown potent anabolic activity with decreased bone resorption, less calcium-mobilizing potential, and improved room temperature stability (Obaidi 2010). Studies performed in animals have demonstrated marked bone anabolic activity following administration of abaloparatide, with complete reversal of bone loss in ovariectomy-induced osteopenic rats and monkeys (Doyle 2013a; Doyle 2013b; Hattersley 2013). Abaloparatide has been developed as a promising anabolic agent for the treatment of osteopenia (e.g., glucocorticoid-induced osteopenia), osteoporosis (e.g. glucocorticoid-induced osteoporosis), and/or osteoarthritis.

Subcutaneous administration of 80 μg abaloparatide (hereinafter the "abaloparatide-SC treatment") has been shown to significantly reduce incidences of new vertebral, non-vertebral, major osteoporotic and clinical fractures versus a placebo. Subcutaneous abaloparatide administration has also been shown to improve bone mineral density (BMD) and/or trabecular bone score (TBS) of treated subjects at the lumbar spine, total hip, and femoral neck. In certain embodiments, the abaloparatide-SC treatment comprises subcutaneous administration of an aqueous formulation comprising abaloparatide (about 2 mg/mL) in an acetate buffer, with a pH of about 4.5 to about 5.6, or about 5.1. Optionally, the aqueous formulation further comprises phenol (about 5 mg/mL). In certain examples of these embodiments, the acetate buffer comprises tri-hydrate sodium acetate (about 5 mg/mL) with pH (e.g., about 4.5 to about 5.6, or about 5.1) adjusted with acetic acid.

Transdermal administration of abaloparatide is an attractive alternative to subcutaneous administration due to its less invasive nature. However, transdermal administration may have different PK profile compared to subcutaneous administration. It has been found that AUC of transdermal abaloparatide administration and subcutaneous abaloparatide administration (80 μg) had a linear relationship with the achieved BMD changes from the baseline after 6 months of treatments (Figure 30B). It is thus desired to develop transdermal abaloparatide administrations that are substantially bioequivalent to the subcutaneous abaloparatide administration to benefit from both the preferred osteoanabolic profile of subcutaneous abaloparatide administration and the convenience of transdermal administration.

As disclosed herein, it has been unexpectedly found that transdermal abaloparatide administration using a patch prepared with a preparation formulation comprising abaloparatide and one or more excipients selected from the group consisting of $ZnCl_2$, PEG, and histidine produces a substantial bioequivalence to subcutaneous administration in a monkey model. Furthermore, preliminary clinical studies indicate that $ZnCl_2$ in a transdermal formulation blunts the pulsatile nature compared with a non-$ZnCl_2$ containing formulation and pushes the curve into one resembling the sc curve. This is a notable achievement in that achieving bioequivalence to a sc dose of abaloparatide would indicate exceptional fracture prevention effects as has been reported for 80 μg sc administration.

Based on these findings, provided herein are PTHrP analogue preparation formulations, transdermal patches prepared using these preparation formulations, transdermal patches comprising these preparation formulations, methods of making these patches, and methods of using the disclosed preparation formulations and patches to administer PTHrP analogues in a transdermal manner and to treat osteoporosis, osteopenia, and osteoarthritis, improve BMD, improve TBS, and treat, prevent, and reduce bone fractures in a subject. In certain embodiments of the preparation formulations, transdermal patches, and methods provided herein, the PTHrP analogue is abaloparatide consisting of the amino acid sequence set forth in SEQ ID NO:1, or an abaloparatide derivative comprising or consisting essentially of the amino acid sequence set forth in SEQ ID NO:1. In certain embodiments of the preparation formulations, transdermal patches, and methods provided herein, the transdermal delivery of the PTHrP analogue produces substantial bioequivalence or bioequivalence to a subcutaneous delivery of abaloparatide at the dosage of about 20 μg to about 200 μg, about 40 μg to about 120 μg, about 60 μg to about 100 μg, about 70 μg to about 90 μg, or about 80 μg. In certain embodiments of the preparation formulations, transdermal patches, and methods provided herein, the transdermal delivery of the PTHrP is a substantial bioequivalence or bioequivalence of the abaloparatide-SC treatment.

As used herein, two treatments of an active agent are bioequivalent to one another if the 90% confidence interval of the ratio of area under the curve (AUC) and/or the peak serum concentration of the active agent ($C_{max}$) falls completely within the range 80-125%. See, e.g., Figure 1A showing a bioequivalence window of the abaloparatide-SC treatment in Chinese Cynomolgus monkeys. Serum abaloparatide concentrations are presented as percentage of $C_{max}$.

As used herein, the term "substantially," "substantial" or "essentially" means nearly completely or completely. In particular, a normal bioequivalent range means a compound in a particular formulation for transdermal delivery within the 80%-125% (for the mean in the 90% confidence interval (CI)) of AUC (0-t, 0-inf) and $C_{max}$ of the reference compound in a reference formulation. In some embodiments the reference formulation is the SC delivery of 80 μg abaloparatide formulated as described herein. In certain embodiments, the transdermal delivery of a compound or more particularly abaloparatide falls within a substantially bioequivalent range wherein said range is 70%-136%, or 65%-141%, or 60%-147%, or 50%-158% (for the mean in the 90% confidence interval (CI)) of AUC (0-t, 0-inf) and $C_{max}$ of the reference compound in a reference formulation.

As used herein, the term "about" or "approximately" means a range of ±0-10%, or ±0-5% or the numeral following the term.

As used herein, the term "transdermal delivery" refers to a delivery of an active agent through the stratum corneum to make contact with the intradermal space without significant pain upon penetration. Because the stratum corneum has no nerves, it may be pierced without stimulating nerves. The terms "transdermal" and "intradermal" are used interchangeably herein. The stratum corneum is composed primarily of several layers of dead skin cells and is not vascularized. Thus, the stratum corneum often poses a formidable barrier to the transdermal delivery of an active agent, especially for charged macromolecules such as peptides. Unlike active agents delivered by subcutaneous injection, which almost provides a complete entrance into the blood stream, many factors (and barriers) can affect the pharmacokinetics of drugs delivered by a transdermal route. For example, the site of application, the thickness, integrity, and hydration condition of the skin, the thickness and density of the adipose tissue under the skin of the application site, the size of the drug molecules, the pH condition and permeability of the membrane of the transdermal device, etc., all may affect the bioavailability of drugs delivered transdermally. In certain embodiments, transdermal delivery involves penetrating the skin through the stratum corneum into the dermis to a depth of up to about 700 μm, or up to about 600 μm, or up to about 500 μm, or up to about 400 μm, or up to about 300 μm, or up to about 250 μm, or up to about 150 μm. In some embodiments, the average needle depth of penetration is approximately 800 μm, or about 700 μm, or about 600 μm, or about 500 μm, or about 400 μm, or about 300 μm, or about 250 μm, or about 150 μm.

### *I. Preparation formulation for transdermal delivery*

Provided herein in certain embodiments are preparation formulations for transdermal delivery of a therapeutically active substance, e.g., a bioactive peptide, a bioactive peptide contains at least 10 amino acids, e.g., a PTHrP analogue (e.g., comprising, consisting essentially of, or consisting of abaloparatide). In certain embodiments, the transdermal delivery produces a substantial bioequivalence or bioequivalence to subcutaneous delivery of the PTHrP analogue (e.g., at 80 μg). These formulations comprise a PTHrP analogue and one or more excipients selected from the group consisting of salts of $Zn^{2+}$, salts of $Mg^{2+}$, salts of $Ca^{2+}$, salts of histidine, salts of carboxylic acids (e.g., fatty acids), NaCl, PEG, PVP, cyclodextrin (CD, e.g., 2-hydroxypropyl-β-cyclodextrin (HPβCD)), and combinations thereof. In certain embodiments the salt of $Zn^{2+}$ is selected from the group consisting of $Zn(OAc)_2$, $ZnCl_2$, $Zn_3(PO_4)_2$, zinc citrate (ZnCitrate), zinc oxalate (ZnOxalate), and combinations thereof, the salt of $Ca^{2+}$ is selected from the group consisting of calcium sorbate (CaSorbate), calcium citrate (CaCitrate), calcium ascorbate (CaAscorbate), $Ca_3(PO_a)_2$, $CaCl_2$, $CaCO_3$, $CaSO_4$, $Ca(OAc)_2$ and combinations thereof, the salt of $Mg^{2+}$ is selected from the group consisting of MgO, magnesium citrate (MgCitrate), $MgSO_4$, magnesium orotate (MgOrotate), magnesium lactate (MgLactate), $MgCO_3$, $MgCl_2$, $Mg(OAc)_2$, and combinations thereof. In certain embodiments, two or more salts of $Mg^{2+}$, $Zn^{2+}$ and/or $Ca^{2+}$ as described herein are combined together for purposes of a transdermal formulation. In certain embodiments, the preparation formulation further comprises water for injection, brine or PBS. In certain embodiments, the PTHrP analogue comprises, consists of, or consists essentially of abaloparatide. In certain embodiments, the transdermal delivery of the PTHrP analogue produces substantial bioequivalence or bioequivalence to a subcutaneous delivery of abaloparatide at the dosage of about 20 μg to about 200 μg, about 40 μg to about 120 μg, about 60 μg to about 100 μg, about 70 μg to about 90 μg, or about 80 μg. In certain embodiments, the transdermal delivery of the PTHrP is a substantial bioequivalence or bioequivalence of the abaloparatide-SC treatment. In certain embodiments, the PTHrP analogue is delivered by a transdermal patch comprising at least one microprojection (e.g., microneedle) prepared using the preparation formulation.

In certain embodiments, the preparation formulation comprises PEG with a molecular weight of about 3,000 to about 3,700, about 2,000 to about 5,000, about 3,00 to about 3,500, or about 1,000 to about 6,000. A concentration by weight of PEG to the total amount of the preparation formulation is about 0.01% to about 50%, about 5% to about 50%, about 5% to about 45%, about 5% to about 40%, about 5% to about 35%, about 5% to about 30%, about 5% to about 25%, about 5% to about 20%, about 5% to about 15%, about 10% to about 50%, about 10% to about 45%, about 10% to about 40%, about 10% to about 35%, about 10% to about 30%, about 10% to about 25%, about 10% to about 20%, about 10% to about 15%, about 15% to about 50%, about 15% to about 45%, about 15% to about 40%, about 15% to about 35%, about 15% to about 30%, about 15% to about 25%, about 15% to about 20%, about 13% to about 17%, about 14% to about 16%, or about 14.9%.

In certain embodiments, the preparation formulation comprises water and a $Zn^{2+}$ salt (also referred to as Zn salt, salt of Zn, or salt of $Zn^{2+}$), in some embodiments said coating formulation comprises $ZnCl_2$, or $Zn(OAc)_2$, or $Zn_3(PO_4)_2$,

or ZnCitrate or ZnOxalate or combinations thereof. The concentration of $Zn^{2+}$ salt (e.g., $ZnCl_2$) in the preparation formulation, for example, by weight to the total amount of the preparation formulation is about 0.01% to about 30%, 0. 1% to about 30%, 0.3% to about 30%, about 0.5% to about 30%, about 0.8% to about 30%, about 1% to about 30%, about 1.5% to about 30%, about 2% to about 30%, about 5% to about 30%, 10% to about 30%, 15% to about 30%, about 20% to about 30%, about 25% to about 30%, about 0.01% to about 20%, 0. 1% to about 20%, 0.3% to about 20%, about 0.5% to about 20%, about 0.8% to about 20%, about 1% to about 20%, about 1.5% to about 20%, about 2% to about 20%, about 5% to about 20%, 10% to about 20%, 15% to about 20%, about 0.01% to about 10%, 0. 1% to about 10%, 0.3% to about 10%, about 0.5% to about 10%, about 0.8% to about 10%, about 1% to about 10%, about 1.5% to about 10%, about 2% to about 10%, about 5% to about 10%, about 0.01% to about 5%, 0. 1% to about 5%, 0.3% to about 5%, about 0.5% to about 5%, about 0.8% to about 5%, about 1% to about 5%, about 1.5% to about 5%, about 2% to about 5%, about 0.01% to about 3%, 0. 1% to about 3%, 0.3% to about 3%, about 0.5% to about 3%, about 0.8% to about 3%, about 1% to about 3%, about 1.5% to about 3%, about 2% to about 3%, about 0.01% to about 30%, 0. 1% to about 30%, 0.3% to about 30%, about 0.5% to about 30%, about 0.8% to about 2%, about 1% to about 2%, about 1.5% to about 2%, about 0.01% to about 1%, 0. 1% to about 1%, 0.3% to about 1%, about 0.5% to about 1%, about 0.8% to about 1%, or about 0.8%. In certain embodiments, the coating formulation for the described ranges comprises a $Ca^{2+}$ salt wherein said $Ca^{2+}$ salt can include CaSorbate, CaCitrate, CaAscorbate, $Ca_3(PO_a)_2$, $CaCl_2$, $CaCO_3$, $CaSO_4$, $Ca(OAc)_2$ or combinations thereof. $Ca^{2+}$ salt is also referred to as Ca salt, salt of Ca, or salt of $Ca^{2+}$. In some embodiments the coating solution comprises a $Mg^{2+}$ salt wherein said $Mg^{2+}$ salt can include MgO, MgCitrate, $MgSO_4$, MgOrotate, MgLactate, $MgCO_3$, $MgCl_2$, $Mg(OAc)_2$, or combinations thereof. $Mg^{2+}$ salt is also referred to as Mg salt, salt of Mg, or salt of $Mg^{2+}$.

In some embodiments of this invention, a formulated patch ready to package and use (the initial coating solution dried to remove water) is provided wherein said formulated patch comprises a $Zn^{2+}$ salt. In certain embodiments the $Zn^{2+}$ salt is $Zn(OAc)_2$, in some embodiments the $Zn^{2+}$ salt is $ZnCl_2$, in certain embodiments it is $Zn_3(PO_4)_2$, in some embodiments it is ZnCitrate and in certain embodiments it is ZnOxalate or combinations thereof. In certain embodiments, the formulated patch is made by coating with the coating solution in one or multiple coating iterations and then drying said patch or allowing said patch to dry to a fairly constant weight and then characterizing said patch as weight by percent of the salt, the metal including its counterions. In certain embodiments, the coated patch, dried and ready to use comprises from 1.0 to 20% Zn salt by weight. In certain embodiments, said coated patch comprises from 1.5% to 15% Zn salt by weight. In some embodiments, the coated and dried patch comprises 1.5% to 10% Zn salt by weight, or 1.8% - 8.5%, or 1.9% to 5.9%, or about 1.9% to 8.5%, or about 2.0% to about 8%, or 5% to 8% by weight or is between 1.7% to 2.25%, or between 5 to 7%, or about 5.8%, or about 1.9%.

In some embodiments of this invention, a formulated patch ready to package and use (the initial coating solution dried to remove water) is provided wherein said formulated patch comprises a $Ca^{2+}$ salt. In some embodiments the $Ca^{2+}$ salt is CaSorbate, CaCitrate, CaAscorbate, $Ca_3(PO_4)_2$, $CaCl_2$, $CaCO_3$, $CaSO_4$, $Ca(OAc)_2$ or combinations thereof. In certain embodiments, the formulated patch is made by coating with the coating solution in one or multiple coating iterations and then drying said patch or allowing said patch to dry to a fairly constant weight and then characterizing said patch as weight by percent of the salt, the metal including its counterions. In certain embodiments, the coated patch, dried and ready to use comprises from 1.0 to 20% Ca salt by weight. In certain embodiments, said coated patch comprises from 1.5% to 15% Ca salt by weight. In some embodiments, the coated and dried patch comprises 1.5% to 10% Ca salt by weight, or 1.8% - 8.5%, or 1.9% to 5.9%, or about 1.9% to 8.5%, or about 2.0% to about 8%, or 5% to 8% by weight.

In some embodiments of this invention, a formulated patch ready to package and use (the initial coating solution dried to remove water) is provided wherein said formulated patch comprises a $Mg^{2+}$ salt. In some embodiments the $Mg^{2+}$ salt is MgO, MgCitrate, $MgSO_4$, MgOrotate, MgLactate, $MgCO_3$, $MgCl_2$, $Mg(OAc)_2$, or combinations thereof. In certain embodiments, the formulated patch is made by coating with the coating solution in one or multiple coating iterations and then drying said patch or allowing said patch to dry to a fairly constant weight and then characterizing said patch as weight by percent of the salt, the metal including its counterions. In certain embodiments, the coated patch, dried and ready to use comprises from 0.5 to 15% Mg salt by weight. In certain embodiments, said coated patch comprises from 1.0% to 10% Mg salt by weight. In some embodiments, the coated and dried patch comprises 1.5% to 10% Mg salt by weight, or 1.8% - 8.5%, or 1.9% to 5.9%, or about 1.9% to 8.5%, or about 2.0% to about 8%, or 5% to 8% by weight.

In certain embodiments, the formulated patch comprises two or more of the $Zn^{2+}$, $Ca^{2+}$ and/or $Mg^{2+}$ salts described immediately above.

Example of coating solution

| Component | Function | Weight % |
|---|---|---|
| Abaloparatide | API | 35.78 |
| Zinc Chloride, USP ($ZnCl_2$) | Complexing Agent | 2.22 |
| Sterile Water for Injection, USP | Solvent* | 62.00 |
| Total | | 100 |

Example of formulation on patch ready to use (after drying)

| Component | Function | Weight % |
|---|---|---|
| Abaloparatide | API | 94.16 |
| Zinc Chloride, USP ($ZnCl_2$) | Complexing Agent | 5.84 |
| Total | | 100 |

Example of coating solution

| Component | Function | Weight |
|---|---|---|
| Abaloparatide | API | 45.11 |
| Zinc Chloride, USP ($ZnCl_2$) | Complexing Agent | 0.89 |
| Sterile Water for Injection, USP | Solvent* | 54.00 |
| Total | | 100 |

Example of formulation on patch ready to use (after drying)

| Component | Function | Weight % |
|---|---|---|
| Abaloparatide | API | 98.07 |
| Zinc Chloride, USP ($ZnCl_2$) | Complexing Agent | 1.93 |
| Total | | 100 |

Example of coating solution

| Component | Function | Weight % |
|---|---|---|
| Abaloparatide | API | 30%-65% |
| Zinc Chloride, USP ($ZnCl_2$) | Complexing Agent | 0.5%-8.5% |
| Sterile Water for Injection, USP | Solvent* | 30%-65% |
| Total | | 100 |

Example of formulation on patch ready to use (after drying)

| Component | Function | Weight % |
|---|---|---|
| Abaloparatide | API | 85%-99% |
| Zinc Chloride, USP ($ZnCl_2$) | Complexing Agent | 0.5%-10% |
| Total | | 100 |

Example of formulation on patch ready to use (after drying)

| Component | Function | Weight % |
|---|---|---|
| Abaloparatide | API | 85%-99% |
| Zinc Chloride, USP ($ZnCl_2$) | Complexing Agent | 4.5%-8.5% |
| Total | | 100 |

Example of formulation on patch ready to use (after drying)

| Component | Function | Weight % |
|---|---|---|
| Abaloparatide | API | 85%-99% |
| Zinc Chloride, USP ($ZnCl_2$) | Complexing Agent | 5%-8% |
| Total | | 100 |

Example of formulation on patch ready to use (after drying)

| Component | Function | Weight % |
|---|---|---|
| Abaloparatide | API | 85%-99% |
| Zinc Acetate USP $Zn(OAc)_2$ | Complexing Agent | 0.5%-10% |
| Total | | 100 |

Example of formulation on patch ready to use (after drying)

| Component | Function | Weight % |
|---|---|---|
| Abaloparatide | API | 85%-99% |
| Zinc Acetate USP $Zn(OAc)_2$ | Complexing Agent | 1.5%-7.5% |
| Total | | 100 |

Example of formulation on patch ready to use (after drying)

| Component | Function | Weight % |
|---|---|---|
| Abaloparatide | API | 85%-99% |
| Zinc Phosphate USP $Zn_3(PO_4)_2$ | Complexing Agent | 0.5%-10% |
| Total | | 100 |

Example of formulation on patch ready to use (after drying)

| Component | Function | Weight % |
|---|---|---|
| Abaloparatide | API | 85%-99% |
| $Ca^{2+}$ salt | Complexing Agent | 0.5%-10% |
| Total | | 100 |

Example of formulation on patch ready to use (after drying)

| Component | Function | Weight % |
|---|---|---|
| Abaloparatide | API | 85%-99% |
| $Ca^{2+}$ salt | Complexing Agent | 0.5%-10% |
| Total | | 100 |

Example of coating solution

| Component | Function | Weight % |
|---|---|---|
| Abaloparatide | API | 40.5% |
| Polyethylene Glycol 3350 NF | Complexing Agent | 14.5% |
| Sterile Water for Injection, USP | Solvent* | 45.00 |
| Total | | 100 |

Example of formulation on patch ready to use (after drying)

| Component | Function | Weight % |
|---|---|---|
| Abaloparatide | API | 73.64% |
| Polyethylene Glycol 3350 NF | Complexing Agent | 26.36% |
| Total | | 100 |

Example of coating solution

| Component | Function | Weight % |
|---|---|---|
| Abaloparatide | API | 34.84% |
| Polyethylene Glycol 3350 NF | Complexing Agent | 12.47% |
| Zinc Chloride, USP | Complexing Agent | 0.69% |
| Sterile Water for Injection, USP | Solvent | 52% |
| Total | | 100 |

Example of formulation on patch ready to use (after drying)

| Component | Function | Weight % |
|---|---|---|
| Abaloparatide | API | 72.58% |
| Polyethylene Glycol 3350 NF | Complexing Agent | 25.98% |
| Zinc Chloride, USP | Complexing Agent | 1.44% |
| Total | | 100 |

Example of coating formulation for PEG, PVP, CD and histidine

| No. | Excipient | MW | Concentration in the transdermal formulation for coating | Molar ratio excipient/ PTHrP analogue |
|---|---|---|---|---|
| 1. | PEG | 3,000-3,700 | About 2.8 - about 15% (by weight) | 0.09-0.52 |
| 2. | PVP | 7,000-11,000 | About 600 mg/mL | Not determined |
| 4. | CD | Approx. 1410 | About 1.3 - about 13.3% (by weight) | About 0.08- about 1.1 |
| 6. | Histidine (e.g., monohydrochloride monohydrate) | 209.6 | About 2 - bout 5 % (by weight) | About 1.4 - about 3.4 |

Example of formulation on patch ready to use (after drying)

| No. | Excipient | MW | Concentration in the transdermal formulation for coating | %weight in final ready to use patch |
|---|---|---|---|---|
| 1. | PEG | 3,000-3,700 | About 2.8 - about 15% (by weight) | About 6% - about 30% |
| 2. | PVP | 7,000-11,000 | About 600 mg/mL | Not determined |
| 4. | CD | Approx. 1410 | About 1.3 - about 13.3% (by weight) | About 3%- about 30% |

(continued)

| No. | Excipient | MW | Concentration in the transdermal formulation for coating | %weight in final ready to use patch |
|---|---|---|---|---|
| 6. | Histidine (e.g., monohydrochloride monohydrate) | 209.6 | About 2 - bout 5 % (by weight) | About 5% - about 13% |

Coating solution, doses and sites of administration for clinical and planned clinical studies

| Cohort | Formulation | Dose | Anatomical Site |
|---|---|---|---|
| 1 | $ZnCl_2$ | 100<br>150 | Abdomen |
| | | 200 | |
| 2 | PEG | 100<br>150<br>200 | Abdomen |
| 3 | $ZnCl_2$/PEG | 100<br>150<br>200 | Abdomen |
| 4 Period 1 | $ZnCl_2$ | 200 | Thigh |
| 4 Period 2 | $ZnCl_2$/PEG | 200 | Thigh |
| 4 Period 3 | $ZnCl_2$ | 2 X 150 | Abdomen |
| 5 Period 1 | $ZnCl_2$ | 200 | Thigh |
| 5 Period 2 | $Zn(OAc)_2$ | 200 | Thigh |
| 5 Period 3 | $ZnCl_2$ | 200 | Thigh |

In certain embodiments, the preparation formulation comprises histidine (e.g., monohydrochloride monohydrate). The concentration of histidine (by weight to the total amount of the preparation formulation) is about 1% to about 15%, about 1% to about 10%, about 1% to about 5%, about 3% to about 15%, about 3% to about 10%, about 3% to about 5%, about 5% to about 15%, about 5% to about 10%, about 3%, about 5%, or about 10%.

In certain embodiments, the preparation formulation comprises two or three excipients selected from the group consisting of PEG, $ZnCl_2$, and histidine, wherein the concentration of each excipient is the same as disclosed herein.

In certain embodiments, the preparation formulation comprises two excipients selected from the group consisting of PEG, $ZnCl_2$, and histidine, e.g., a combination of PEG and $ZnCl_2$, a combination of histidine and PEG, and a combination of histidine and $ZnCl_2$.

In certain embodiments, the preparation formulation comprises a combination of PEG, $ZnCl_2$, and histidine.

In certain embodiments, the preparation formulation comprises the PTHrP analogue at a concentration of about 5% to about 15%, about 12.5% to about 20%, about 15% to about 60%, about 40% to about 48%, about 43% to about 48%, about 40% to about 46%, about 40% to about 52%, about 46% to about 48%, about 46% to about 52%, about 50% to about 62%, about 52% to about 60%, or about 54% to about 58% by weight.

In certain embodiments, the preparation formulation has a viscosity at 25°C of greater than about 500 centipoise, greater than about 550 centipoise, greater than about 600 centipoise, greater than about 700 centipoise, greater than about 800 centipoise, greater than about 900 centipoise, greater than about 1,000 centipoise, greater than about 1,500 centipoise, greater than about 2,000 centipoise, greater than about 10,000 centipoise, about 500 to about 5,000 centipoise, about 500 to about 2,000 centipoise, or about 500 to about 1,000 centipoise, about 550 to about 5,000 centipoise, about 550 to about 2,000 centipoise, or about 550 to about 1,000 centipoise.

In certain embodiments, the preparation formulations disclosed herein further comprise a bioactive peptide or protein. In certain embodiments, the preparation formulations disclosed herein comprise an antibody.

In certain embodiments, the preparation formulations disclosed herein further comprise excipients selected from the group consisting of $ZnCl_2$, $Zn(OAc)_2$, $Zn_3(PO_4)_2$, ZnCitrate, ZnOxalate, MgO, MgCitrate, $MgSO_4$, MgOrotate, MgLactate, $MgCO_3$ CaSorbate, CaCitrate, CaAscorbate, $Ca_3(PO_a)_2$, $CaCl_2$, $CaCO_3$, $CaSO_4$, and $Ca(OAc)_2$. In certain embodiments, the preparation formulations disclosed herein comprise excipients selected from $ZnCl_2$, $Zn(OAc)_2$ and combinations thereof. In certain embodiments, the preparation formulations disclosed herein have a molar ratio of the excipient or excipients to the therapeutically active substance selected from the ranges of about 0.1 to about 2.0, about 0.2 to about 1.5, or about 0.25 to about 1.0.

### *II. Transdermal patches*

Provided herein in certain embodiments are transdermal patches for administration of a PTHrP analogue comprising one or more microprojections prepared using a preparation formulation of the PTHrP analogue as disclosed herein, wherein transdermal delivery of the PTHrP analogue using the patch produces substantial bioequivalence or bioequivalence to subcutaneous delivery of the PTHrP. In certain embodiments, the PTHrP analogue comprises, consists of, or consists essentially of abaloparatide. In certain embodiments, the transdermal delivery of the PTHrP analogue produces substantial bioequivalence or bioequivalence to a subcutaneous delivery of abaloparatide at the dosage of about 20 μg to about 250 μg, about 20 μg to about 200 μg, about 40 μg to about 120 μg, about 60 μg to about 100 μg, about 70 μg to about 90 μg, about 80 μg, about 100 μg, about 150 μg, or about 200 μg. In certain embodiments, transdermal delivery of the PTHrP analogue produces substantial bioequivalence or bioequivalence to the abaloparatide-SC treatment.

In certain embodiments, the transdermal patches provided herein are designed for passive diffusion of the PTHrP analogue as provided herein. In other embodiments, the transdermal patches are designed for active delivery of the PTHrP analogue using an external energy source.

In certain embodiments, the preparation formulation of the PTHrP analogue is used to prepare one or more microprojections on a transdermal patch, resulting in the transdermal patches comprising the PTHrP analogue. For example, at least part of the one or more microprojections on the transdermal patch comprises the PTHrP analogue. In certain embodiments, at least part of the one or more microprojections on the transdermal patch further comprises one or more excipients selected from the group consisting of PEG, $ZnCl_2$ and histidine.

In certain embodiments, the amount of each excipient per patch is about 1 μg to about 300 μg, about 10 μg to about 300 μg, about 100 μg to about 300 μg, about 200 μg to about 300 μg, about 1 μg to about 200 μg, about 10 μg to about 200 μg, about 100 μg to about 200 μg, about 150 μg to about 200 μg, about 1 μg to about 150 μg, about 10 μg to about 150 μg, about 100 μg to about 150 μg, about 1 μg to about 100 μg, about 10 μg to about 100 μg, about 50 μg to about 100 μg, about 1 μg to about 50 μg, about 10 μg to about 50 μg, about 20 μg to about 50 μg, about 1 μg to about 20 μg, about 10 μg to about 20 μg, about 15 μg to about 20 μg, about 1 μg, about 5 μg, about 10 μg, about 20 μg, about 30 μg, about 40 μg, about 50 μg, about 60 μg, about 70 μg, about 80 μg, about 90 μg, about 100 μg, about 110 μg, about 120 μg, about 130 μg, about 140 μg, about 150 μg, about 160 μg, about 170 μg, about 180 μg, about 190 μg, about 200 μg, about 210 μg, about 220 μg, about 230 μg, about 240 μg, about 250 μg, about 260 μg, about 270 μg, about 280 μg, about 290 μg, or about 300 μg.

In certain embodiments, the amount of the PTHrP analogue (e.g., abaloparatide) per patch is 1 μg to about 300 μg, about 10 μg to about 300 μg, about 100 μg to about 300 μg, about 200 μg to about 300 μg, about 1 μg to about 200 μg, about 10 μg to about 200 μg, about 50 μg to about 200 μg, about 80 μg to about 200 μg, about 100 μg to about 200 μg, about 150 μg to about 200 μg, about 1 μg to about 150 μg, about 10 μg to about 150 μg, about 50 μg to about 150 μg, about 80 μg to about 150 μg, about 100 μg to about 150 μg, about 1 μg to about 100 μg, about 10 μg to about 100 μg, about 50 μg to about 100 μg, about 1 μg to about 50 μg, about 10 μg to about 50 μg, about 20 μg to about 50 μg, about 1 μg to about 20 μg, about 10 μg to about 20 μg, about 15 μg to about 20 μg, about 1 μg, about 5 μg, about 10 μg, about 20 μg, about 30 μg, about 40 μg, about 50 μg, about 60 μg, about 70 μg, about 80 μg, about 90 μg, about 100 μg, about 110 μg, about 120 μg, about 130 μg, about 140 μg, about 150 μg, about 160 μg, about 170 μg, about 180 μg, about 190 μg, about 200 μg, about 210 μg, about 220 μg, about 230 μg, about 240 μg, about 250 μg, about 260 μg, about 270 μg, about 280 μg, about 290 μg, or about 300 μg.

In certain embodiments, the transdermal patches provided herein comprise a plurality of these microprojections. The term "microprojection" as used herein refers to a piercing element of any shape or size on a transdermal patch that is capable of piercing the stratum corneum of the skin. These small piercing elements can have various materials, shapes and dimensions. In certain embodiments, one or more of the microprojections on the disclosed transdermal patches are microneedles. The term "microneedle" as used herein refers to a microprojection comprising a base and a tip, wherein the tip has a smaller diameter, width, perimeter or circumference than the base. A transdermal patch comprising one or more microneedles may also be referred to as a "transdermal microneedle patch" or a "microneedle transdermal patch."

A microneedle in the transdermal patches provided herein may have any size, shape, or design commonly used in the art. In certain embodiments, the microneedles have their greatest diameter, width, perimeter, or circumference at the base. In certain embodiment, the microneedles have a tapered design, meaning that the microneedle from base to tip reflects a relatively constant narrowing over the length. In certain embodiments, the ratio of the diameter, width, perimeter, or circumference at the base of the microneedle to the diameter, width, perimeter, or circumference at the tip of the microneedle is greater than 2. In other embodiments, the ratio is greater than 4 or greater than 6. In certain embodiments, the microneedles have a generally circular perimeter about the axis that is broader at the base than the tip. In certain embodiments, the microneedles are pyramidal in shape, with an approximately rectangular base that tapers to an apex, wherein said apex is approximately rectangular. In certain embodiments, the microneedles are pyramidal in shape, with a square base that tapers to an apex wherein said apex is approximately square. In certain embodiments, the microneedles are pyramidal in shape with a rectangular or square base and a shape that is not readily characterized as rectangular or square at the top.

The microprojection may have various length, e.g., about 30 μm to about 1,500 μm, about 50 μm to about 1,500 μm, about 100 μm to about 1,500 μm, about 250 μm to about 1,500 μm, about 500 μm to about 1,500 μm, about 600 μm to about 1,500 μm, about 750 μm to about 1,500 μm, about 800 μm to about 1,500 μm, about 1,000 μm to about 1,500 μm, about 30 μm to about 1,00 μm, about 50 μm to about 1,500 μm, about 30 μm to about 1,000 μm, about 50 μm to about 1,000 μm, about 750 μm to about 1,200 μm, about 800 μm to about 1,200 μm, about 100 μm to about 1,000 μm, about 250 μm to about 1,000 μm, about 500 μm to about 1,000 μm, about 600 μm to about 1,000 μm, about 750 μm to about 1,000 μm, about 800 μm to about 1,000 μm, about 30 μm to about 750 μm, about 50 μm to about 750 μm, about 100 μm to about 750 μm, about 250 μm to about 750μm, about 500 μm to about 750 μm, about 600 μm to about 750 μm, about 600 μm to about 800 μm, about 30 μm to about 600 μm, about 50 μm to about 600 μm, about 100 μm to about 600 μm, about 250 μm to about 600 μm, about 500 μm to about 600 μm, about 30 μm to about 500 μm, about 50 μm to about 500 μm, about 100 μm to about 500 μm, about 250 μm to about 500 μm, about 30 μm to about 250 μm, about 50 μm to about 250 μm, about 100 μm to about 250 μm, about 30 μm, about 50 μm, about 100 μm, about 150 μm, about 200 μm, about 250 μm, about 300 μm, about 500 μm, about 750 μm, or about 1,500 μm.

Microprojections on the transdermal patches provided herein can be made from any suitable material, including for example carbon, polymers, metals, or a combination thereof, to achieve a desirable flexural modulus. In some embodiments, the microprojection has a flexural modulus of greater than 1,000 MPa, greater than 2,000 MPa, greater than 3,000 MPa, or between 3,000 MPa and 15,000 MPa. As used herein, "ISO 178" refers to ISO test standards for determination of flexural properties of plastics.

In certain embodiments, the transdermal patches provided herein comprise a first backing layer on which the microprojections are arrayed. In these embodiments, the microprojections may be affixed to or integral with the first backing layer. In certain embodiments, the microprojections are made from the same material as the first backing layer. For example, the microprojections may be formed by etching or punching from the first backing layer. In certain embodiments, the microprojections are made by an injection molding process. In other embodiments, the microprojections may be made of a different material than the first backing layer. In certain of these embodiments, the microprojections are affixed to the first backing layer via an adhesive. In certain of these embodiments, the microprojections are detachable from the first backing layer and/or the second backing layer.

In certain embodiments, the transdermal patches provided herein further comprise a second backing layer on which the first backing layer is affixed. The second backing layer may be flexible or inflexible.

In certain embodiments, the transdermal patches provided herein comprise an adhesive material to facilitate the patch staying in place on a subject's skin before and/or during transdermal administration of the PTHrP analogue. In certain of these embodiments, the adhesive material is comprised on the first and/or second backing layer(s).

In certain embodiments of the transdermal patches provided herein, the vertical axis of the one or more microprojections extends at an angle of at least 45 degrees or at least 60 degrees from the first and/or second backing layer(s). In some embodiments, the microprojections are perpendicular to the first and/or second backing layer(s).

In certain embodiments of the transdermal patches provided herein, the patches have a microprojection density of about 20 to about 2,000 microprojections per $cm^2$, about 50 to about 2,000 microprojections per $cm^2$, about 100 to about 2,000 microprojections per $cm^2$, about 250 to about 2,000 microprojections per $cm^2$, about 500 to about 2,000 microprojections per $cm^2$, about 750 to about 2,000 microprojections per $cm^2$, about 1,000 to about 2,000 microprojections per $cm^2$, about 1,500 to about 2,000 microprojections per $cm^2$, about 300 to about 500 microprojections per $cm^2$. In certain embodiments, the patches comprise about 50 to about 4,000 microprojections, about 100 to about 4,000 microprojections, about 250 to about 4,000 microprojections, the patches comprise about 1,400 to about 4,000 microprojections, about 1,600 to about 4,000 microprojections, about 2,000 to about 4,000 microprojections, about 3,000 to about 4,000 microprojections, about 3,500 to about 4,000 microprojections, the patches comprise about 50 to about 3,500 microprojections, about 100 to about 3,500 microprojections, about 250 to about 3,500 microprojections, about 1,400 to about 3,500 microprojections, about 1,600 to about 3,500 microprojections, about 2,000 to about 3,500 microprojections, about 3,000 to about 3,500 microprojections, about 50 to about 3,000 microprojections, about 100 to about 3,000 microprojections, about 250 to about 3,000 microprojections, about 1,400 to about 3,000 microprojections, about 1,600 to about 3,000 microprojections, about 2,000 to about 3,000 microprojections, about 50 to about 600 microprojections, about 100 to about 500 microprojections, about 250 to about 400 microprojections, about 300 to about 375 microprojections, about 300 to about 750 microprojections, about 366 microprojections, about 316 microprojections, or about 320 microprojections.

In certain embodiments, the transdermal patch of a PTHrP analogue comprises at least one microprojection at least partially coated with a of the PTHrP analogue (hereinafter the "coated microprojection").

The term "coated" as used herein with regard to an individual microprojection means that the microprojection comprises a PTHrP analogue composition on at least part of its surface. In certain embodiments, the microprojection comprises a PTHrP analogue composition on about 1% to about 100%, 1% to about 80%, about 1% to about 50%, about 2% to about 40%, about 5% to about 35%, 10% to about 30%, 15% to about 20%, or about 30% to about 50% of its total surface area. In certain embodiments, the microprojection comprises a PTHrP analogue composition on about 30% to about 50% of the top of the microprojection (as used herein, "top" means the end of the microprojection which would contact the skin).

The term "coated" as used herein with regard to a plurality of microprojections means that two or more of the microprojections in the plurality are coated as the term is used above with regard to an individual microprojection. In certain embodiments, more than 10%, more than 20%, more than 30%, more than 40%, more than 50%, more than 60%, more than 70%, more than 80%, or more than 90% of the microprojections in a plurality of microinjections are coated. In certain embodiments, about 1% to about 100%, 1% to about 80%, about 1% to about 50%, about 2% to about 40%, about 5% to about 35%, 10% to about 30%, 15% to about 20%, or about 30% to about 50% of the total microprojection surface area in the plurality of microinjections are coated. "Microprojection surface area" as used herein refers to the combined surface area of all microprojections on a single transdermal patch.

In certain embodiments, a transdermal patch comprising one or more coated microprojections may further comprise a PTHrP analogue composition on at least part of its surface of the first backing layer. For example, the transdermal patch comprises a PTHrP analogue composition on more than about 1% to about 100%, 1% to about 80%, about 1% to about 50%, about 2% to about 40%, about 5% to about 35%, 10% to about 30%, 15% to about 20%, or about 30% to about 50% of its total surface area of the first backing layer.

In certain embodiments, the transdermal patch of a PTHrP analogue comprises at least one microprojection comprising a plurality of layers arranged roughly parallel (e.g. at least about 80% parallel, at least about 90% parallel, or at least about 95% parallel) to the first backing layer, and at least one layer of the plurality of layers comprises the PTHrP analogue (hereinafter the "active agent layer") (hereinafter the "layered microprojection").

In certain embodiments, the first backing layer of the transdermal patch comprising at least one layered microprojection further comprises an active agent layer. In certain embodiments, the active agent layer forms a tip of the microprojection which can penetrate through the stratum corneum for the transdermal delivery. The tip of the microprojection may adopt any shape as disclosed supra regarding the shapes of microprojections (e.g., pyramid, square, rectangle, etc.).

In other embodiments, the microprojection provided herein comprises a reservoir that is in fluid communication with the skin when applied to the subject. The reservoir is loaded with the PTHrP analogue to be administered. The reservoir may be an inner space of the microprojection in fluid communication with the skin when applied, e.g., microprojections comprising a hollow portion. In certain embodiments, the hollow portion may have a side-opening.

In certain embodiments, the transdermal patches disclosed herein comprise a plurality of microprojections wherein at least one microprojection (e.g., microneedles)in the array is covered at least in part by a coating, said coating comprising a therapeutically active substance and one or more excipients selected from the group consisting of $Zn^{2+}$ salts, $Mg^{2+}$ salts, $Ca^{2+}$ salts, polyethylene glycols and hydroxypropyl beta-cyclodextrins. In certain embodiments, the therapeutically active substance comprises a bioactive peptide or protein. In certain embodiments, the therapeutically active substance comprises an antibody. In certain embodiments, the transdermal patches disclosed herein comprise a bioactive peptide or protein, e.g., bioactive peptide containing at least 10 amino acids, such as abaloparatide as set forth in SEQ ID NO: 1. In certain embodiments, the one or more excipients the transdermal patches disclosed herein has are selected from the group consisting of $ZnCl_2$, $Zn(OAc)_2$, $Zn_3(PO_4)_2$, ZnCitrate, ZnOxalate, MgO, MgCitrate, $MgSO_4$, MgOrotate, MgLactate, $MgCO_3$ CaSorbate, CaCitrate, CaAscorbate, $Ca_3(PO_a)_2$, $CaCl_2$, $CaCO_3$, $CaSO_4$, and $Ca(OAc)_2$. In certain embodiments, the one or more excipients the transdermal patches disclosed herein has are selected from the group consisting of $ZnCl_2$ and $Zn(OAc)_2$ and combinations thereof. In certain embodiments, the transdermal patches disclosed herein have a molar ratio of the excipient or excipients to the therapeutically active substance selected from the range of about 0.1 to about 2.0, about 0.2 to about 1.5, or about 0.25 to about 1.0. In certain embodiments, the transdermal patches disclosed herein have abaloparatide in an amount of between 90-110 μg, 140-160 μg, 185-220 μg, 225-275 μg or about 100 μg, about 150 μg, about 200 μg or about 250 μg.

Transdermal patches may also be prepared as disclosed in US Application Nos. 14/361,787, 14/361,802, 13/452,412, 13/791,170, 13/791,360, which are incorporated herein by reference in their entirety, including drawings.

### *III. Method of preparing transdermal patches*

Provided herein in certain embodiments are methods of preparing a transdermal patch for administration of a PTHrP analogue as disclosed herein, comprising preparing at least one microprojection on a transdermal patch with a preparation formulation disclosed herein. In certain embodiments the microprojections are microneedles. In certain embodiments, the PTHrP analogue comprises, consists of, or consists essentially of abaloparatide. In certain embodiments, the transdermal delivery of the PTHrP analogue produces substantial bioequivalence or bioequivalence to a subcutaneous delivery of abaloparatide at the dosage of about 20 μg to about 200 μg, about 40 μg to about 120 μg, about 60 μg to about 100 μg, about 70 μg to about 90 μg, or about 80 μg. In certain embodiments,, and transdermal delivery of the PTHrP analogue produces substantial bioequivalence or bioequivalence to the abaloparatide-SC treatment.

In certain embodiments, the preparation methods provided herein comprise contacting one or more microprojections on a blank (i.e., previously free of the PTHrP analogue) transdermal patch with the preparation formulations provided herein. In certain of these embodiments, the microprojections are coated with the preparation formulation by dipping a blank transdermal patch into the preparation formulation, then removing the patch and allowing it to dry.

In certain of these embodiments, the microprojections are layered microprojections and are prepared by casting or depositing the layers onto the first and/or second backing layer, then removing the patch and allowing it to dry.

In certain embodiments, accelerated drying conditions are applied to the transdermal patch, including for example circulating air flow, desiccants, vacuum, and/or heat.

In certain embodiments, transdermal delivery of the PTHrP analogue by applying the transdermal patches to a subject produces substantial bioequivalence or bioequivalence to subcutaneous delivery of the PTHrP analogue.

### *IV. Method of treatments*

Provided herein in certain embodiments are methods of treating osteoporosis, osteopenia, and osteoarthritis, improving bone mineral density (BMD), improving trabecular bone score (TBS), and/or treating, preventing, and/or reducing bone fractures in a subject comprising transdermally administering a therapeutically effective amount of a PTHrP analogue comprised in a preparation formulation provided herein. In certain embodiments, transdermal administration is accomplished using a transdermal patch as provided herein, wherein the patch comprises at least one microprojection prepared using a preparation formulation provided herein. The bone fractures being treated, prevented, or reduced and/or the bone with improved BMD and/or TBS may be vertebral or non-vertebral, clinical and major osteoporotic fractures. In certain embodiments, the PTHrP analogue comprises, consists of, or consists essentially of abaloparatide. In certain embodiments, the transdermal delivery of the PTHrP analogue produces substantial bioequivalence or bioequivalence to a subcutaneous delivery of abaloparatide at the dosage of about 20 μg to about 200 μg, about 40 μg to about 120 μg, about 60 μg to about 100 μg, about 70 μg to about 90 μg, or about 80 μg. In certain embodiments, transdermal delivery of the PTHrP analogue produces substantial bioequivalence or bioequivalence to the abaloparatide-SC treatment.

The term "subject" as used herein refers to a mammalian subject. Examples of suitable subjects include, without limitation, subjects with one or more conditions selected from the group consisting of osteopenia, glucocorticoid-induced osteopenia, osteoporosis, glucocorticoid-induced osteoporosis, osteoarthritis, bone fractures, and high cortical porosity (e.g., subjects with diabetes, especially type II diabetes), female mammals, male mammals, dogs, cats, humans, men, women, women with osteoporosis, postmenopausal women, postmenopausal women with osteoporosis, mammals with high cortical porosity, and men and women with high cortical porosity.

As used herein, the term "cortical porosity" means the fraction of the cortical bone volume that is not occupied by the bone. The cortical porosity may be measured by Digital X-ray radiogrammetry (DXR) or other methods to provide an estimation of the local intensity minima ("holes") in the cortical bone regions using a recursive (climbing) algorithm starting from the outer region (Dhainaut 2013). A combined porosity measure is derived from the area percentage of holes found in the cortical part relative to the entire cortical area, by averaging over the involved bones and scaled to reflect a volumetric ratio rather than the projected area. A "high cortical porosity" means a porosity of about 10% higher, about 15% higher, about 20% higher, about 50% higher, about 100% higher, or about 150% higher than that of healthy subjects from the same age group as controls. For example, the subject may have a cortical porosity of about 0.01256, which the control group has a cortical porosity of about 0.01093 (Dhainaut 2013). Subjects with type II diabetes may have a cortical porosity up to twice that of controls (Oei 2013). Subject may have normal BMD or slightly lower BMD while have high cortical porosity.

The term "therapeutically effective amount" as used herein refers to an amount of a PTHrP formulation as provided herein that is sufficient to elicit the required or desired therapeutic and/or prophylactic response, as the particular treatment context may require. Examples of therapeutically effective amounts of a PTHrP analogue include, without limitation, 20 μg, 40 μg, 60 μg, 80 μg, 100 μg, 120 μg, 140 μg, 160 μg 180 μg, 200 μg, 220 μg, 240 μg, 260 μg, 280

μg, or 300 μg. Other examples of therapeutically effective amounts of a PTHrP analogue may also include, without limitation, between 1 μg/kg and 50 μg/kg, 5 μg/kg and 50 μg/kg, 1 μg/kg and 40 μg/kg, 1 μg/kg and 30 μg/kg, 1 μg/kg and 20 μg/kg, 1 μg/kg and 10 μg/kg, 1 μg/kg and 5 μg/kg, 5μg/kg and 40 μg/kg, 5 μg/kg and 30 μg/kg, 5 μg/kg and 20 μg/kg, 5 μg/kg and 10 μg/kg, 10 μg/kg and 50 μg/kg, 10 μg/kg and 40 μg/kg, 10 μg/kg and 30 μg/kg, 10 μg/kg and 20 μg/kg, 10 μg/kg and 15 μg/kg, 20 μg/kg and 50 μg/kg, 20 μg/kg and 40 μg/kg, or 20 μg/kg and 30 μg/kg, of body weight of the subject.

Examples of bones which may exhibit improved BMD and/or TBS following the transdermal delivery of the PTHrP analogue include, without limitation, the lumbar spine, total hip, wrist, femur, cortical bone of the femur (femoral diaphysis), and/or femoral neck in the subject.

The transdermal delivery of the PTHrP analogue may be administered at any treatment interval necessary for therapeutic effectiveness. In certain embodiments, the transdermal delivery of the PTHrP analogue is administered on a daily basis. In other embodiments, the transdermal delivery of the PTHrP analogue may be administered every other day, every 3rd day, every 4th day, every 5th day, once a week, or once or twice a month. One of ordinary skill in the art will recognize that the treatment interval may vary over the course of treatment. For example, the transdermal delivery of the PTHrP analogue may be administered more frequently at the start of treatment, then less frequently over time as one or more therapeutic benchmarks are achieved. Alternatively, the transdermal delivery of the PTHrP analogue may be administered less frequently at the start of treatment, with the treatment interval decreasing over time.

In those embodiments of the methods provided herein wherein the transdermal delivery of the PTHrP analogue is administered using a transdermal patch provided herein. The transdermal patch may be placed in contact with the skin for any period of time necessary to achieve satisfactory analogue delivery. In certain embodiments, the transdermal patch may remain in contact with the skin for about 1 second to about 30 seconds, about 1 second to about 1 minute, about 15 second to about 30 seconds, about 15 second to about 1 minute, about 30 second to about 1 minute, about 1 minute to about 5 minutes, about 5 minutes to about 10 minutes, about 10 minutes to about 15 minutes, about 15 minutes to about 20 minutes, about 20 minutes to about 25 minutes, about 25 minutes to about 30 minutes, at least 5 minutes, at least 10 minutes, at least 15 minutes, at least 20 minutes, at least 25 minutes, at least 30 minutes, at least 35 minutes, at least 40 minutes, at least 45 minutes, at least 50 minutes, at least 55 minutes, at least 60 minutes, at least 75 minutes, at least 90 minutes, or at least 120 minutes. In certain embodiments, two or more transdermal patches may be placed in contact with the skin in a sequential manner to achieve the desired contact duration. In certain embodiments, more than one transdermal patch may be applied simultaneously.

In certain embodiments of the methods provided herein, the treatment is carried out for a set period determined in advance. In other embodiments, the treatment is carried out until one or more therapeutic benchmarks are reached. Examples of a suitable timeframe for treatment include, without limitation, 6 weeks, 12 weeks, 3 months, 24 weeks, 6 months, 48 weeks, 12 months, 18 months, and 24 months. In certain embodiments, the treatment is carried out via once a day administration of a transdermal patch for 18 months.

In certain embodiments, a subject administered a PTHrP analogue via transdermal delivery as provided herein achieves a $C_{max}$ which is about 80% to about 125% of the $C_{max}$ achieved by a subcutaneous administration of the same active agent. In certain embodiments, the PTHrP analogue comprises, consists of, or consists essentially of abaloparatide, and the transdermal delivery of the PTHrP analogue achieves a $C_{max}$ which is about 80% to about 125% of the $C_{max}$ achieved by the abaloparatide-SC treatment.

In certain embodiments, a subject administered a PTHrP analogue via transdermal delivery as provided herein achieves an AUC which is about 80% to about 125% of the AUC achieved by a subcutaneous administration of a corresponding formulation. In certain embodiments, the PTHrP analogue comprises, consists of, or consists essentially of abaloparatide, and the transdermal delivery of the PTHrP analogue achieves a AUC which is about 80% to about 125% of the AUC achieved by the abaloparatide-SC treatment.

In certain embodiments, the PTHrP analogue formulation is administrated in combination with one or more additional osteoporosis therapies, including for example alendronate therapy. In these embodiments, the additional osteoporosis therapy may be administered before, during, or after the treatment with the PTHrP analogue formulation.

In certain embodiments of the methods disclosed herein, said administration comprises application of a force to the transdermal patch sufficient to drive one or more of the microprojections through the stratum corneum of the patient. In certain embodiments of the methods disclosed herein, the site of administration is the abdomen or the thigh.

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention. It will be understood that many variations can be made in the procedures herein described while still remaining within the bounds of the present invention. It is the intention of the inventors that such variations are included within the scope of the invention.

## Examples

### Example 1: Pharmacokinetics of abaloparatide delivered via transdermal patch prepared using preparation formulations comprising PEG or $ZnCl_2$ in non-human primates:

Microneedle transdermal patches coated with various formulations of abaloparatide were provided ready for use and stored refrigerated at 2-8°C. At least one hour prior to use, the transdermal patches in individual pouches were placed at room temperature.

Eight female non-naive Chinese Cynomolgus monkeys (2-4 kg at time of dosing) were included in the study. The same eight animals were used to test each formulation, with a three day washout period between tests. Each animal received a fixed dose of abaloparatide without correcting for body weight.

The skin was prepared 24 hours prior to each transdermal patch application. A small area (5 x 5 cm) of the dorsal flank was prepared by close clipping of the hair with a small animal clipper. Care was taken during the clipping procedure to avoid abrasion of the skin. Both sides of the dorsal flank (thigh) were prepared for each administration to ensure a side without skin irritation was used for dose administration. The skin was wiped with an alcohol swab 15 minutes prior to patch application. Extra care was taken to ensure the collar of the path was firmly attached to the applicator prior to application and that the transdermal patch was firmly seated on the leg for administration.

Body weights of the animals were recorded prior to Day 1. 1.5 mL of whole blood from a peripheral vessel was collected pre-dose on Day 1 into a $K_3$EDTA/aprotinin tube containing 15 μL (of 2.5 mg protein/mL/aprotinin solution) per ml of whole blood.

The transdermal patch was left in place for 15 minutes after placement. A line was drawn around the site of administration to enable post-dose observations. Each dose site was scored using the Draize scoring system pre-dose on Day 1 and at 1 hour and 24 hours post-dose. After patch removal, the transdermal patch was analyzed for residual content.

1.5 mL of whole blood from a peripheral vessel was collected at 5, 10, 20, 30, 60, and 90 minutes after patch application into a $K_3$EDTA/aprotinin tube containing 15 μL (of 2.5 mg protein/mL/aprotinin solution) per ml of whole blood. Whole blood samples were collected within ± 5% of the scheduled collection time, with actual collection times recorded. Samples were kept on wet ice until processed. Animals were observed at each study blood collection time point. Any abnormalities were recorded by exception and reported immediately.

Whole blood samples were processed to plasma. Blood was centrifuged for 10 ± 2 minutes in a refrigerated centrifuge. Plasma samples were transferred to two approximately equal aliquots (aliquot 1 and aliquot 2). Samples were frozen at - 70°C ± 10 °C.

Abaloparatide concentrations were analyzed by LC-MS/MS. Abaloparatide serum concentrations were shown as percentage of the $C_{max}$ in Figures 1A-1B and Figure 2.

The bioequivalence "window" for the abaloparatide-SC treatment was established by identifying the 80%-125% serum concentration of abaloparatide versus time following the abaloparatide-SC treatment (Figure 1A). The abaloparatide-SC treatment was carried out by single subcutaneous administration of an aqueous formulation of abaloparatide (2 mg/mL) in an acetate buffer (5 mg/mL tri-hydrate sodium acetate, pH 5.1 adjusted with acetic acid) further comprising phenol (5 mg/mL) with a dose of 80 μg abaloparatide.

Application of a transdermal patch (hereinafter, the "TD-A32") prepared by coating a microneedle array with an abaloparatide preparation formulation comprising 0.8% $ZnCl_2$ and 50-60% abaloparatide in water (Preparation Formulation A32, Figure 1B) resulted in a pharmacokinetic profile that overlapped significantly with the bioequivalence window of Figure 1A. The patch ("patch-A32")is loaded with 79 μg of abaloparatide.

Application of a transdermal patch prepared by coating a microneedle array with an abaloparatide preparation formulation comprising 14.9% PEG, and 50-60% abaloparatide in water (Preparation Formulation A31, Figure 1C) resulted in a pharmacokinetic profile hat overlapped significantly with the bioequivalence window of Figure 1 a. The patch is loaded with 125 μg of abaloparatide.

Furthermore, modeling using fix increments were carried out to the pharmacokinetic profile of TD-A32 obtained as described supra. The abaloparatide-SC treatment data and TD-A32 data with dose of 79 μg were obtained from the experiments described supra (Table 1). The TD-A32 data with doses of 118.5 μg, 146.95 μg, 158 μg, and 177.75 μg (Table 1) were obtained by modeling of the experimental data of TD-A32 with dose of 79 μg, with the following formulations:

$$\frac{C_{max}\text{ of TD-A32 with dose of A } \mu g}{C_{max}\text{ of TD-A32 with dose of 79 } \mu g} = \frac{A}{79}$$

$$\frac{\text{AUC of TD-A32 with dose of A } \mu g}{\text{AUC of TD-A32 with dose of 79 } \mu g} = \frac{A}{79}$$

The TD-A32 modeling data with $C_{max}$ 90% CI and AUC 90% CI both within the range of 80-125% were bioequivalent to the abaloparatide-SC treatment (e.g., Table 1, TD-A32 with a dose of about 177.75 μg). Thus, Table shows that adjusting dose of abaloparatide of the transdermal administration may adjust the PK profile to achieve bioequivalence or substantial bioequivalence of the abaloparatide-SC treatment.

Example 2: Pharmacokinetics of abaloparatide delivered via transdermal patch prepared using preparation formulations comprising PEG or $ZnCl_2$ in humans:

The pharmacokinetic profile of transdermal administration of abaloparatide and the abaloparatide-SC treatment was assessed in healthy postmenopausal women from 50 to 80 years of age, inclusive. Subjects received a single application of a transdermal patch (100 μg abaloparatide) prepared by coating with an abaloparatide formulation comprising 54% abaloparatide in 1x PBS buffer (Figure 2, square), or SC-injection of 80 μg of abaloparatide in an aqueous formulation comprising acetate buffer (5 mg/mL tri-hydrate sodium acetate, pH 5.1 adjusted with acetic acid), 5 mg/mL phenol, and 2 mg/mL abaloparatide (Figure 2, diamond). Blood samples were collected at baseline and 5, 10, 15, 20, 30, 60, 90 and 120 minutes post dose. Abaloparatide concentrations were analyzed by LC-MS/MS method.

Transdermal delivery of abaloparatide using a transdermal patch prepared using an abaloparatide formulation without PEG or $ZnCl_2$ provided a much faster release of abaloparatide than the abaloparatide-SC treatment. Transdermal delivery using a transdermal patch prepared using an abaloparatide formulation with $ZnCl_2$ or PEG as an excipient as provided herein resulted in a PK profile that was much more similar to that of the abaloparatide-SC treatment.

Example 3: Pharmacokinetics of abaloparatide delivered via transdermal patch prepared using preparation formulations comprising $ZnCl_2$ (Formulation A) in humans:

The pharmacokinetic profile of transdermal administration of abaloparatide and the abaloparatide-SC treatment was assessed in healthy postmenopausal women.

Subjects received a single application of a transdermal patch (500x550 patch configuration with microprojections with length of 500 micrometer) loaded with 100 μg, 150 μg, or 200 μg abaloparatide, or a SC-injection of 80 μg of abaloparatide.

Certain transdermal patches were prepared by coating with an abaloparatide formulation (Formulation A) comprising 0.7% $ZnCl_2$, 39.2% abaloparatide, 60.1% WFI (water for injection) (Abaloparatide 100 μg TD, Abaloparatide 150 μg TD, and Abaloparatide 200 μg TD, respectively). Certain transdermal patches were loaded with 150 μg abaloparatide using a first general abaloparatide formulation comprising abaloparatide in PBS buffer (historical 150 μg TD).

Certain SC-injections of 80 μg of abaloparatide were administered by an injection pen of an aqueous formulation comprising acetate buffer (5 mg/mL tri-hydrate sodium acetate, pH 5.1 adjusted with acetic acid), 5 mg/mL phenol, and 2 mg/mL abaloparatide using an injection pen (UnoPen 80 μg SC). Certain SC-injections of 80 μg of abaloparatide were administered by injection of Formulation A (Abaloparatide 80 μg SC).

Blood samples were collected at baseline and various time points up to 4 hours post dose. Abaloparatide concentrations were analyzed by LC-MS/MS method. NCA (non-compartmental analyses) was performed using extravascular model. Relative actual times were used when possible, otherwise, relative nominal times were used. Nominal doses were used for the analysis. BQL were set to zero, and no subject or sample exclusions applied. Unless otherwise specified, in Figures 11 to 24, the box represent the 25th through 75th percentile of observations, the broken line represents the median of observations, the solid line represents the mean of observations, and the whiskers represents the extreme observations

PK results are summarized in Table 2, Figure 3 (longitude of median plasma abaloparatide concentration v. time post administration), Figure 4 (median plasma abaloparatide concentration v. time post administration), Figure 5 (longitude of mean plasma abaloparatide concentration v. time post administration), Figure 6 (mean plasma abaloparatide concentration v. time post administration), Figure 7 (longitude of median of dose normalized plasma abaloparatide concentration v. time post administration), Figure 8 (median of dose normalized plasma abaloparatide concentration v. time post administration), Figure 9 (longitude of mean of dose normalized plasma abaloparatide concentration v. time post administration), and Figure 10 (mean of dose normalized plasma abaloparatide concentration v. time post administration), respectively.

PK results of treatment of Abaloparatide 100 μg TD, Abaloparatide 150 μg TD, Abaloparatide 200 μg TD, Abaloparatide 80 μg SC, and historical 150 μg TD were compared to UnoPen 80 μg SC. (Table 3), and shown in Figures 11, 13, 15, 17, 19, 21, and 23 for $C_{max}$, $AUC_{last}$, $AUC_{inf}$, $C_{max}$/D ($C_{max}$ per dosage), CL/F, HL_Lambda_z, and $T_{max}$, respectively. Abaloparatide 100 μg TD, Abaloparatide 150 μg TD, and Abaloparatide 200 μg TD treatments resulted in similar exposure, wherein Abaloparatide 200 μg TD showed the most promising results.

PK results of treatment of Abaloparatide 100 μg TD, Abaloparatide 150 μg TD, Abaloparatide 200 μg TD, Abaloparatide 80 μg SC, and UnoPen 80 μg SC were compared to historical 150 μg TD (Table 4). PK results of treatment of Abaloparatide 200 μg TD, and Abaloparatide 80 μg SC, compared to historical 150 μg TD were shown in Figures 12, 14, 16, 18, 20, 22, and 24 for Cmax, $AUC_{last}$, $AUC_{inf}$, $C_{max}$/D ($C_{max}$ per dosage), CL/F, HL_Lambda_z, and $T_{max}$, respectively. Abaloparatide 100 μg TD, Abaloparatide 150 μg TD, and Abaloparatide 200 μg TD treatments all significantly enhanced abaloparatide delivery (about twice of AUC), with lower $C_{max}$ (about 60% to about 70%), longer $t_{1/2}$ (about doubled), and later $T_{max}$ compared to the historical TD formulation. The variability was similar between the two routes of administration (SC and TD), although the range (maximum-minimum) appeared lower for the TD administration (Figure 16). Comparison of the $C_{max}$ of the TD and SC delivery of Formulation A suggested a small incremental dose escalation may be needed to be more comparable (Figure 12). Figure 25 shows a PK profile of a subject treated with a transdermal patch prepared using Formulation A was within a comparable range of a UnoPen 80 μg SC treatment.

Example 4: Phase 2 study of transdermal patch prepared using first generation abaloparatide formulations in humans

Randomized, parallel-group, placebo-controlled, comparator-controlled, phase 2 study was carried out using transdermal patch prepared using a first generation abaloparatide formulation comprising abaloparatide and PBS. For 6 months, subjects received a daily TD application of a transdermal patch (with microprojections with length of 500 micrometer) loaded with 50 μg, 100 μg, or 150 μg abaloparatide (TD ABL 50 mcg, TD ABL 100 mcg, and TD ABL 150 mcg, respectively), a daily SC-injection of 80 μg of abaloparatide (SC ABL 80 mcg), or a placebo (TD Placebo) (Table 5).

Percent BMD changes from baseline of the subjects were determined at lumber spine (Figure 26), and total hip (Figure 27), respectively, N = 231 total. Local tolerance data were summarized in Figure 28 for % of subjects showing swelling or dermal response.

Summary of $C_{max}$, AUC and percent BMD change from baseline of the subjects treated with the abaloparatide transdermal patch (TD-50 mcg, TD-100 mcg and TD-150 mcg) and abaloparatide SC-injection (SC-80 mcg) were further summarized in Table 6.

Analysis of the PK/PD relationship ($C_{max}$ vs BMD and AUC vs BMD) revealed a dose dependence and a linear relationship with AUC, suggesting that AUC rather than $C_{max}$ was a key driver of efficacy (Figures 30A-30B). AUC of the subjects treated with the abaloparatide transdermal patch (green diamonds) and the subjects treated with abaloparatide SC-injection (orange diamond) showed a linear relationship versus percent BMD changes from baseline of the subjects (Figure 30B), while $C_{max}$ of these subjects did not (Figure 30A). Such data suggested AUC was a key driver of the efficacy of abaloparatide treatments.

As shown in Figure 28, the TD patches were well tolerated. POC for TD delivery of abaloparatide was shown, but with lower BMD gain achieved compared to the SC delivery.

PK profile of the TD patch prepared using the first generation abaloparatide formulation showed more pulsatile delivery than SC delivery with comparable $C_{max}$ and lower AUC (about 25-30% of SC) (Figure 29). Preliminary user experience surveys and US prescriber research suggested that both physicians and patients preferred the TD patch over SC injection by nearly 3:1.

Example 5: Pharmacokinetics of abaloparatide delivered via transdermal patch prepared using preparation formulation comprising zinc (Formulation B) in humans

The pharmacokinetic profile of transdermal administration of abaloparatide and the abaloparatide-SC treatment was assessed in healthy postmenopausal women.

Subjects received a single application of a SC-injection of 80 μg of abaloparatide, or a single application to the thigh of a transdermal patch (500x550 patch configuration with microprojections with length of 500 micrometer) loaded with 200 μg abaloparatide prepared from preparation formulation B described below and compared to prior data generated from first generation preparation formulation containing 150 μg (historical td) abaloparatide in PBS.

Certain transdermal patches were prepared by coating with an abaloparatide formulation (Formulation B) comprising abaloparatide and 2% $ZnCl_2$ in sterile water, e.g., WFI (water for injection) (Formula B TD, with 5-6% zinc chloride in the dried patch formulation). Certain transdermal patches were prepared using a first general abaloparatide formulation (First Generation TD, abaloparatide in PBS without $ZnCl_2$).

The SC-injections of 80 μg of abaloparatide were administered by an injection pen of an aqueous formulation comprising acetate buffer (5 mg/mL tri-hydrate sodium acetate, pH 5.1 adjusted with acetic acid), 5 mg/mL phenol, and 2 mg/mL abaloparatide using an injection pen (SC-injection).

Blood samples were collected at baseline and various time points up to 3 hours post dose. Abaloparatide concentrations were analyzed by LC-MS/MS method. The bars in Figure 31 represent the 25th through 75th percentile of observations.

PK results are summarized in Figures 31 and 32, showing the plasma abaloparatide concentration v. time post administration.

Delivery of Formula B TD provided a PK profile much more comparable to that of SC than the first generation TD (Figures 31 and 32). Figure 31 shows the geometric mean PK profile of the subjects treated with SC-injection (SC, n = 60, average of sc dosing studies from multiple comparator transdermal v sc studies), with Formula B TD (TD Formulaion, n = 19) as geometric mean, and with First Generation TD (TD First Generation, n = 12).

Example 6 Pharmacokinetics of transdermal formulations containing PEG

Healthy postmenopausal volunteers were treated with an 80 μg sc injection as described previously or with a transdermal patch formulated to contain 100, 150 or 200 μg abaloparatide. The transdermal formulations were coated with PEG 3350 NF with a coating formulation consisting of approximately 40% abaloparatide, 15% PEG 3350 NF and 45% sterile water (all by weight %). The PEG patch upon drying consisted essentially of 74% abaloparatide and 26% PEG 33550 NF. The administration site was the abdomen and the pK parameters are shown in Figures 33-35.

Example 7 Pharmacokinetics of transdermal formulations containing PEG/$ZnCl_2$

Healthy postmenopausal volunteers were treated with an 80 μg sc injection as described previously or with a transdermal patch formulated to contain 100, 150 or 200 μg abaloparatide. The transdermal formulations were coated with PEG 3350 NF/$ZnCl_2$ with a coating solution consisting of approximately 35% abaloparatide, 12.5% PEG3350NF, 0.7% $ZnCl_2$ and 52% water. The PEG/$ZnCl_2$ patch upon drying consisted essentially of 73% abaloparatide and 26% PEG 33550 NF, 1.5% $ZnCl_2$. The administration site was the abdomen and the pK parameters are shown in Figures 36-38.

As stated above, the foregoing is merely intended to illustrate various embodiments of the present invention. The specific modifications discussed above are not to be construed as limitations on the scope of the invention. It will be apparent to one skilled in the art that various equivalents, changes, and modifications may be made without departing from the scope of the invention, and it is understood that such equivalent embodiments are to be included herein. All references in the present disclosure are hereby incorporated by reference herein in their entireties.

Example 8: Blood sample collection from female non-human primates following administration of the Abaloparatide via transdermal patch:

Microneedle transdermal patches coated with various formulations of the PTHrP analogue of SEQ ID NO:1 were provided ready for use and stored refrigerated at 2-8°C. At least one hour prior to use, the transdermal patches in individual pouches were placed at room temperature. Eight female non-naive Chinese Cynomolgus monkeys (2-4 kg at time of dosing) were included in the study. The same eight animals were used to test each formulation, with a three day washout period between tests.

Time points were calculated from the time of patch application at 5 minutes, 10 minutes, 20 minutes, 30 minutes, 1 hour, and 1.5 hours post-dose. A time point of pre-dose was taken at Day 1. 1.5 mL of whole blood was collected at each time point. $K_3EDTA$/aprotinin was used as an anti-coagulant.

Each animal received a fixed dose of SEQ ID NO:1 without correcting for body weight. The dose administration was performed as follows:

Day 1: Test material was delivered by application of a transdermal patch. The skin was prepared 24 hours prior to each transdermal patch application as follows: a small area (5 x 5 cm) of the dorsal flank was prepared by close clipping of the hair with a small animal clipper. Care was taken during the clipping procedure to avoid abrasion of the skin. Both sides of the dorsal flank (thigh) were prepared for each administration to ensure a side without skin irritation was used for dose administration. Fifteen (15) minutes prior to patch application the skin was wiped with an alcohol swab. Extra care was taken to ensure the collar of the path was firmly attached to the applicator prior to application and that the transdermal patch was firmly seated on the leg for administration.

Days 4. 7, and 10: Test material was delivered by application of a transdermal patch. The skin was prepared 24 hours prior to each transdermal patch application as described above. Extra care was taken to ensure the collar of the path is firmly attached to the applicator prior to application and that the transdermal patch was firmly seated on the leg for administration.

Days 1, 4, 7, and 10: The transdermal patch was left in place for 15 minutes after placement. A line was drawn around the site of administration to enable post dose observations. After patch removal, the transdermal patch was analyzed for residual content.

Animals were observed at each study blood collection time point. Any abnormalities were recorded by exception and reported immediately. Each dose site at pre-dose, 1 hour and 24 hours post-dose was scored using the Draize scoring system. Body weights of the animals were recorded prior to Day 1.

All blood samples were collected from a peripheral vessel. At Day 1, 1.5 mL of whole blood was collected pre-dose into a $K_3$EDTA/aprotinin tube containing 15 μL (of 2.5 mg protein/mL/aprotinin solution) per ml of whole blood. At Days 1, 4, 7, and 10, 1.5 mL of whole blood was collected at each time point (5 minutes, 10 minutes, 20 minutes, 30 minutes, 60 minutes and 90 minutes after patch application) into a $K_3$EDTA/aprotinin tube containing 15 μL (of 2.5 mg protein/mL/aprotinin solution) per ml of whole blood. Whole blood samples were collected within ± 5% of the scheduled collection time, with actual collection times recorded. Samples were kept on wet ice until processed.

The whole blood samples were processed to plasma. Blood was centrifuged for 10 ± 2 minutes in a refrigerated centrifuge. Plasma samples were transferred to two approximately equal aliquots (aliquot 1 and aliquot 2). Samples were frozen at -70°C ± 10°C.

A): Pharmacokinetics of Abaloparatide formulations delivered via transdermal patch with different length microneedles:

As shown in Figure 39, abaloparatide (SEQ ID NO:1) without any excipient was delivered by subcutaneous administration and by transdermal patches comprising different length of microneedles, short, regular, and long.

B): Pharmacokinetics of various Abaloparatide formulations delivered via transdermal patch

Figures 40 and 41 show Cmax and AUC of delivery of Abaloparatide (SEQ ID NO:1) upon administration by transdermal patches coated with various coating formulations disclosed herein in comparison to those of Abaloparatide administered subcutaneously.

Figure 42 show the PK profile of subcutaneous (SC) delivery of SEQ ID NO: 1 (ABL), and transdermal delivery (TD) of SEQ ID NO:1 (ABL) using patches prepared by various transdermal formulations for coating. Filled diamond: ABL administered SC; unfilled triangle: an ABL formulation without excipient administered TD; filled circle: an ABL formulation comprising a PVP administered TD; filled square: an ABL formulation comprising a PLGA administered TD;_filled triangle: an ABL formulations comprising a PLGA administered TD; X: an ABL formulations comprising a HPβCD administered TD; star: an ABL formulations comprising a PLGA administered TD; unfilled circle: an ABL formulations comprising a PEG administered TD; +: an ABL formulations comprising a HPβCD administered TD; unfilled square: an ABL formulations comprising $ZnCl_2$ administered TD. ABL plasma concentration at various time after each administration is summarized in the following table.

| **Administration Route** | **SC** | **TD** | **TD** | **TD** | **TD** | **TD** | **TD** | **TD** | **TD** | **TD** |
|---|---|---|---|---|---|---|---|---|---|---|
| Formulation | | AB L only | AB L+ PVP C17 | ABL + PLG A | ABL + PLG A | ABL + HPbC D* | ABL + PLG A | ABL + PEG 3350 | ABL + HPbC D* | ABL + $ZnCl_2$ |
| % formulation or concentratio n | | | | | | | | 15% | 4.90% | 2.60% |
| Dose/patch, mcg | | 145 | 120 | 96 | 88 | 113 | 253 | 188 | 84 | 119.0 0 |
| **Time (min)** | **Avarage ABL plasma concentration (pg/mL)** | | | | | | | | | |
| **0** | 0 | 0 | 0 | 0 | 0 | 0 | 4 | 0 | 0 | 0 |
| **5** | 1900 | 390 3 | 143 2 | 2605 | 2309 | 17283 | 7731 | 1669 0 | 5591 | 4448 |
| **10** | 880 | 242 2 | 160 8 | 1959 | 2385 | 18503 | 7651 | 1679 9 | 6496 | 10651 |
| **20** | 542 | 178 9 | 195 3 | 2250 | 2648 | 19764 | 7290 | 1383 0 | 6260 | 18404 |
| **30** | 222 | 698 | 893 | 1287 | 2675 | 20134 | 6390 | 1246 9 | 5391 | 20460 |
| **60** | 120 | 186 | 538 | 465 | 2539 | 15212 | 4634 | 8846 | 5082 | 17271 |
| **90** | 224 | 68 | 108 | 335 | 1927 | 13082 | 5042 | 7564 | 4913 | 14879 |

(continued)

| Time (min) | Avarage ABL plasma concentration (pg/mL) | | | | | | | | | |
|---|---|---|---|---|---|---|---|---|---|---|
| **Cmax** | 3341 | 380 0 | 275 8 | 3237 | 3439 | 22261 | 8757 | 1764 0 | 7096 | 22090 |
| **Tmax** | 7 | 9 | 20 | 9 | 14 | 17 | 13 | 8 | 25 | 26 |
| **T1/2** | 14 | 15 | 19 | 22 | 57 | 142 | 54 | 63 | 94 | 148 |
| **AUC** | 1316 10 | 743 91 | 743 71 | 9492 5 | 2042 33 | 14337 88 | 4742 46 | 9759 61 | 44458 0 | 14366 78 |
| *: HPbCD is a HPβCD. | | | | | | | | | | |

References

References listed below are herein incorporated by reference.


1. Augustine et al., "Parathyroid hormone and parathyroid hormone-related protein analogs as therapies for osteoporosis," Curr Osteoporos Rep 11:400-406 (2013).
2. Bostrom et al., "Parathyroid hormone-related protein analog RS-66271 is an effective therapy for impaired bone healing in rabbits on corticosteroid therapy," Bone 26:437-442 (2000).
3. Dean et al., "Altered selectivity of parathyroid hormone (PTH) and PTH-related protein (PTHrP) for distinct conformations of the PTH/PTHrP receptor," Mol Endocrinol 22:156-166 (2008).
4. Dempster et al., "Skeletal histomorphometry in subjects on teriparatide or zoledronic acid therapy (SHOTZ) study: a randomized controlled trial," J Clin Endocrinol Metab 97:2799-2808 (2012).
5. Dhainaut et al., "Cortical hand bone porosity and its association with distal radius fracture in middle aged and elderly women," PLoS One 8:e68405 (2013).
6. Doyle et al., "BA058, a novel human PTHrP analog: reverses ovariectomy-induced bone loss and strength at the lumbar spine in aged cynomolgus monkeys," J Bone Miner Res 28 (Suppl 1) (2013a).
7. Doyle et al., "Long term effect of BA058, a hovel human PTHrP analog, restores bone mass in the aged osteopenic ovariectomized cynomolgus monkey," J Bone Miner Res 28 (Suppl 1) (2013a).
8. Hattersley G, Lesage E, Varela A, Mith SY. BA058, a Novel Human PTHrP Analog, Restores Bone Density and Increases Bone Strength At the Spine and Femur in Osteopenic Rats. Endocr Rev. 2013;34(abstract).
9. Horwitz et al., "Safety and tolerability of subcutaneous PTHrP(1-36) in healthy human volunteers: a dose escalation study," Osteoporos Int 17:225-230 (2006).
10. Horwitz et al., "Parathyroid hormone-related protein for the treatment of postmenopausal osteoporosis: defining the maximal tolerable dose," J Clin Endocrinol Metab 95:1279-1287 (2010).
11. Kronenberg, "PTHrP and skeletal development," Ann N Y Acad Sci 1068:1-13 (2006).
12. Leder et al., "Two years of Denosumab and teriparatide administration in postmenopausal women with osteoporosis (The DATA Extension Study): a randomized controlled trial," J Clin Endocrinol Metab 99:1694-1700 (2014).
13. Ma et al., "Comparative effects of teriparatide and strontium ranelate in the periosteum of iliac crest biopsies in postmenopausal women with osteoporosis," Bone 48:972-978 (2011).
14. MacLean et al., "Systematic review: comparative effectiveness of treatments to prevent fractures in men and women with low bone density or osteoporosis," Ann Intern Med 148:197-213 (2008).
15. Neer et al., "Effect of parathyroid hormone (1-34) on fractures and bone mineral density in postmenopausal women with osteoporosis," N Engl J Med 344:1434-1441 (2001)
16. Obaidi et al., "Pharmacokinetics and Pharmacokinetics and pharmacodynamic of subcutaneously (SC) administered doses of BA058, a bone mass density restoring agent in healthy postmenopausal women," AAPS Abstract W5385 (2010)
17. Oei et al., "High bone mineral density and fracture risk in type 2 diabetes as skeletal complications of inadequate glucose control: the Rotterdam Study," Diabetes Care 36:1619-1628 (2013).
18. Okazaki et al., "Prolonged signaling at the parathyroid hormone receptor by peptide ligands targeted to a specific receptor conformation," Proc Natl Acad Sci USA 105:16525-16530 (2008)
19. Pioszak et al., "Structural basis for parathyroid hormone-related protein binding to the parathyroid hormone receptor and design of conformation-selective peptides," J Biol Chem 284:28382-28391 (2009).
20. Recker et al., "Comparative effects of teriparatide and strontium ranelate on bone biopsies and biochemical markers of bone turnover in postmenopausal women with osteoporosis," J Bone Miner Res 24:1358-1368 (2009).

The invention also provides the following numbered embodiments:

1. A preparation formulation suitable for coating a transdermal patch wherein said preparation formulation comprises a therapeutically active substance, water and one or more excipients selected from the group consisting of $Zn^{2+}$ salts, $Mg^{2+}$ salts, $Ca^{2+}$ salts, polyethylene glycols and hydroxypropyl beta-cyclodextrins.
2. The preparation formulation of embodiment 1 further comprising a bioactive peptide or protein.
3. The preparation formulation of embodiments 1-2 comprising an antibody.
4. The preparation formulation of embodiments 1-2 comprising a bioactive peptide.
5. The preparation formulation of embodiment 4 wherein said bioactive peptide contains at least 10 amino acids.
6. The preparation formulation of embodiment 5 wherein said bioactive peptide is abaloparatide as set forth in SEQ ID NO:1.
7. The preparation formulation of any one of the preceding embodiments wherein said excipients are selected from the group consisting of $ZnCl_2$, $Zn(OAc)_2$, $Zn_3(PO_4)_2$, ZnCitrate, ZnOxalate, MgO, MgCitrate, $MgSO_4$, MgOrotate, MgLactate, $MgCO_3$ CaSorbate, CaCitrate, CaAscorbate, $Ca_3(PO_4)_2$, $CaCl_2$, $CaCO_3$, $CaSO_4$, and $Ca(OAc)_2$.
8. The preparation formulation of embodiment 7 wherein said excipient is selected from $ZnCl_2$ and $Zn(OAc)_2$ and combinations thereof.
9. The preparation formulation of any of the preceding embodiments wherein the molar ratio of said excipient or excipients to said therapeutically active substance is 0.1 to 2.0.
10. The preparation formulation of any of the preceding embodiments wherein the molar ratio of said excipient or excipients to said therapeutically active substance is 0.2 to 1.5.
11. The preparation formulation of any of the preceding embodiments wherein the molar ratio of said excipient or excipients to said therapeutically active substance is 0.25 to 1.0.
12. A transdermal patch comprising a plurality of microproj ections wherein at least one microprojection in the array is covered at least in part by a coating, said coating comprising a therapeutically active substance and one or more excipients selected from the group consisting of $Zn^{2+}$ salts, $Mg^{2+}$ salts, $Ca^{2+}$ salts, polyethylene glycols and hydroxypropyl beta-cyclodextrins.
13. The transdermal patch of embodiment 12, wherein the microprojections are microneedles.
14. The transdermal patch of any one of embodiments 12-13 wherein said therapeutically active substance comprises a bioactive peptide or protein.
15. The transdermal patch according to any of embodiments 12-14 wherein said therapeutically active substance comprises an antibody.
16. The transdermal patch according to any of embodiments 12-14 comprising a bioactive peptide.
17. The transdermal patch according to embodiment 17 wherein said bioactive peptide contains at least 10 amino acids.
18. The transdermal patch according to embodiment 17 wherein said bioactive bioactive peptide is abaloparatide as set forth in SEQ IDNO 1.
19. The transdermal patch according to embodiments 12-18 wherein and said excipients are selected from the group consisting of $ZnCl_2$, $Zn(OAc)_2$, $Zn_3(PO_4)_2$, ZnCitrate, ZnOxalate, MgO, MgCitrate, $MgSO_4$, MgOrotate, MgLactate, $MgCO_3$ CaSorbate, CaCitrate, CaAscorbate, $Ca_3(PO_4)_2$, $CaCl_2$, $CaCO_3$, $CaSO_4$, and $Ca(OAc)_2$.
20. The transdermal patch according to any one of embodiments 12-19 wherein said excipient is selected from $ZnCl_2$ and $Zn(OAc)_2$ and combinations thereof.
21. The transdermal patch according to any one of embodiments 12-20 wherein the molar ratio of said excipient or excipients to said therapeutically active substance is 0.1 to 2.0.
22. The transdermal patch according to any one of embodiments 12-21 wherein the molar ratio of said excipient or excipients to said therapeutically active substance is 0.2 to 1.5.
23. The transdermal patch according to any one of embodiments 12-22 wherein the molar ratio of said excipient or excipients to said therapeutically active substance is 0.25 to 1.0.
24. The transdermal patch according to any one of embodiments 18-23 wherein said abaloparatide is present on said microprojection array in an amount between 90-110 $\mu$g, 140-160 $\mu$g, 185-220 $\mu$g, 225-275 $\mu$g or about 100 $\mu$g, about 150 $\mu$g, about 200 $\mu$g or about 250 $\mu$g.
25. A method of treating a condition selected from the group consisting of osteoporosis, osteopenia, osteoarthritis, and bone fracture in a subject comprising administering a transdermal patch according to any one of embodiments 18-24.
26. A method of preventing vertebral, non-vertebral, clinical and major osteoporotic fractures comprising administering a transdermal patch according to any one of embodiments 18-24.
27. A method of improving bone mineral density (BMD), improving trabecular bone score (TBS), and/or reducing bone fractures in a subject comprising administering to the subject a transdermal patch according to any one of embodiments 18-24.

28. The method according to any one of embodiments 25-27 wherein said patch comprises between 300-750 microprojections.

29. The method according to any one of embodiments 25-28 wherein said administration comprises application of a force to the transdermal patch sufficient to drive one or more of the microprojections through the stratum corneum of the patient.

30. The method according to any one of embodiments 25-29 where the site of administration is the abdomen or the thigh.

**Table 1. Modeling of TD-A32 data for bioequivalence for the abaloparatide-SC treatment (SC)**

| | **SC** | **TD-A32** | **SC** | **TD-A32** | **SC** | **TD-A32** | **SC** | **TD-A32** | **SC** | **TD-A32** |
|---|---|---|---|---|---|---|---|---|---|---|
| **Dose (μg)** | | **79** | | **118.5** | | **146.9 5** | | **158** | | **177.7 5** |
| **n** | 34 | 16 | 34 | 34 | 34 | 34 | 34 | 34 | 34 | 34 |
| **$C_{max}$ (pg/mL)** | 634 2 | 3381 | 634 2 | 5072 | 634 2 | 6289 | 634 2 | 6763 | 634 2 | 7608 |
| **$C_{max}$ SD** | 317 1 | 1690 . 5 | 317 1 | 2536. 0 | 317 1 | 3144. 5 | 317 1 | 3381 . 5 | 317 1 | 3804. 0 |
| **$C_{max}$ SC/TD-A32** | | **1.87 6** | | **1.250** | | **1.008** | | **0.93 8** | | **0.834** |
| **$C_{max}$ 90% CI** | **145 %** | **259 %** | **107 %** | **147 %** | **86 %** | **118 %** | **80 %** | **110 %** | **71 %** | **98%** |
| | | | | | | | | | | |
| **AUC** | 375 862 | 1722 71 | 375 862 | 2584 07 | 375 862 | 3204 25 | 375 862 | 3445 42 | 375 862 | 3876 11 |
| **AUC SD** | 187 931 | 8613 5.5 | 187 931 | 1292 03.5 | 187 931 | 1602 12.5 | 187 931 | 1722 71 | 187 931 | 1938 05.5 |
| **AUC SC/TD-A32** | | **2.18 2** | | **1.455** | | **1.173** | | **1.09 1** | | **0.970** |
| **AUC 90% CI** | **164** % | **318 %** | **123 %** | **173 %** | **100 %** | **138 %** | **93 %** | **128 %** | **83 %** | **113 %** |

**Table 2. PK Results of Abaloparatide 100 μg TD, Abaloparatide 150 μg TD, Abaloparatide 200 μg TD, Abaloparatide 80 μg SC,** and **Historical 150 μg TD - site of administration is abdomen**

| TRT | | T1/2 (h) | Tlag (h) | Tmax (h) | Cmax (pg/mL) | Cmax/D (pg/mL/ug) | AUClast (h*pg/mL) | AUCINF (h*pg/ml) | Vz/F (L) | CL/F (L/h) |
|---|---|---|---|---|---|---|---|---|---|---|
| ABALOPARATIDE80 ug SC | N | 9 | 9 | 9 | 9 | 9 | 9 | 9 | 9 | 9 |
| | Mean | 0.92 | 0.00926 | 0.526 | 566 | 7.07 | 793 | 919 | 131 | 102 |
| | Min | 0.657 | 0 | 0.167 | 300 | 3.75 | 421 | 464 | 77.3 | 51.7 |
| | Median | 0.875 | 0 | 0.5 | 608 | 7.6 | 654 | 791 | 132 | 101 |
| | Max | 1.25 | 0.0833 | 1 | 768 | 9.6 | 1304 | 1549 | 207 | 172 |
| | CV% | 19.7 | 300 | 56 | 28.6 | 28.6 | 39.2 | 42.1 | 37 | 40.5 |
| | Geo Mean | 0.905 | | 0.457 | 542 | 6.78 | 741 | 849 | 123 | 94.2 |
| | CV% GeoMean | 19.9 | | 62 | 33 | 33 | 40.7 | 44.1 | 39.8 | 44.1 |
| ABALOPARATIDE 100 ug TD | N | 4 | 8 | 8 | 8 | 8 | 8 | 4 | 4 | 4 |
| | Mean | 1.34 | 0.0104 | 0.252 | 354 | 3.54 | 414 | 545 | 380 | 195 |
| | Min | 0.913 | 0 | 0.167 | 148 | 1.48 | 150 | 399 | 221 | 135 |
| | Median | 1.45 | 0 | 0.167 | 364 | 3.64 | 406 | 519 | 371 | 197 |
| | Max | 1.54 | 0.0833 | 0.5 | 554 | 5.54 | 736 | 743 | 556 | 250 |
| | CV% | 21.8 | 283 | 50.7 | 35.9 | 35.9 | 49.4 | 28.8 | 39.7 | 27.3 |
| | Geo Mean | 1.31 | | 0.229 | 330 | 3.3 | 363 | 529 | 357 | 189 |
| | CV% GeoMean | 24.9 | | 47.7 | 44.4 | 44.4 | 64.1 | 29 | 43.3 | 29 |

(continued)

| TRT | | T1/2 (h) | Tlag (h) | Tmax (h) | Cmax (pg/mL) | Cmax/D (pg/mL/ug) | AUClast (h*pg/mL) | AUCINF (h*pg/ml) | Vz/F (L) | CL/F (L/h) |
|---|---|---|---|---|---|---|---|---|---|---|
| ABALOPARATIDE 150 ug TD | N | 7 | 8 | 8 | 8 | 8 | 8 | 7 | 7 | 7 |
| | Mean | 1.29 | 0 | 0.292 | 387 | 2.58 | 487 | 445 | 694 | 370 |
| | Min | 1.09 | 0 | 0.167 | 202 | 1.35 | 235 | 280 | 345 | 219 |
| | Median | 1.3 | 0 | 0.333 | 427 | 2.85 | 389 | 412 | 626 | 364 |
| | Max | 1.57 | 0 | 0.5 | 530 | 3.53 | 1250 | 683 | 1030 | 535 |
| | CV% | 14.9 | | 40.4 | 30.1 | 30.1 | 68.2 | 33.3 | 36.3 | 31.6 |
| | Geo Mean | 1.28 | | 0.27 | 368 | 2.45 | 420 | 424 | 653 | 353 |
| | CV% GeoMean | 15 | | 44.3 | 36.5 | 36.5 | 58.4 | 33.8 | 39.7 | 33.8 |
| ABALOPARATIDE 200 ug TD | N | 8 | 9 | 9 | 9 | 9 | 9 | 8 | 8 | 8 |
| | Mean | 1.26 | 0 | 0.281 | 435 | 2.17 | 494 | 635 | 676 | 373 |
| | Min | 0.904 | 0 | 0.167 | 234 | 1.17 | 160 | 271 | 272 | 191 |
| | Median | 1.17 | 0 | 0.2 | 394 | 1.97 | 459 | 611 | 702 | 327 |
| | Max | 1.94 | 0 | 0.5 | 752 | 3.76 | 914 | 1045 | 1280 | 737 |
| | CV% | 25.9 | | 50.3 | 41 | 41 | 50.5 | 40.8 | 49 | 47.9 |
| | Geo Mean | 1.22 | | 0.253 | 405 | 2.02 | 434 | 586 | 602 | 341 |
| | CV% GeoMean | 24.2 | | 50.7 | 41.5 | 41.5 | 61.1 | 46.8 | 57.1 | 46.8 |

(continued)

| (h) | (h) | (h) | (pg/mL) | (pg/mL/ug) | (h*pg/mL) | (h*pg/mL) | (L) | (L/h) | | |
|---|---|---|---|---|---|---|---|---|---|---|
| Historical 150 µg TD | N | 12 | 12 | 12 | 12 | 12 | 12 | 12 | 12 | 12 |
| | Mean | 0.676 | 0 | 0.135 | 536 | 3.57 | 225 | 262 | 549 | 698 |
| | Min | 0.185 | 0 | 0.0833 | 238 | 1.59 | 62.8 | 75.3 | 251 | 389 |
| | Median | 0.61 | 0 | 0.125 | 504 | 3.36 | 236 | 293 | 534 | 512 |
| | Max | 1.81 | 0 | 0.208 | 860 | 5.73 | 368 | 386 | 1090 | 1990 |
| | CV% | 63.2 | | 35 | 31.8 | 31.8 | 35.8 | 35.4 | 39.9 | 63 |
| | Geo Mean | 0.572 | | 0.128 | 509 | 3.39 | 208 | 242 | 513 | 621 |
| | CV% GeoMean | 67 | | 37.1 | 35.4 | 35.4 | 48.2 | 48.4 | 40.1 | 48.4 |

**Table 3. Comparisons of Abaloparatide 100 μg TD, Abaloparatide 150 μg TD, Abaloparatide 200 μg TD, Abaloparatide 80 μg SC, and Historical 150 μg TD to UnoPen 80 μg SC, respectively - Site of administration is abdomen**

| Test | Dependent | GeoMean Ref | GeoMean Test | %Ratio | 90% CI | |
|---|---|---|---|---|---|---|
| ABALOPARATIDE 80 ug SC | AUCinf | 913 | 849 | 92.9 | 71 | 122 |
| | AUClast | 861 | 741 | 86.1 | 63.3 | 117 |
| | CL/F | 87.6 | 94.2 | 108 | 82.1 | 141 |
| | Cmax | 602 | 542 | 90 | 72.1 | 112 |
| | Cmax/D | 7.53 | 6.78 | 90 | 72.1 | 112 |
| | T1/2 | 1.01 | 0.905 | 89.9 | 71.1 | 114 |
| ABALOPARATIDE 100 ug TD | AUCinf | 913 | 529 | 57.9 | 39.7 | 84.4 |
| | AUClast | 861 | 363 | 42.1 | 30.5 | 58.1 |
| | CL/F | 87.6 | 189 | 216 | 148 | 315 |
| | Cmax | 602 | 330 | 54.8 | 43.5 | 69.1 |
| | Cmax/D | 7.53 | 3.3 | 43.8 | 34.8 | 55.3 |
| | T1/2 | 1.01 | 1.31 | 130 | 93.7 | 181 |
| ABALOPARATIDE 150 ug TD | AUCinf | 913 | 424 | 46.5 | 34.5 | 62.6 |
| | AUClast | 861 | 420 | 48.7 | 35.3 | 67.2 |
| | CL/F | 87.6 | 353 | 404 | 300 | 544 |
| | Cmax | 602 | 368 | 61.1 | 48.5 | 77.1 |
| | Cmax/D | 7.53 | 2.45 | 32.6 | 25.8 | 41.1 |
| | T1/2 | 1.01 | 1.28 | 127 | 98.3 | 165 |
| ARAI OPARATIDE200 Historical 150 μg TD | AUCinf | 913 | 586 | 64.2 | 48.4 | 85.1 |
| | AUClast | 861 | 434 | 50.4 | 37.1 | 68.6 |
| | CL/F | 87.6 | 341 | 390 | 294 | 517 |
| | Cmax | 602 | 405 | 67.2 | 53.8 | 83.9 |
| | Cmax/D | 7.53 | 2.02 | 26.9 | 21.5 | 33.6 |
| | T1/2 | 1.01 | 1.22 | 122 | 95.1 | 156 |
| Historical 150 μg TD | AUCinf | 913 | 242 | 26.5 | 20.8 | 33.7 |
| | AUClast | 861 | 208 | 24.1 | 18.3 | 31.8 |
| | CL/F | 87.6 | 621 | 709 | 556 | 904 |
| | Cmax | 602 | 509 | 84.6 | 69.3 | 103 |
| | Cmax/D | 7.53 | 3.39 | 45.1 | 36.9 | 55.1 |
| | T1/2 | 1.01 | 0.572 | 56.9 | 46.1 | 70.4 |

**Table 4. Comparisons of Abaloparatide 100 μg TD, Abaloparatide 150 μg TD, Abaloparatide 200 μg TD, Abaloparatide 80 μg SC to Historical 150 μg TD, Respectively - Site of administration is abdomen**

| Test | Dependent | GeoMean Ref | GeoMean Test | %Ratio | 90% CL | |
|---|---|---|---|---|---|---|
| ABALOPARATIDE 80 ug SC | AUCinf | 242 | 849 | 351 | 257 | 479 |
| | AUClast | 208 | 741 | 357 | 251 | 508 |
| | CL/F | 621 | 94.2 | 15.2 | 11.1 | 20.7 |
| | Cmax | 509 | 542 | 106 | 82.5 | 137 |
| | Cmax/D | 3.39 | 6.78 | 200 | 155 | 258 |
| | T1/2 | 0.572 | 0.905 | 158 | 121 | 207 |
| ABALOPARATIDE 100 ug TD | AUCinf | 242 | 529 | 219 | 146 | 329 |
| | AUClast | 208 | 363 | 175 | 121 | 252 |
| | CL/F | 621 | 189 | 30.5 | 20.3 | 45.8 |
| | Cmax | 509 | 330 | 64.8 | 49.8 | 84.4 |
| | Cmax/D | 3.39 | 3.3 | 97.2 | 74.6 | 127 |
| | T1/2 | 0.572 | 1.31 | 229 | 160 | 326 |
| ABALOPARATIDE 150 ug TD | AUCinf | 242 | 424 | 176 | 126 | 245 |
| | AUClast | 208 | 420 | 202 | 140 | 292 |
| | CL/F | 621 | 353 | 57 | 40.7 | 79.6 |
| | Cmax | 509 | 368 | 72.3 | 55.5 | 94.1 |
| | Cmax/D | 3.39 | 2.45 | 72.3 | 55.5 | 94.1 |
| | T1/2 | 0.572 | 1.28 | 224 | 167 | 300 |
| ABALOPARATIDE 200 ug TD | AUCinf | 242 | 586 | 242 | 176 | 334 |
| | AUClast | 208 | 434 | 209 | 147 | 298 |
| | CL/F | 621 | 341 | 55 | 39.9 | 75.9 |
| | Cmax | 509 | 405 | 79.5 | 61.6 | 103 |
| | Cmax/D | 3.39 | 2.02 | 59.6 | 46.2 | 76.9 |
| | T1/2 | 0.572 | 1.22 | 214 | 161 | 283 |

**Table 5. Design for a Phase 2 study of transdermal delivery of abaloparatide using a transdermal patch prepared by a first generation (PBS) abaloparatide formulation**

| Treatment | Daily Dose | Number of Patients |
|---|---|---|
| ABL-TD | 50 μg | 50 |
| ABL-TD | 100 μg | 50 |
| ABL-TD | 150 μg | 50 |
| ABL-SC | 80 μg | 50 |
| Placebo | - | 50 |
| | **Total** | **250** |

**Table 6. $C_{max}$, AUC, and BMD improvement of a Phase 2 study of transdermal delivery of abaloparatide using a transdermal patch prepared by a first generation (PBS buffer) abaloparatide formulation**

| Administration method - Dose | $C_{max}$ (pg/mL) | AUC (pg/ml.hr) | BMD Lumbar spine at 6month (%change from baseline (n=231)) |
|---|---|---|---|
| TD-50 mcg | 275 | 4,499 | 1.87 |
| TD-100 mcg | 468 | 7,953 | 2.33 |
| TD-150 mcg | 656 | 13,557 | 2.95 |
| SC-80 mcg | 592 | 49,270 | 5.8 |

## Claims

1. A preparation formulation suitable for coating a transdermal patch wherein said preparation formulation comprises a therapeutically active substance, water and one or more excipients selected from the group consisting of $Zn^{2+}$ salts, $Mg^{2+}$ salts, $Ca^{2+}$ salts, polyethylene glycols and hydroxypropyl beta-cyclodextrins.

2. The preparation formulation of claim 1:

   (i) further comprising a bioactive peptide or protein; and/or
   (ii) comprising an antibody.

3. The preparation formulation of claims 1-2 comprising a bioactive peptide, optionally wherein said bioactive peptide contains at least 10 amino acids, optionally wherein said bioactive peptide is abaloparatide as set forth in SEQ ID NO:1.

4. The preparation formulation of any one of the preceding claims wherein said excipients are selected from the group consisting of $ZnCl_2$, $Zn(OAc)_2$, $Zn_3(PO_4)_2$, ZnCitrate, ZnOxalate, MgO, MgCitrate, $MgSO_4$, MgOrotate, MgLactate, $MgCO_3$ CaSorbate, CaCitrate, CaAscorbate, $Ca_3(PO_4)_2$, $CaCl_2$, $CaCO_3$, $CaSO_4$, and $Ca(OAc)_2$, optionally wherein said excipient is selected from $ZnCl_2$ and $Zn(OAc)_2$ and combinations thereof.

5. The preparation formulation of any of the preceding claims wherein:

   (i) the molar ratio of said excipient or excipients to said therapeutically active substance is 0.1 to 2.0;
   (ii) the molar ratio of said excipient or excipients to said therapeutically active substance is 0.2 to 1.5; and/or
   (iii) the molar ratio of said excipient or excipients to said therapeutically active substance is 0.25 to 1.0.

6. A transdermal patch comprising a plurality of microprojections wherein at least one microprojection in the array is covered at least in part by a coating, said coating comprising a therapeutically active substance and one or more excipients selected from the group consisting Of $Zn^{2+}$ salts, $Mg^{2+}$ salts, $Ca^{2+}$ salts, polyethylene glycols and hydroxypropyl beta-cyclodextrins.

7. The transdermal patch of claim 6, wherein:

   (i) the microprojections are microneedles;
   (ii) said therapeutically active substance comprises a bioactive peptide or protein; and/or
   (iii) said therapeutically active substance comprises an antibody.

8. The transdermal patch according to claim 6 or 7 comprising a bioactive peptide, optionally wherein said bioactive peptide contains at least 10 amino acids, optionally wherein said bioactive bioactive peptide is abaloparatide as set forth in SEQ IDNO 1.

9. The transdermal patch according to claims 6-8 wherein and said excipients are selected from the group consisting of $ZnCl_2$, $Zn(OAc)_2$, $Zn_3(PO_4)_2$, ZnCitrate, ZnOxalate, MgO, MgCitrate, $MgSO_4$, MgOrotate, MgLactate, $MgCO_3$ CaSorbate, CaCitrate, CaAscorbate, $Ca_3(PO_4)_2$, $CaCl_2$, $CaCO_3$, $CaSO_4$, and $Ca(OAc)_2$, optionally wherein said

excipient is selected from $ZnCl_2$ and $Zn(OAc)_2$ and combinations thereof.

**10.** The transdermal patch according to any one of claims 6-9 wherein:

(i) the molar ratio of said excipient or excipients to said therapeutically active substance is 0.1 to 2.0;
(ii) the molar ratio of said excipient or excipients to said therapeutically active substance is 0.2 to 1.5; and/or
(iii) the molar ratio of said excipient or excipients to said therapeutically active substance is 0.25 to 1.0.

**11.** The transdermal patch according to any one of claims 8-10 wherein said abaloparatide is present on said microprojection array in an amount between 90-110 μg, 140-160 μg, 185-220 μg, 225-275 μg or about 100 μg, about 150 μg, about 200 μg or about 250 μg.

**12.** Abaloparatide for use in a method of treating a condition selected from the group consisting of osteoporosis, osteopenia, osteoarthritis, and bone fracture in a subject, the method comprising administering a transdermal patch according to any one of claims 8-11.

**13.** Abaloparatide for use in a method of preventing vertebral, non-vertebral, clinical and major osteoporotic fractures, the method comprising administering a transdermal patch according to any one of claims 8-11.

**14.** Abaloparatide for use in a method of improving bone mineral density (BMD), improving trabecular bone score (TBS), and/or reducing bone fractures in a subject comprising administering to the subject atransdermal patch according to any one of claims 8-11.

**15.** Abaloparatide for use according to any one of claims 12-14 wherein:

(i) said patch comprises between 300-750 microprojections;
(ii) said administration comprises application of a force to the transdermal patch sufficient to drive one or more of the microprojections through the stratum corneum of the patient; and/or
(iii) the site of administration is the abdomen or the thigh.

Figure 1A

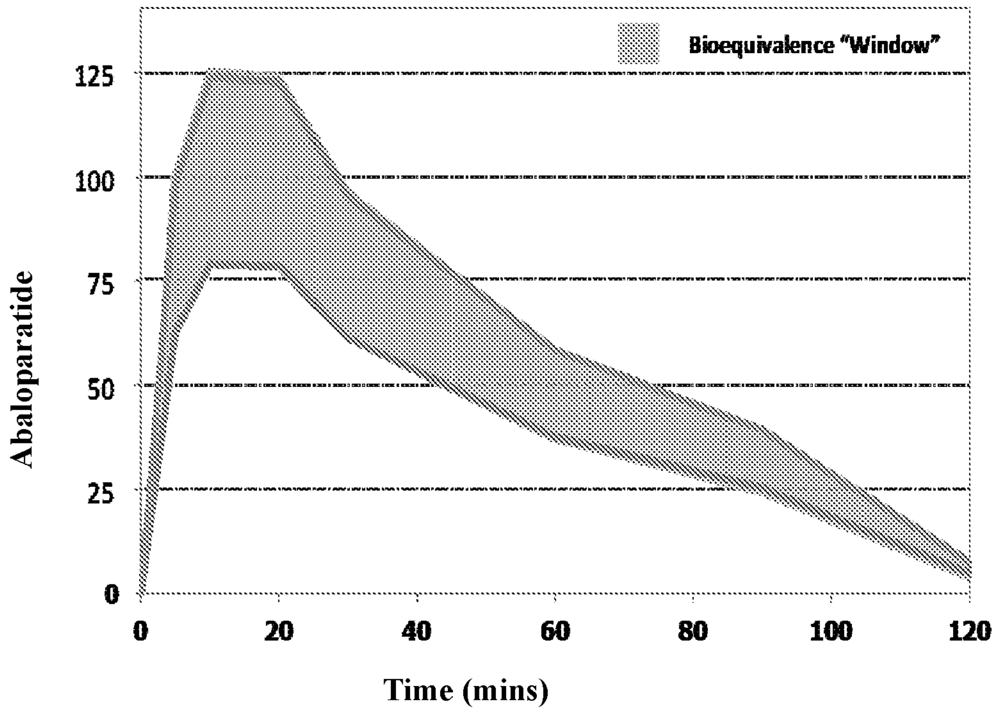


Figure 1B

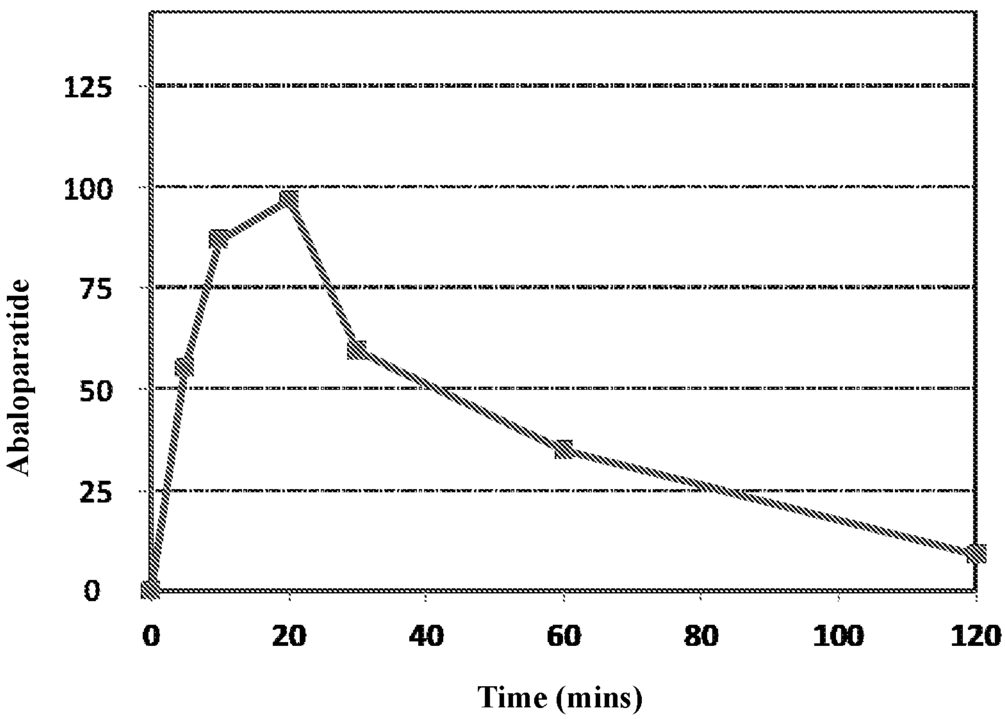

**Figure 1C**

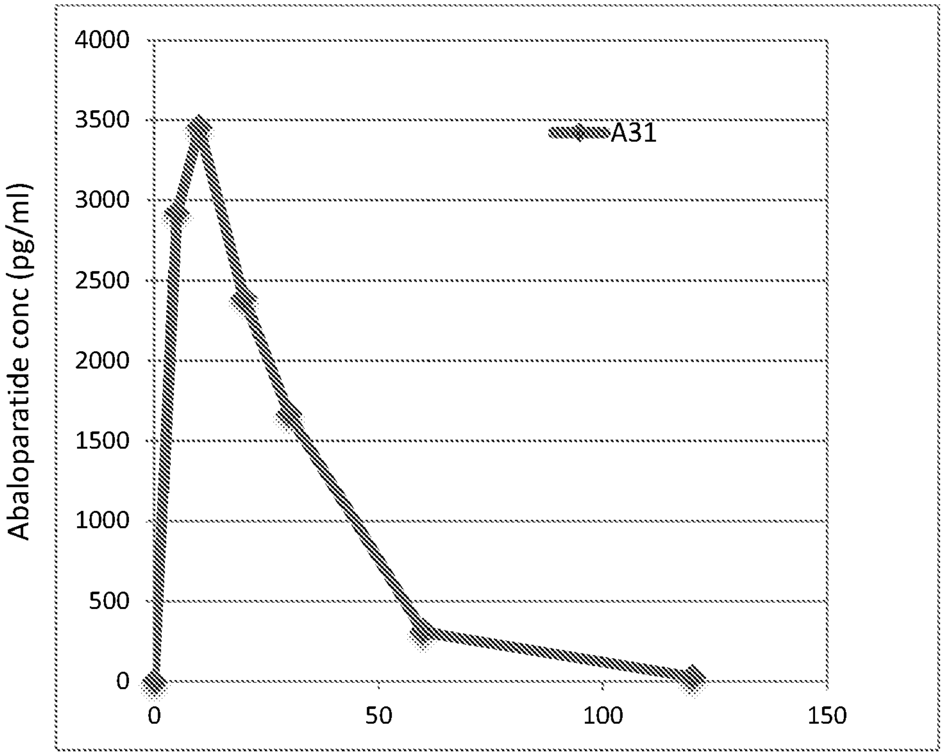


Time (mins)

Figure 2

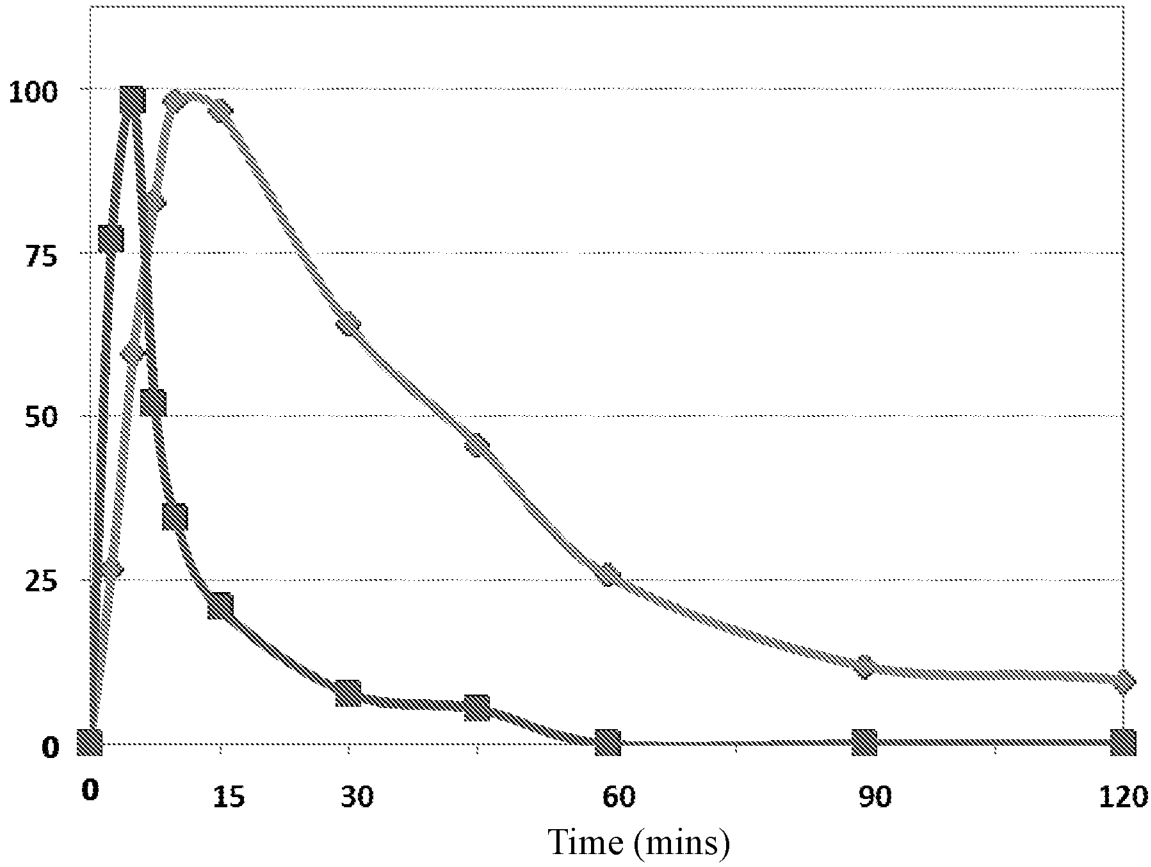

# Figure 3

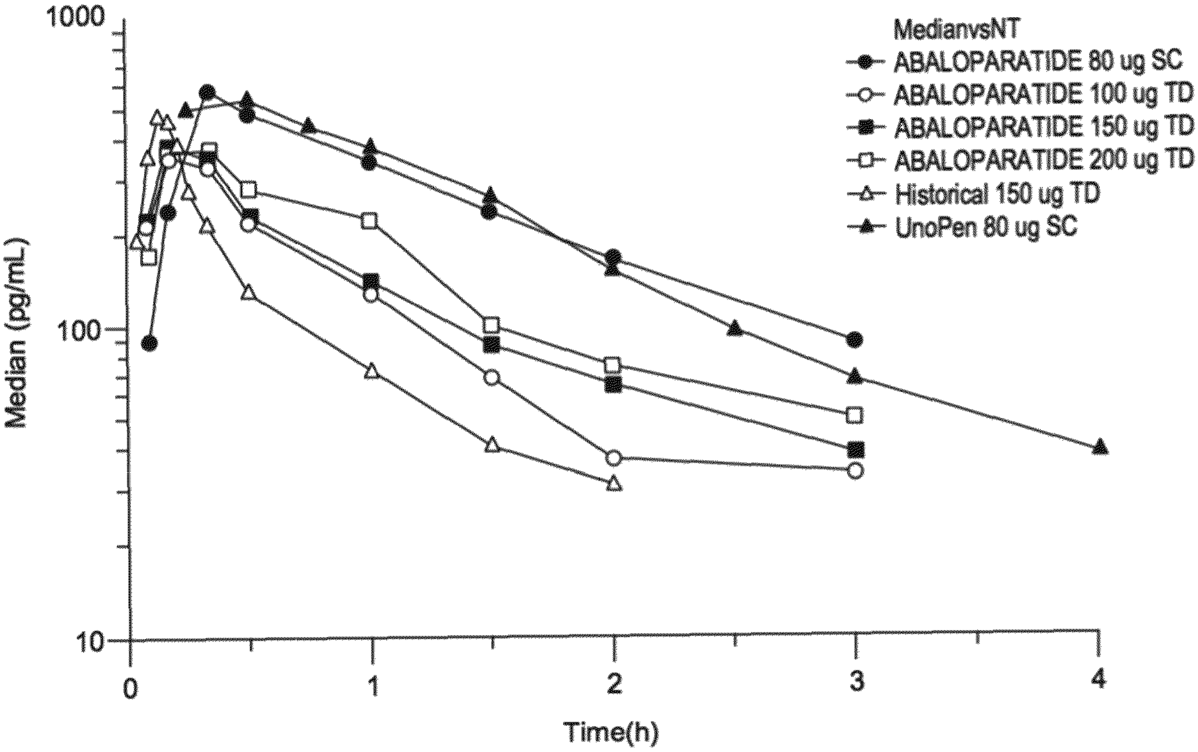
MedianvsNT
ABALOPARATIDE 80 ug SC
ABALOPARATIDE 100 ug TD
ABALOPARATIDE 150 ug TD
ABALOPARATIDE 200 ug TD
Historical 150 ug TD
UnoPen 80 ug SC
1000
100
10
0
1
2
3
4
Median (pg/mL)
Time(h)

# Figure 4

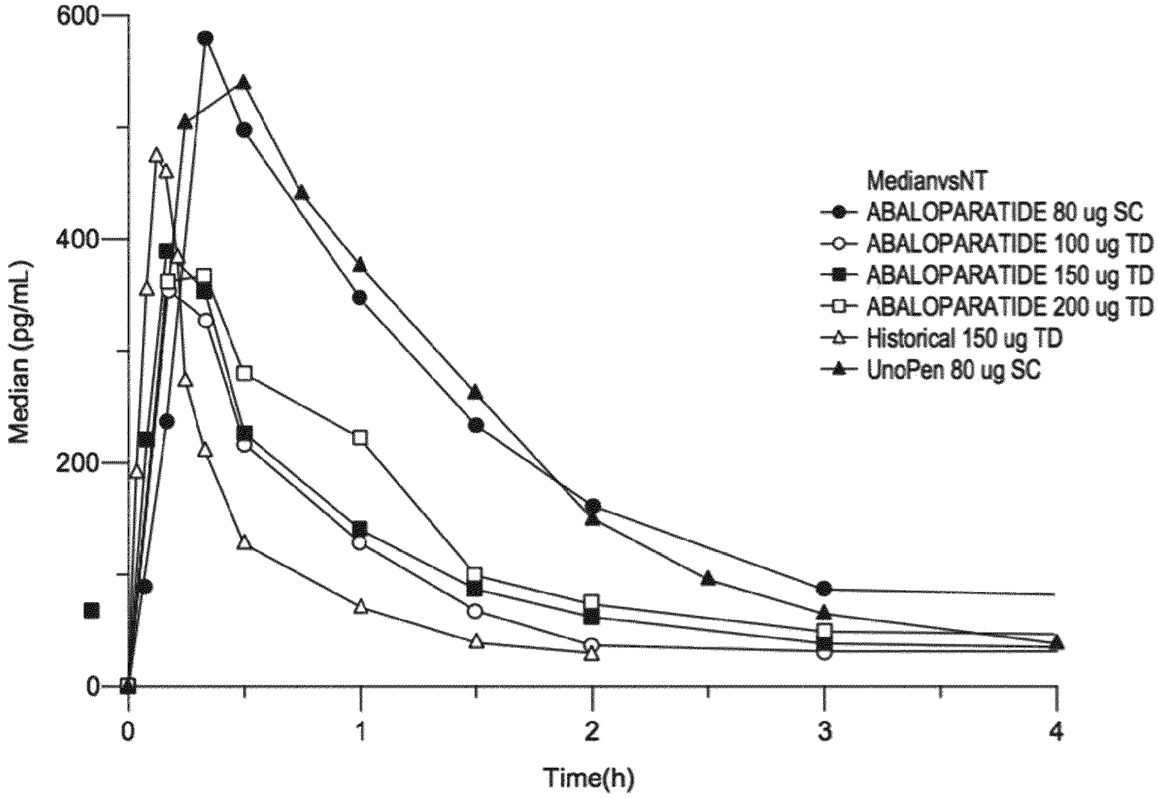


# Figure 5

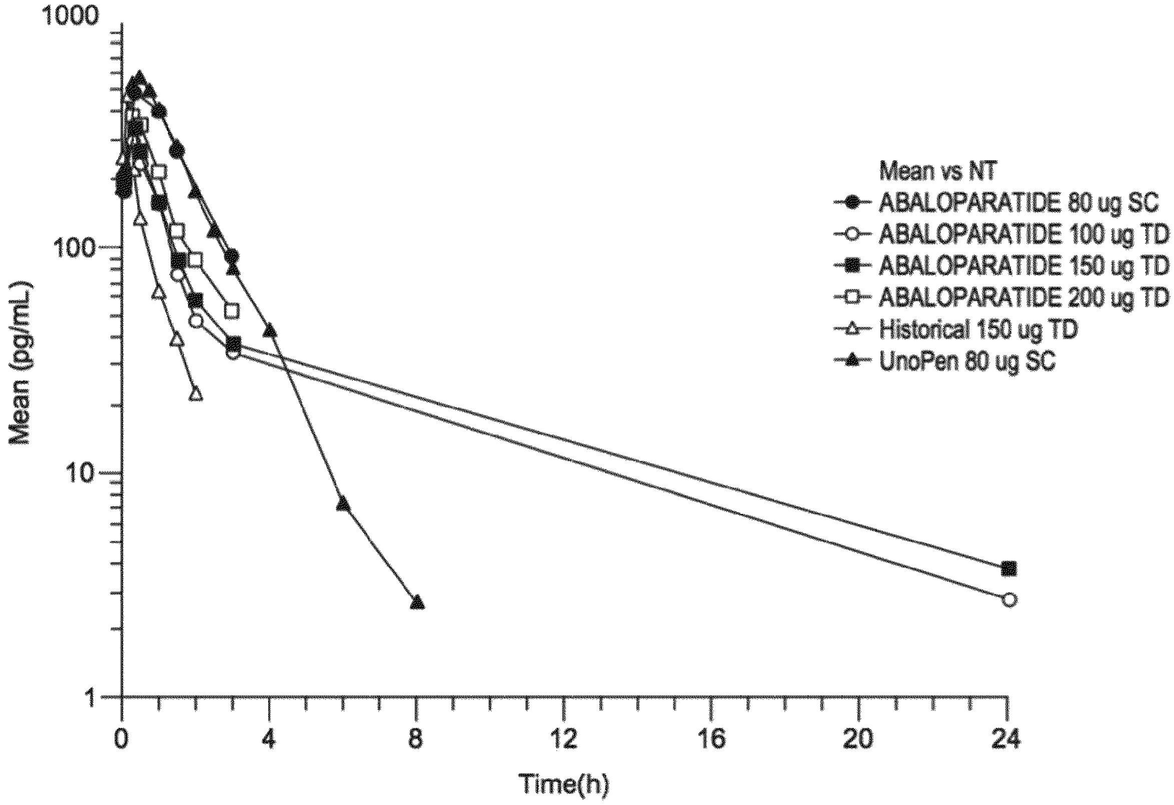

# Figure 6

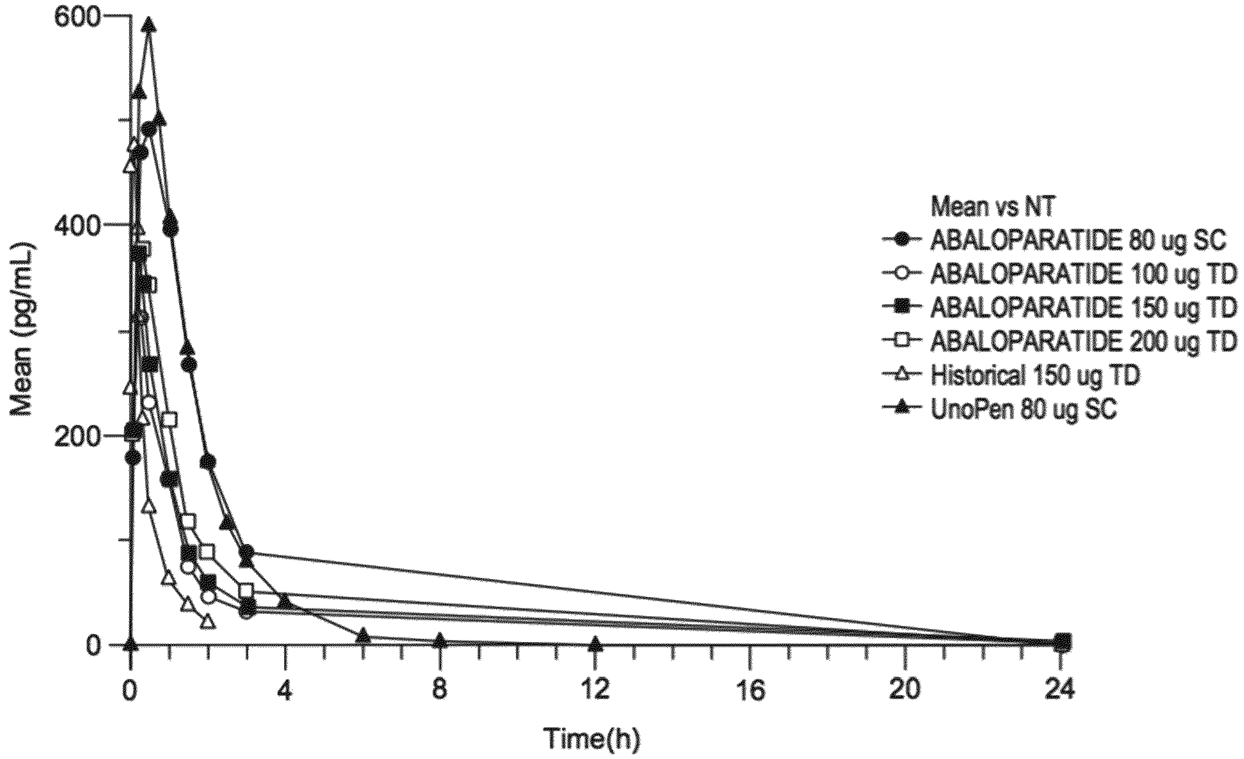


# Figure 7

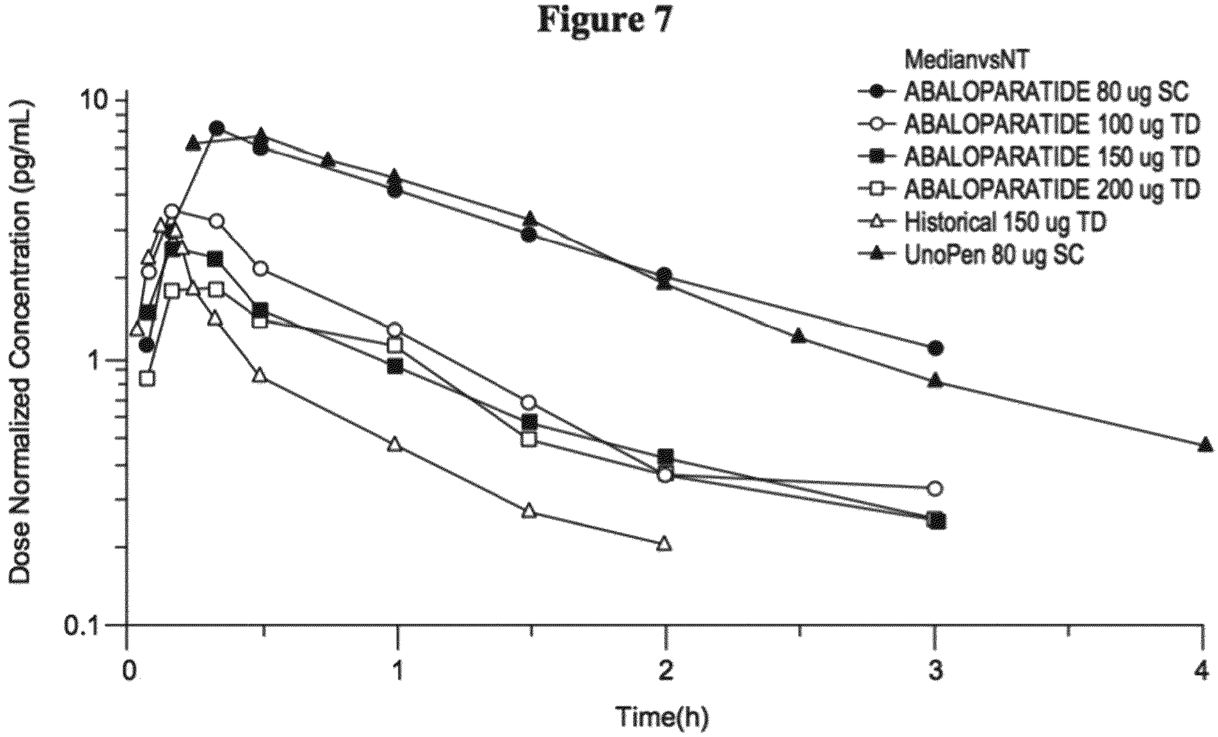

Figure 8

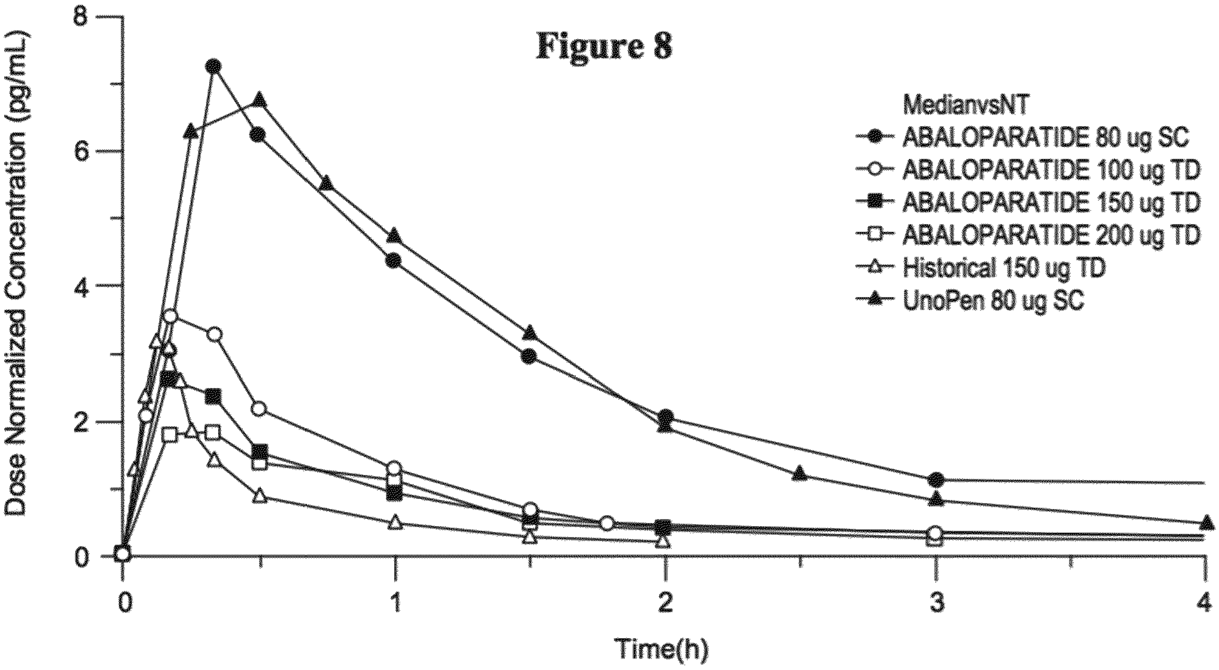


Figure 9

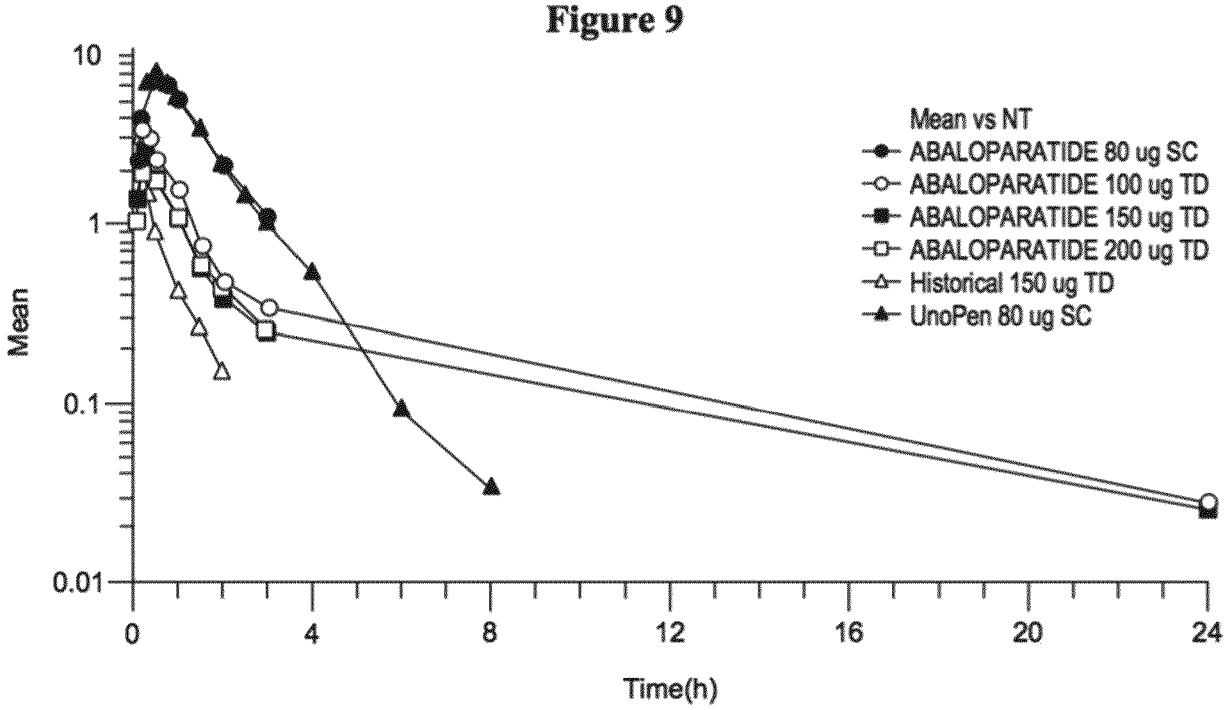

# Figure 10

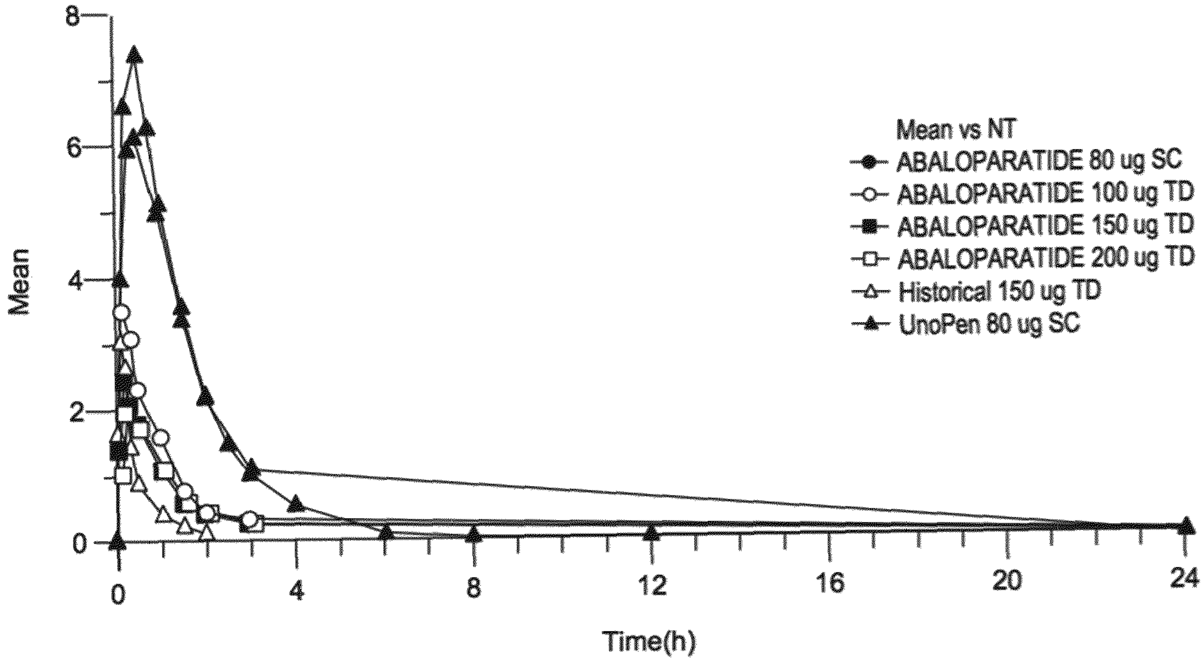
Mean
Time(h)
0
2
4
6
8
0
4
8
12
16
20
24
Mean vs NT
ABALOPARATIDE 80 ug SC
ABALOPARATIDE 100 ug TD
ABALOPARATIDE 150 ug TD
ABALOPARATIDE 200 ug TD
Historical 150 ug TD
UnoPen 80 ug SC

Figure 11

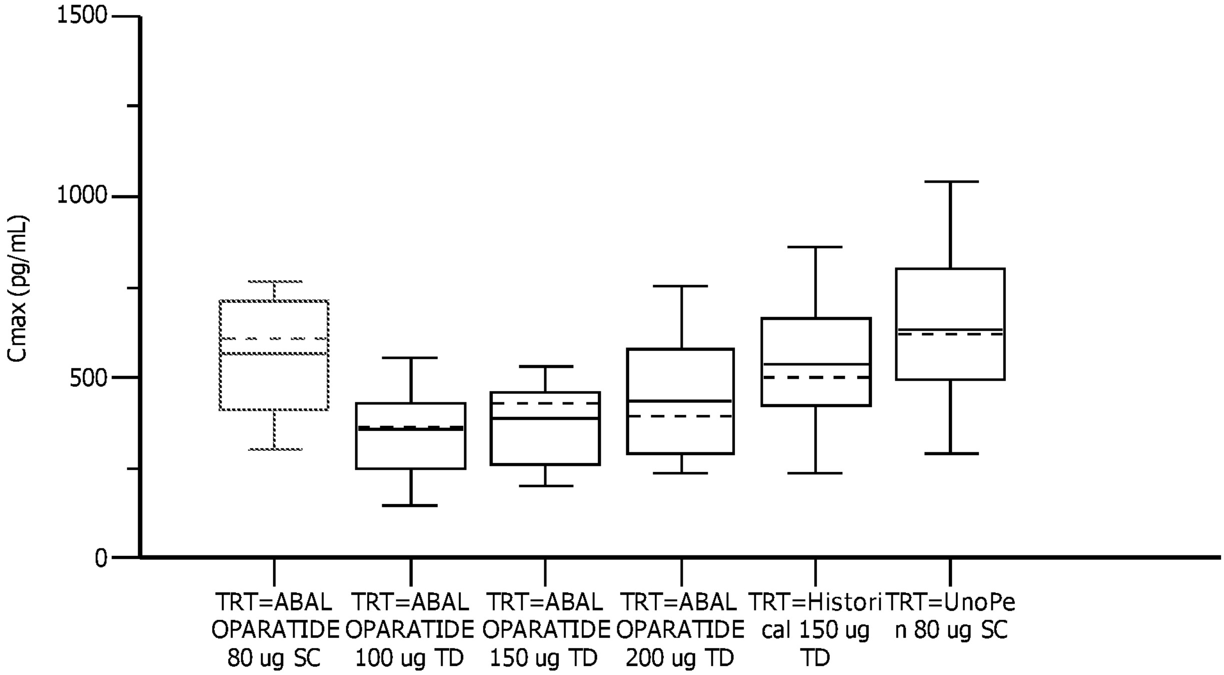
1500
1000
500
0
Cmax (pg/mL)
TRT=ABAL OPARATIDE 80 ug SC
TRT=ABAL OPARATIDE 100 ug TD
TRT=ABAL OPARATIDE 150 ug TD
TRT=ABAL OPARATIDE 200 ug TD
TRT=Historical 150 ug TD
TRT=UnoPen 80 ug SC

**Figure 12**

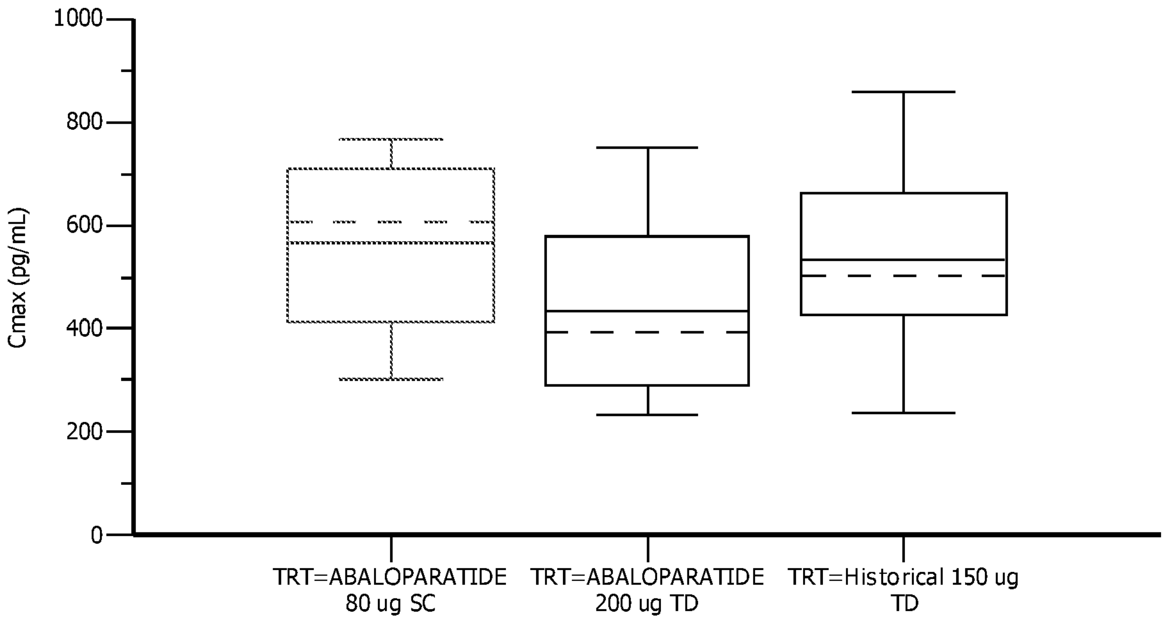

1000
800
600
400
200
0
Cmax (pg/mL)
TRT=ABALOPARATIDE 80 ug SC
TRT=ABALOPARATIDE 200 ug TD
TRT=Historical 150 ug TD

**Figure 13**

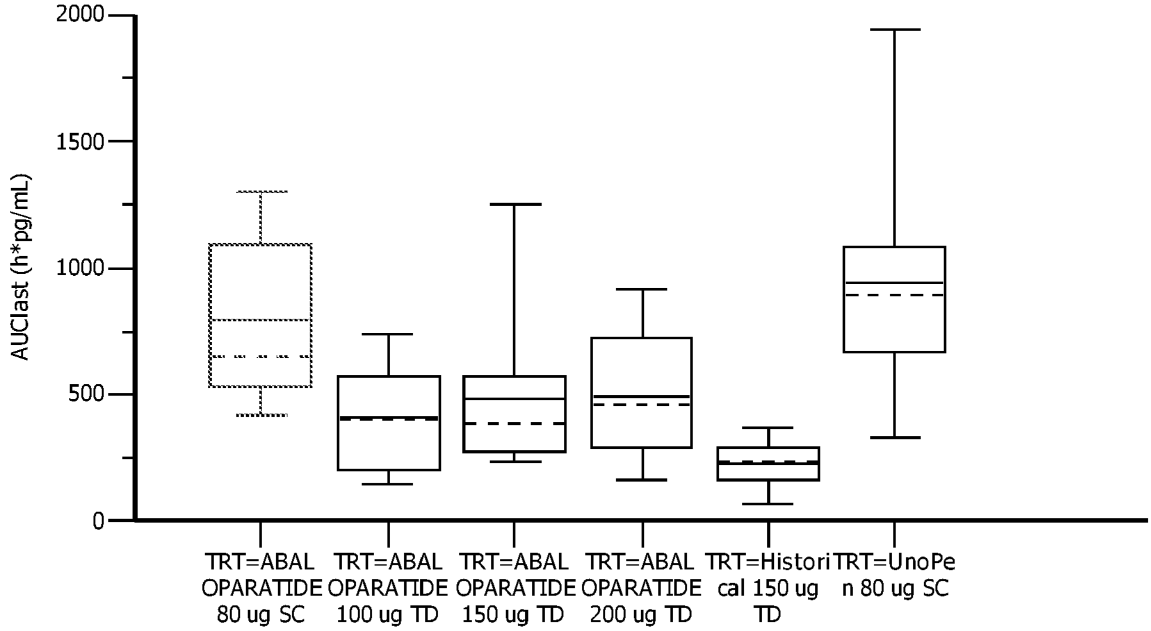
AUClast (h*pg/mL)
2000
1500
1000
500
0
TRT=ABAL OPARATIDE 80 ug SC
TRT=ABAL OPARATIDE 100 ug TD
TRT=ABAL OPARATIDE 150 ug TD
TRT=ABAL OPARATIDE 200 ug TD
TRT=Histori cal 150 ug TD
TRT=UnoPe n 80 ug SC

**Figure 14**

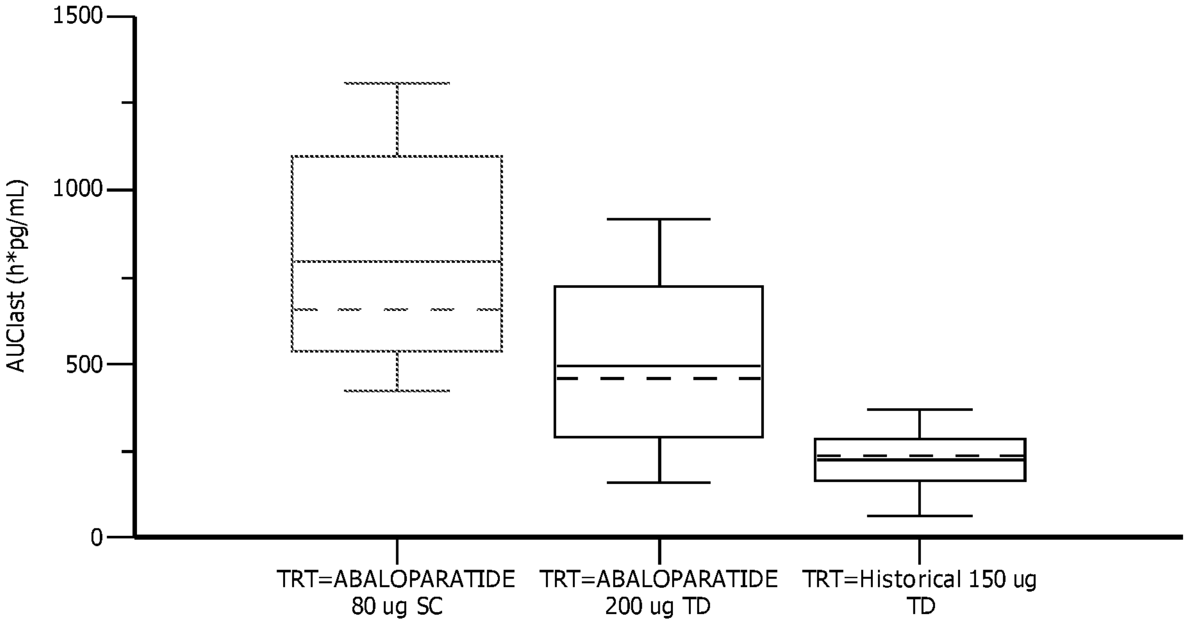
1500
1000
500
0
AUClast (h*pg/mL)
TRT=ABALOPARATIDE 80 ug SC
TRT=ABALOPARATIDE 200 ug TD
TRT=Historical 150 ug TD

**Figure 15**

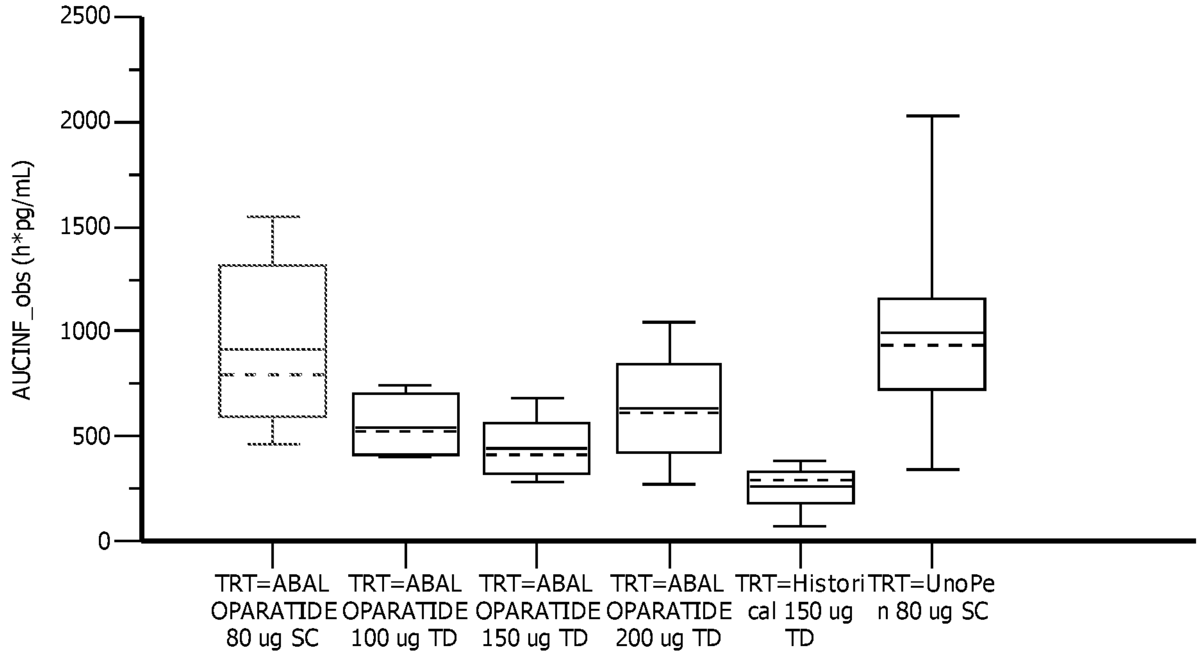
2500
2000
1500
1000
500
0
AUCINF_obs (h*pg/mL)
TRT=ABAL OPARATIDE 80 ug SC
TRT=ABAL OPARATIDE 100 ug TD
TRT=ABAL OPARATIDE 150 ug TD
TRT=ABAL OPARATIDE 200 ug TD
TRT=Histori cal 150 ug TD
TRT=UnoPe n 80 ug SC

**Figure 16**

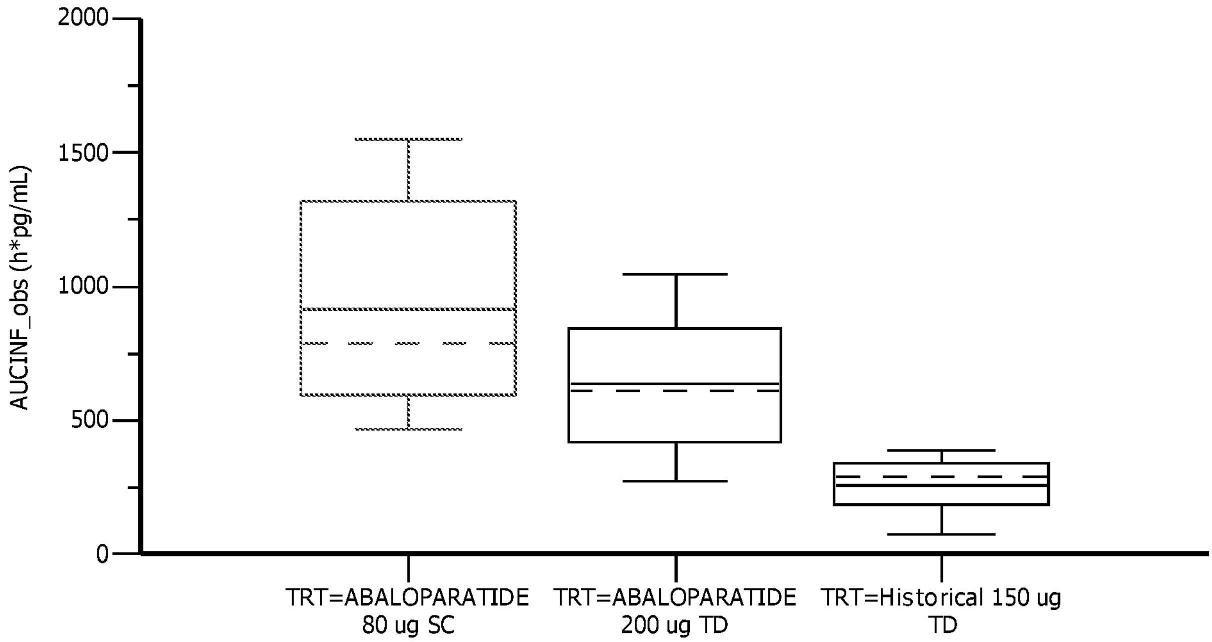
2000
1500
1000
500
0
AUCINF_obs (h*pg/mL)
TRT=ABALOPARATIDE 80 ug SC
TRT=ABALOPARATIDE 200 ug TD
TRT=Historical 150 ug TD

**Figure 17**

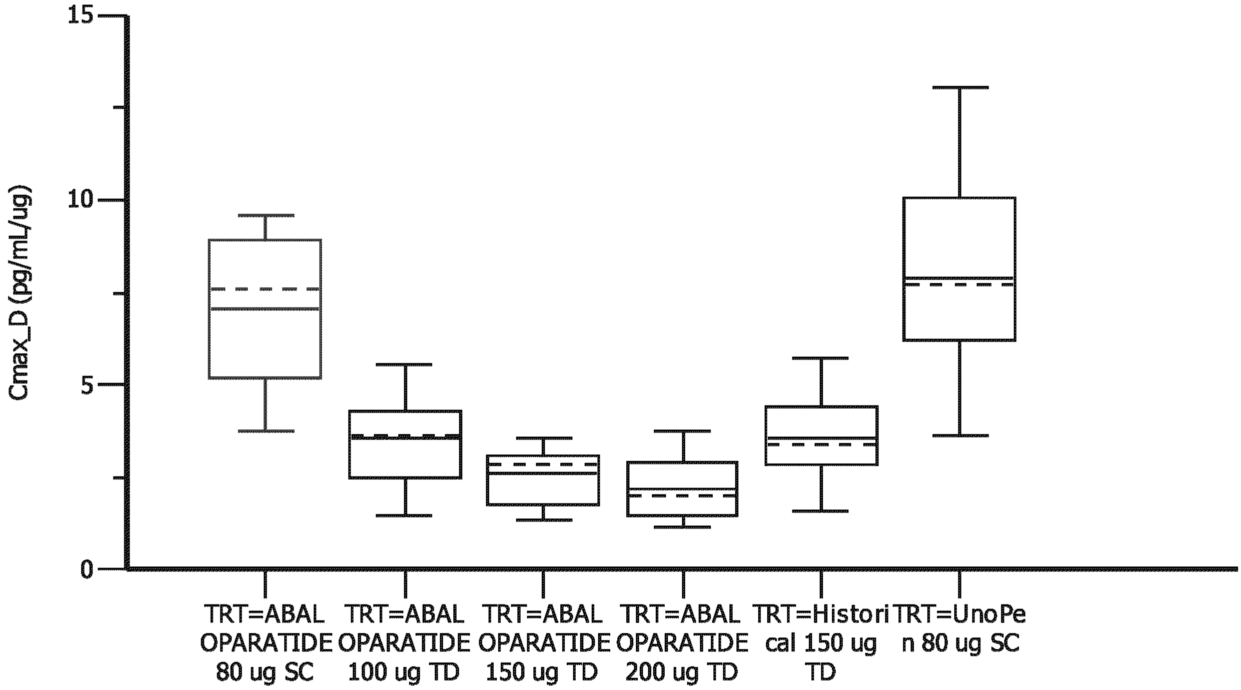

Cmax_D (pg/mL/ug)
15
10
5
0
TRT=ABALOPARATIDE 80 ug SC
TRT=ABALOPARATIDE 100 ug TD
TRT=ABALOPARATIDE 150 ug TD
TRT=ABALOPARATIDE 200 ug TD
TRT=Historical 150 ug TD
TRT=UnoPen 80 ug SC

Figure 18

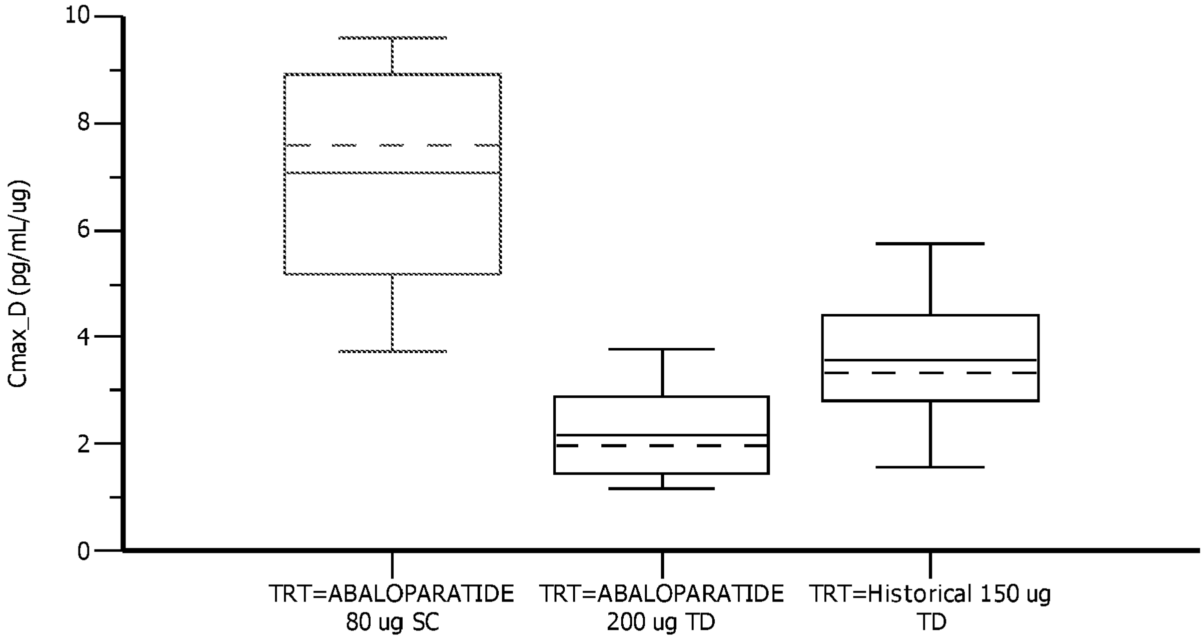
Cmax_D (pg/mL/ug)
10
8
6
4
2
0
TRT=ABALOPARATIDE 80 ug SC
TRT=ABALOPARATIDE 200 ug TD
TRT=Historical 150 ug TD

Figure 19

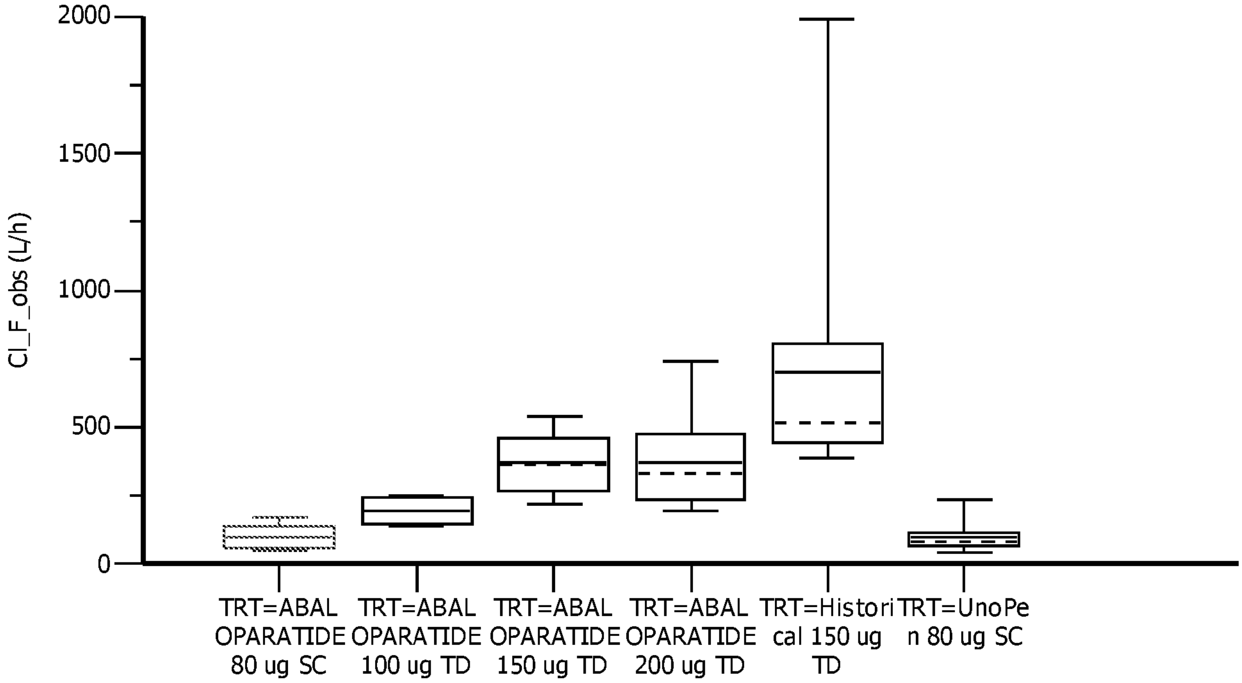
2000
1500
1000
500
0
Cl_F_obs (L/h)
TRT=ABAL OPARATIDE 80 ug SC
TRT=ABAL OPARATIDE 100 ug TD
TRT=ABAL OPARATIDE 150 ug TD
TRT=ABAL OPARATIDE 200 ug TD
TRT=Historical 150 ug TD
TRT=UnoPen 80 ug SC

**Figure 20**

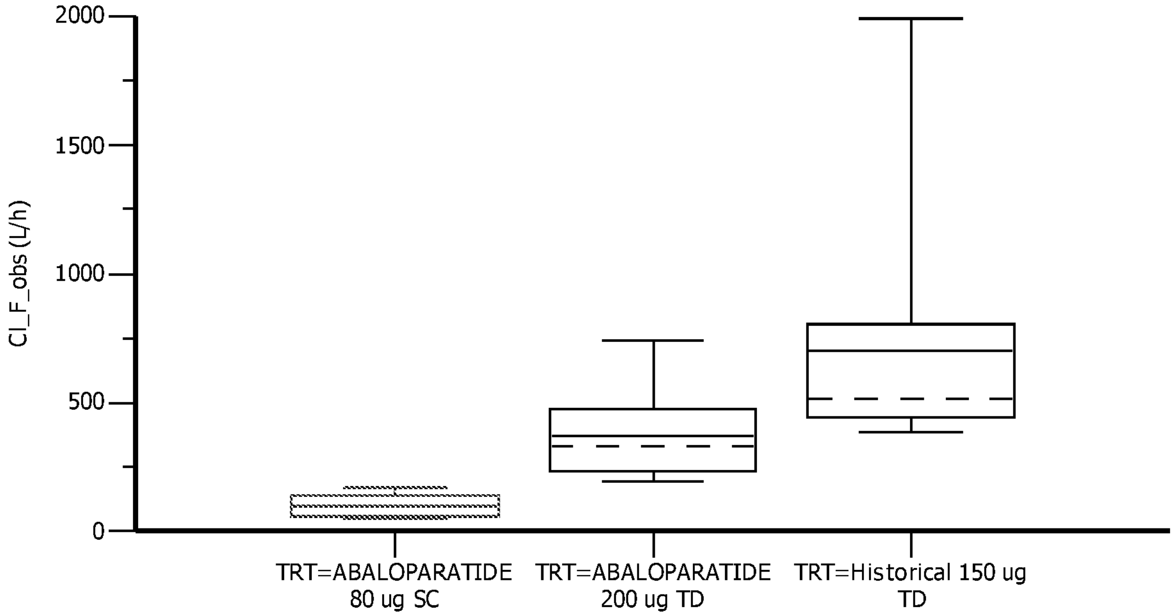
2000
1500
1000
500
0
Cl_F_obs (L/h)
TRT=ABALOPARATIDE 80 ug SC
TRT=ABALOPARATIDE 200 ug TD
TRT=Historical 150 ug TD

**Figure 21**

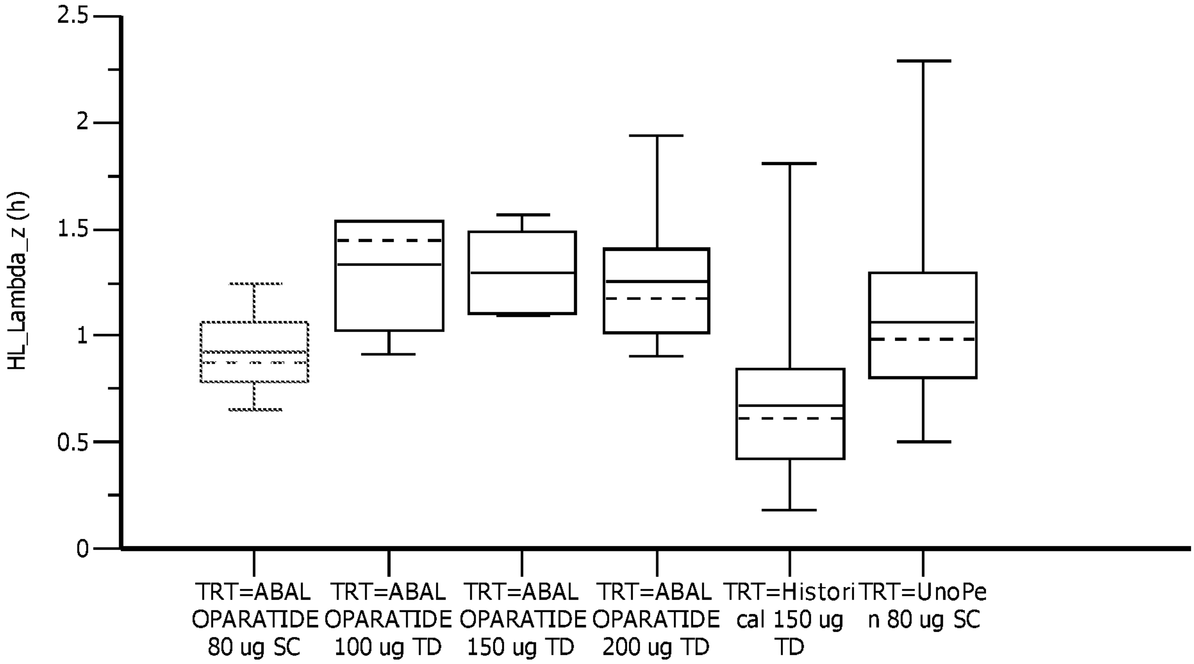
HL_Lambda_z (h)
2.5
2
1.5
1
0.5
0
TRT=ABALOPARATIDE 80 ug SC
TRT=ABALOPARATIDE 100 ug TD
TRT=ABALOPARATIDE 150 ug TD
TRT=ABALOPARATIDE 200 ug TD
TRT=Historical 150 ug TD
TRT=UnoPen 80 ug SC

**Figure 22**

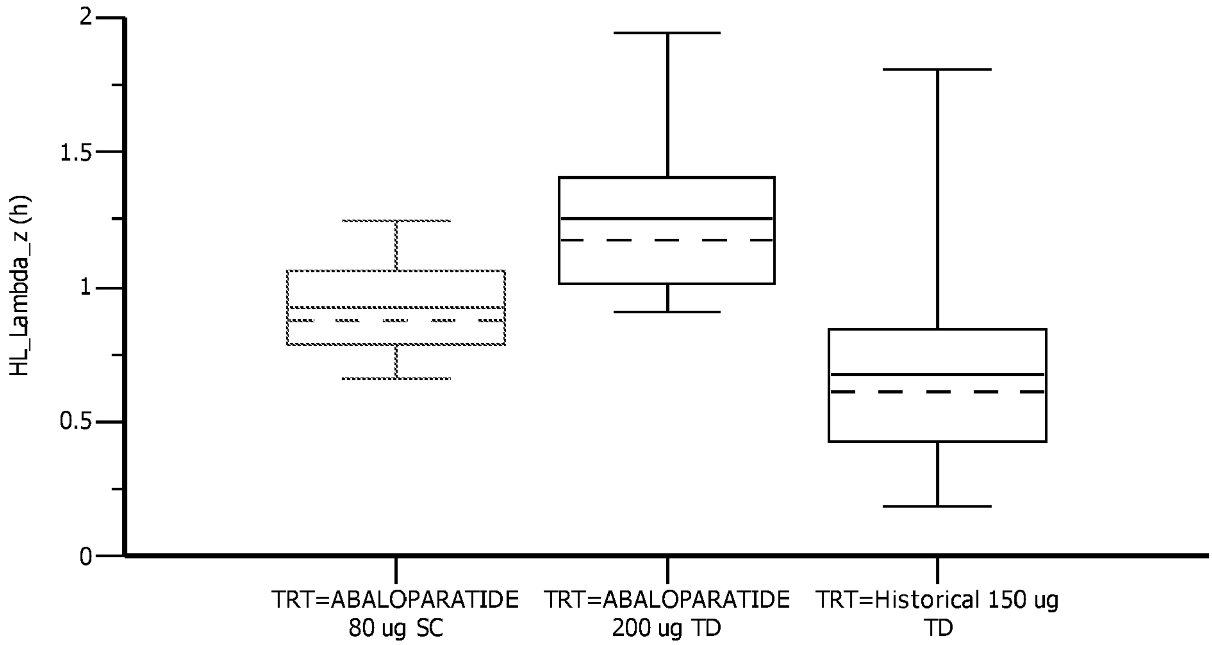
HL_Lambda_z (h)
2
1.5
1
0.5
0
TRT=ABALOPARATIDE 80 ug SC
TRT=ABALOPARATIDE 200 ug TD
TRT=Historical 150 ug TD

**Figure 23**

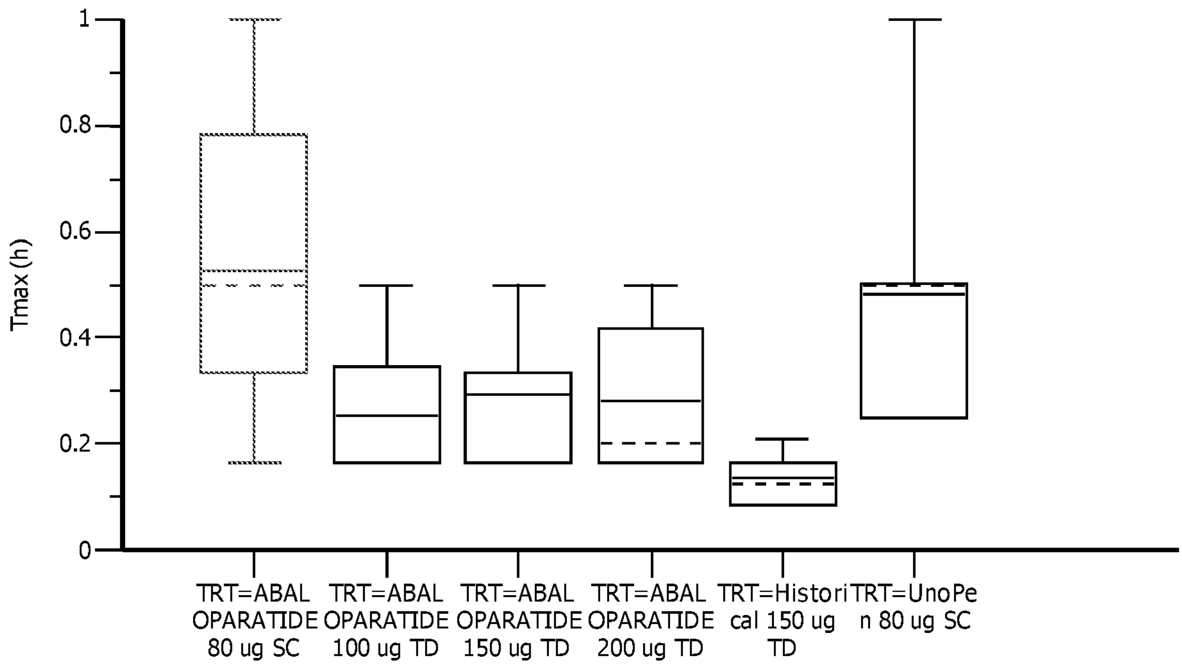
Tmax (h)
1
0.8
0.6
0.4
0.2
0
TRT=ABAL OPARATIDE 80 ug SC
TRT=ABAL OPARATIDE 100 ug TD
TRT=ABAL OPARATIDE 150 ug TD
TRT=ABAL OPARATIDE 200 ug TD
TRT=Historical 150 ug TD
TRT=UnoPen 80 ug SC

**Figure 24**

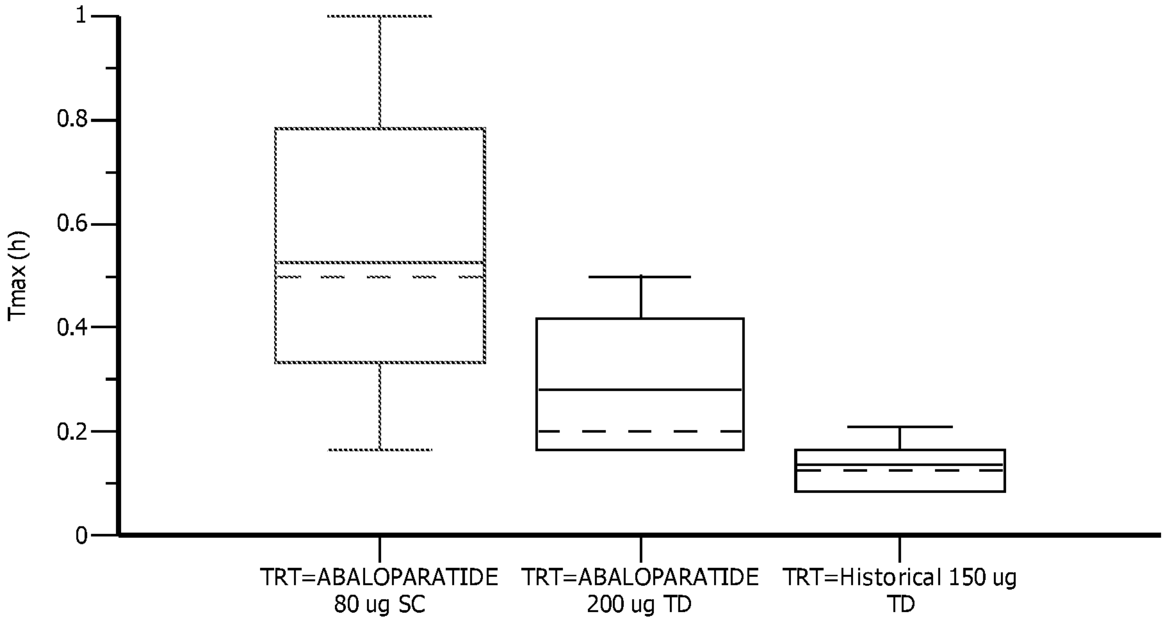
Tmax (h)
1
0.8
0.6
0.4
0.2
0
TRT=ABALOPARATIDE 80 ug SC
TRT=ABALOPARATIDE 200 ug TD
TRT=Historical 150 ug TD

Figure 25

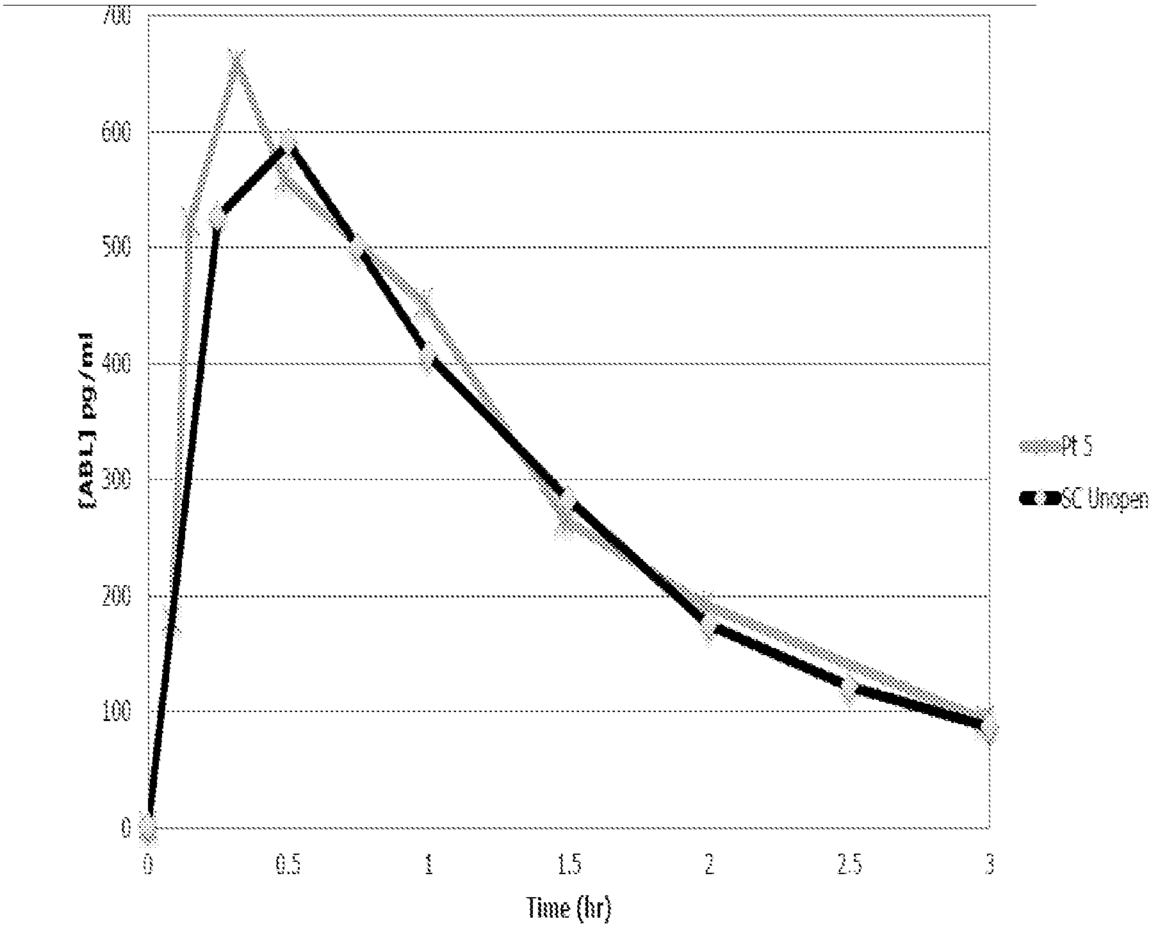
700
600
500
400
300
200
100
0
[ABL] pg/ml
0
0.5
1
1.5
2
2.5
3
Time (hr)
Pt 5
SC Unopen

Figure 26

Lumbar spine BMD (mITT population, N = 231)

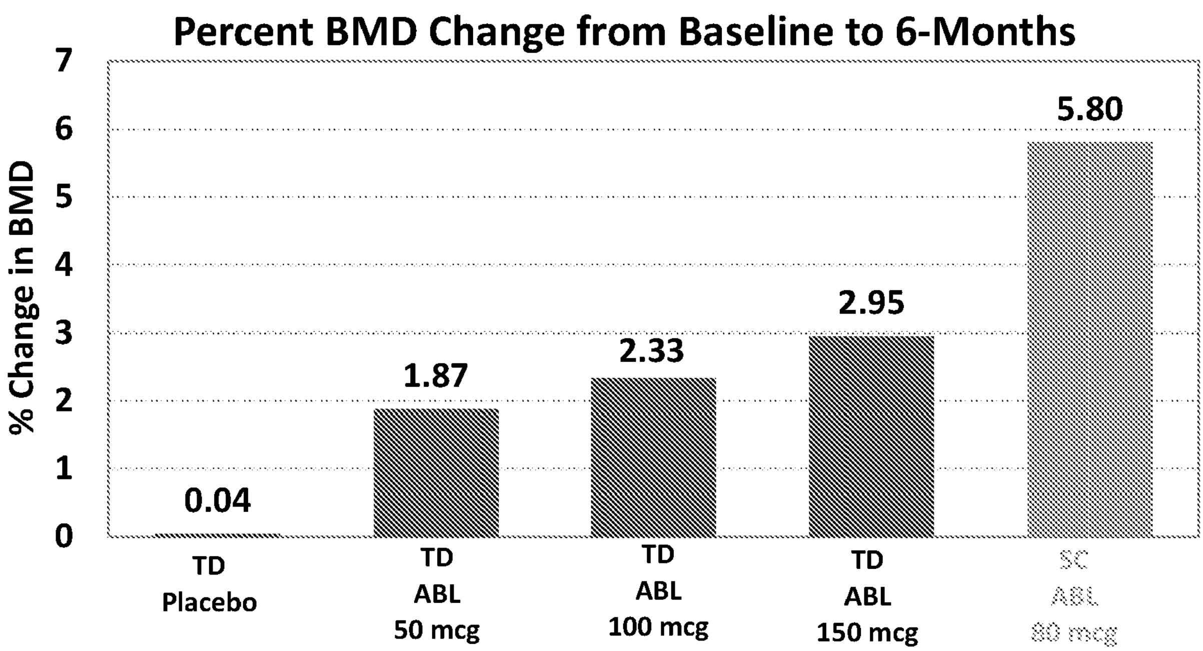

**Figure 27**

**Total Hip BMD (mITT population, N = 231)**

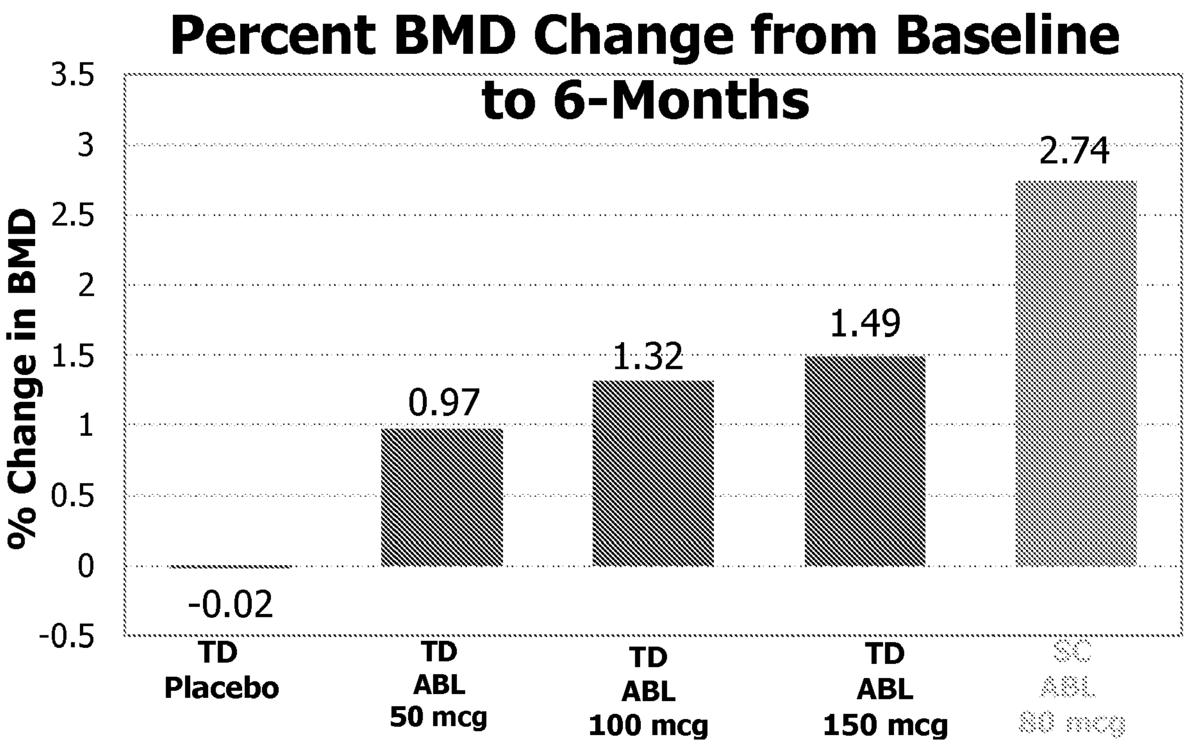

**Figure 28**

**Local Tolerance (Safety population, N = 249)**

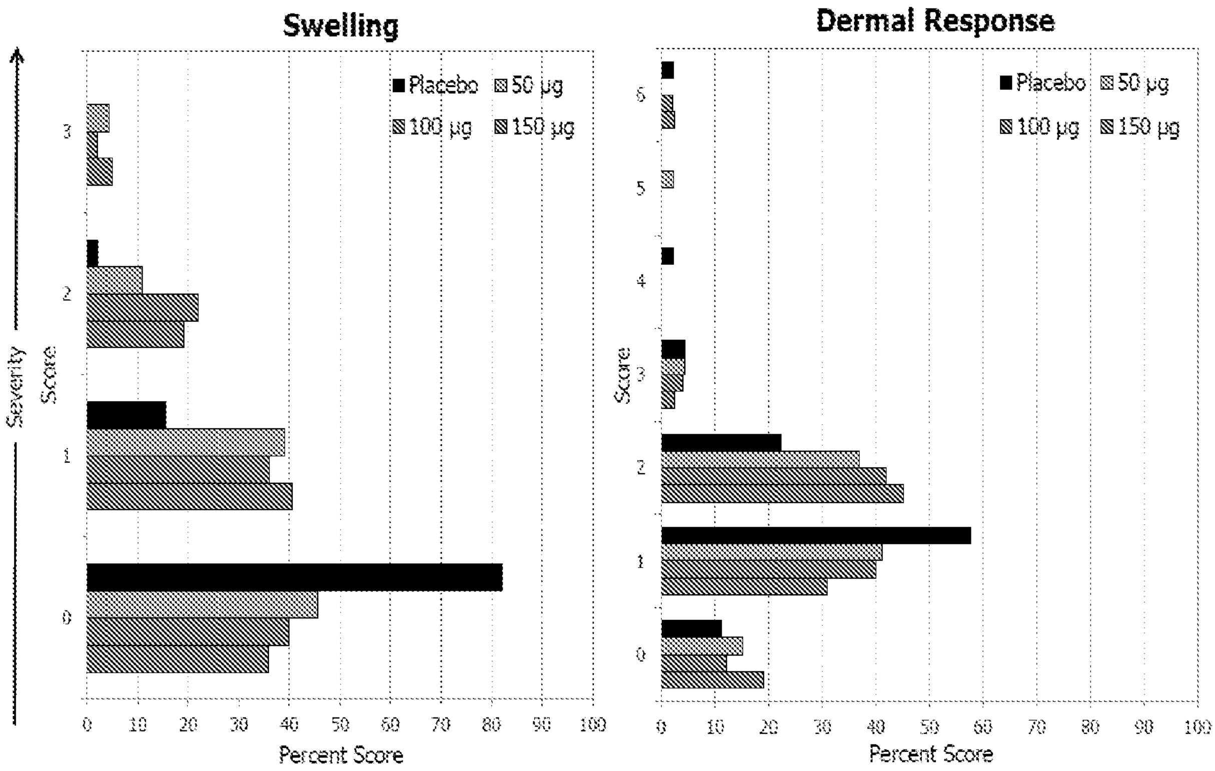

**Figure 29**

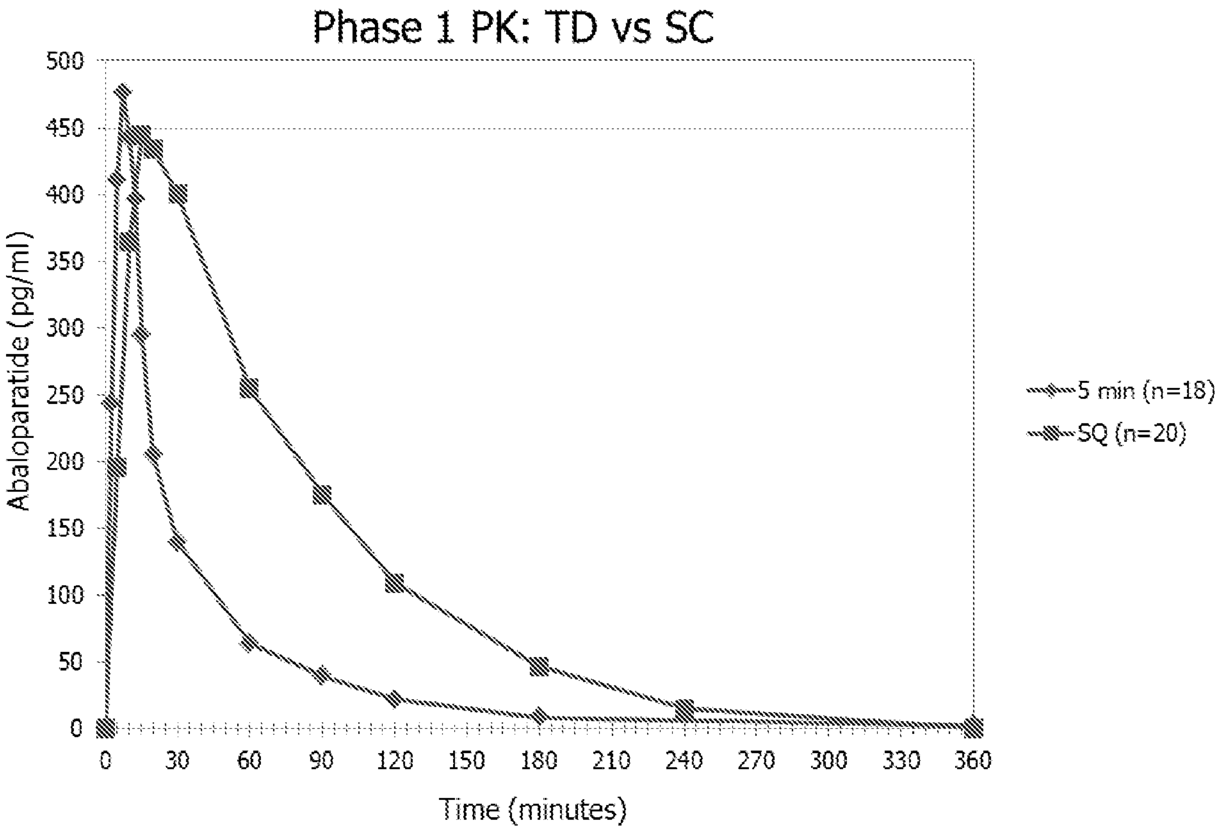
Phase 1 PK: TD vs SC
Abaloparatide (pg/ml)
500
450
400
350
300
250
200
150
100
50
0
0
30
60
90
120
150
180
210
240
270
300
330
360
Time (minutes)
5 min (n=18)
SQ (n=20)

Figure 30A

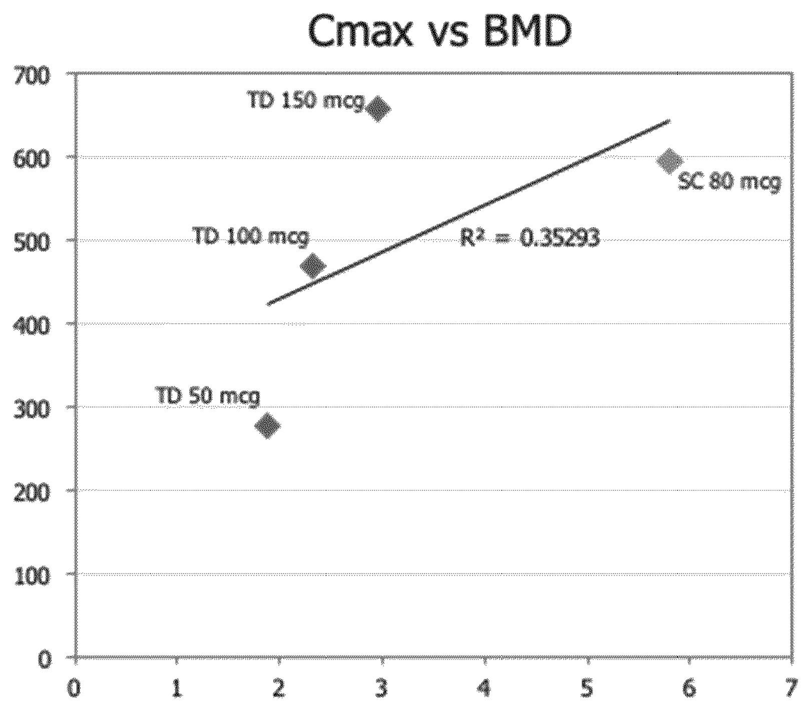


Figure 30B

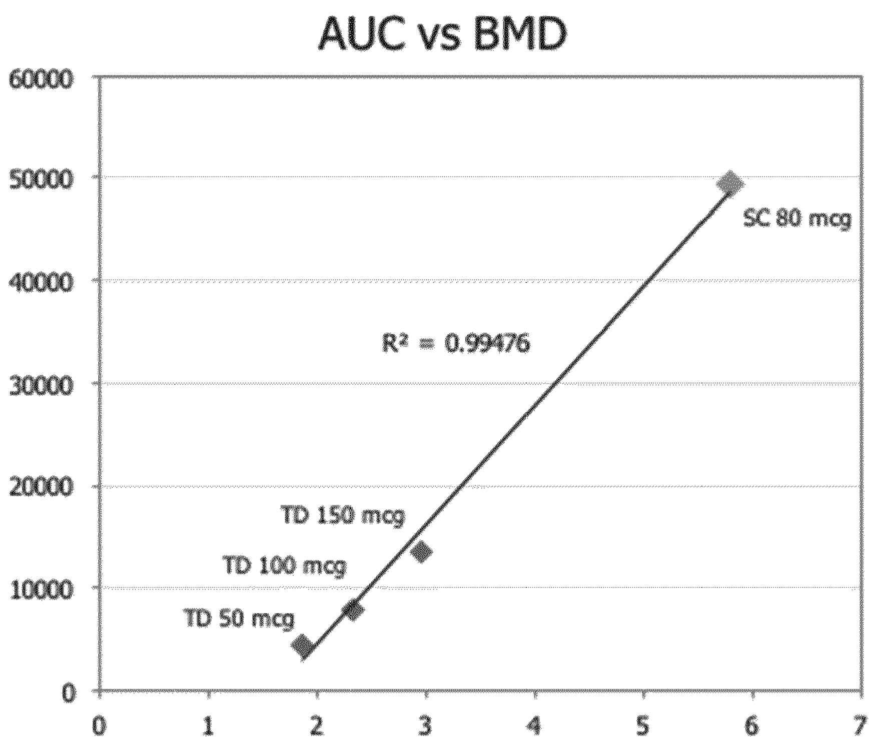

Figure 31

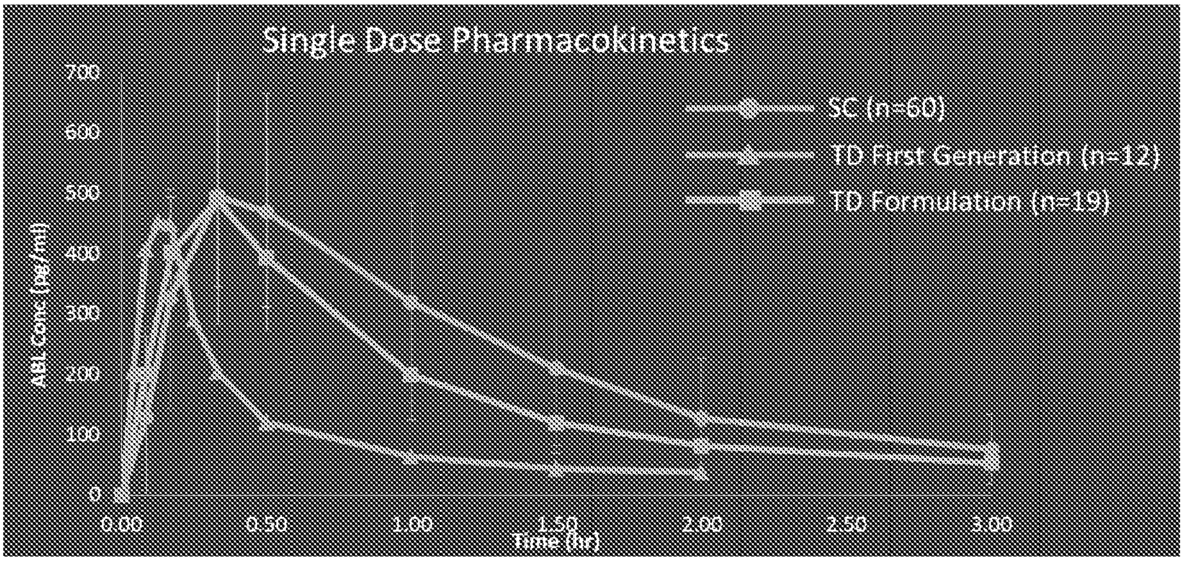
Single Dose Pharmacokinetics
SC (n=60)
TD First Generation (n=12)
TD Formulation (n=19)
ABL Conc (pg/ml)
700
600
500
400
300
200
100
0
0.00
0.50
1.00
1.50
2.00
2.50
3.00
Time (hr)

**Figure 32**

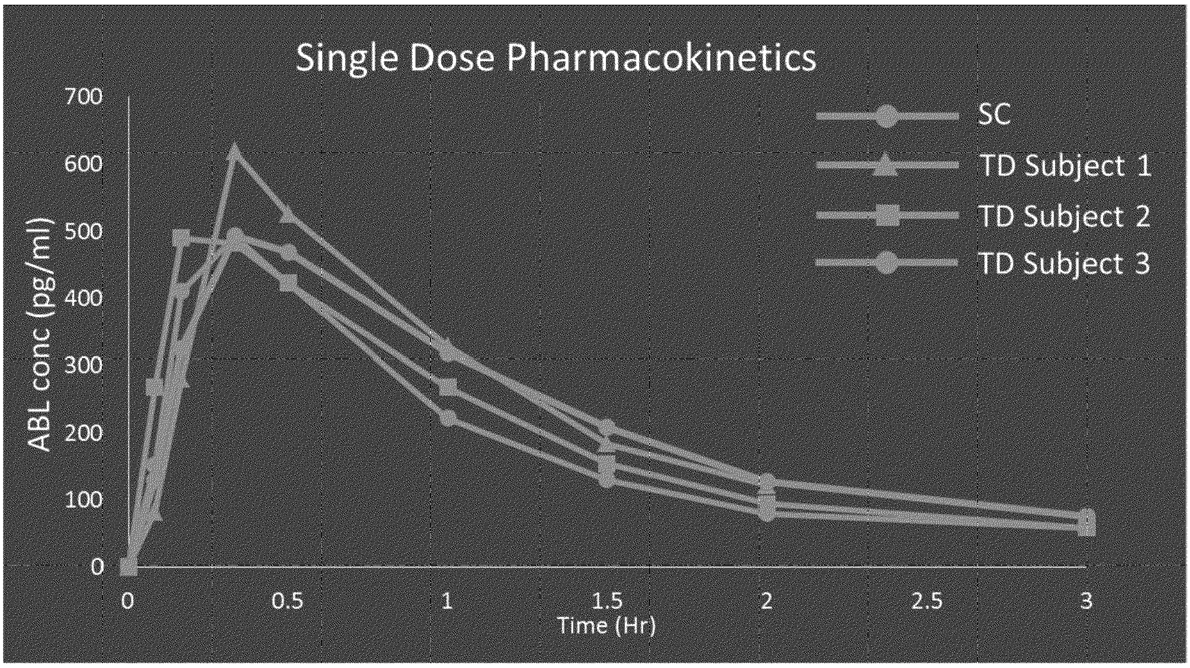


**Figure 33**

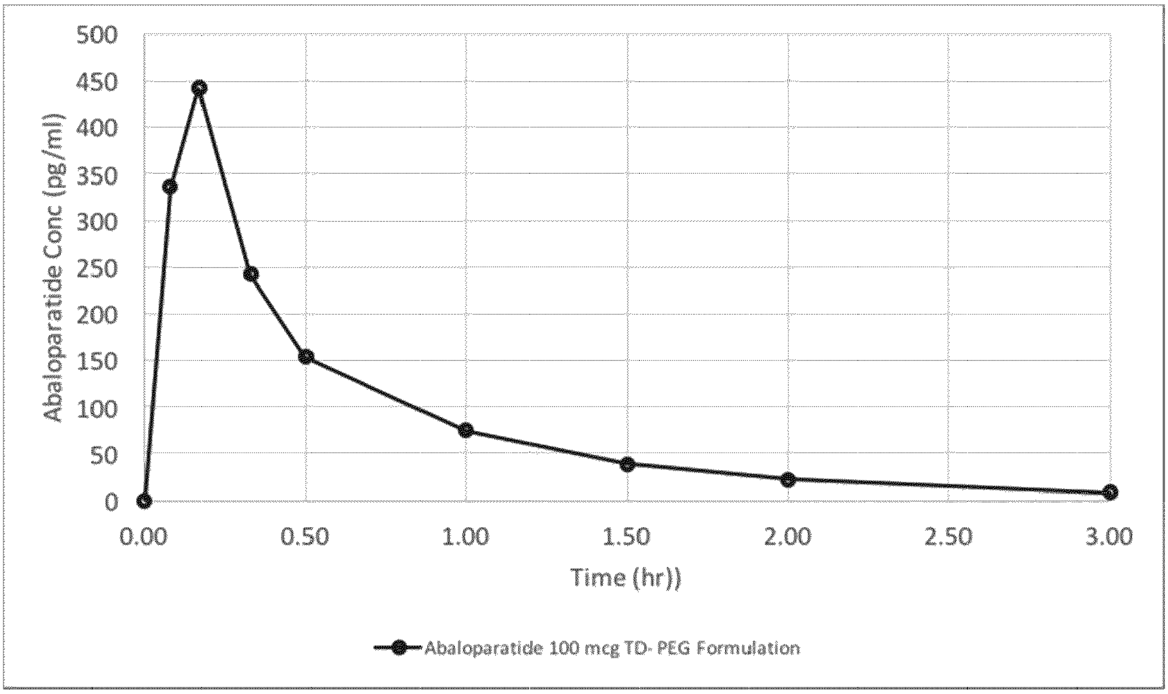

**Figure 34**

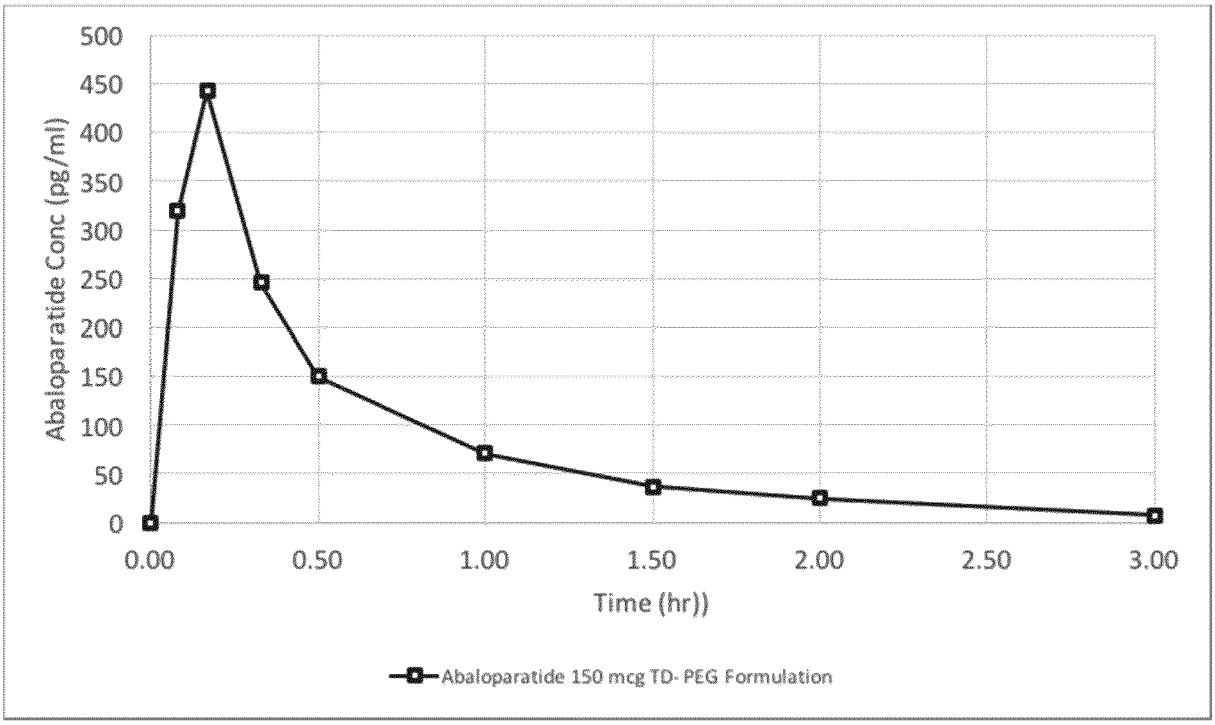


**Figure 35**

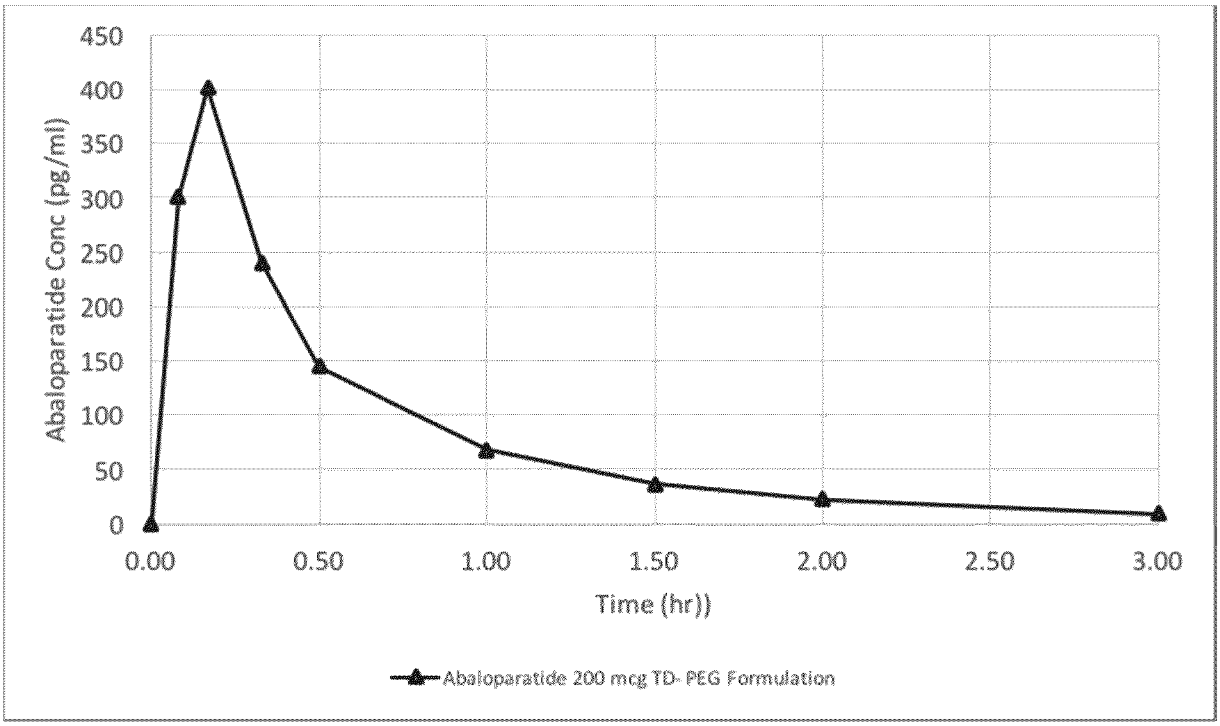

**Figure 36**

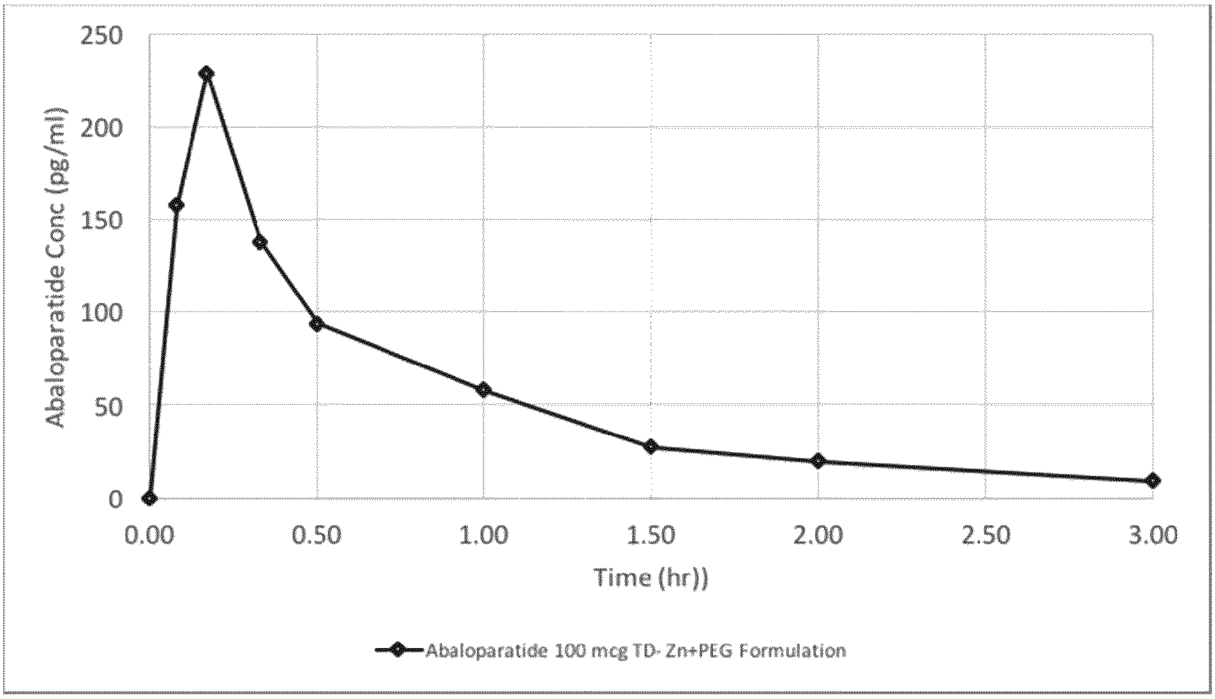


**Figure 37**

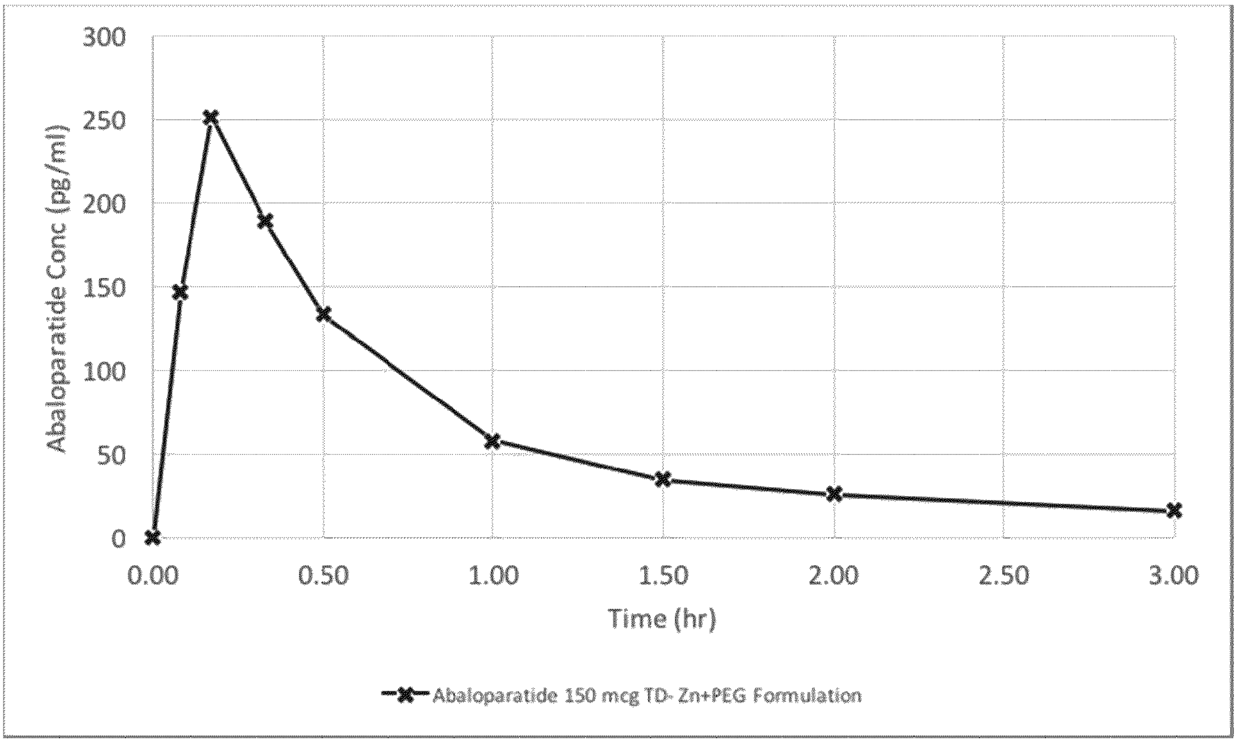

**Figure 38**

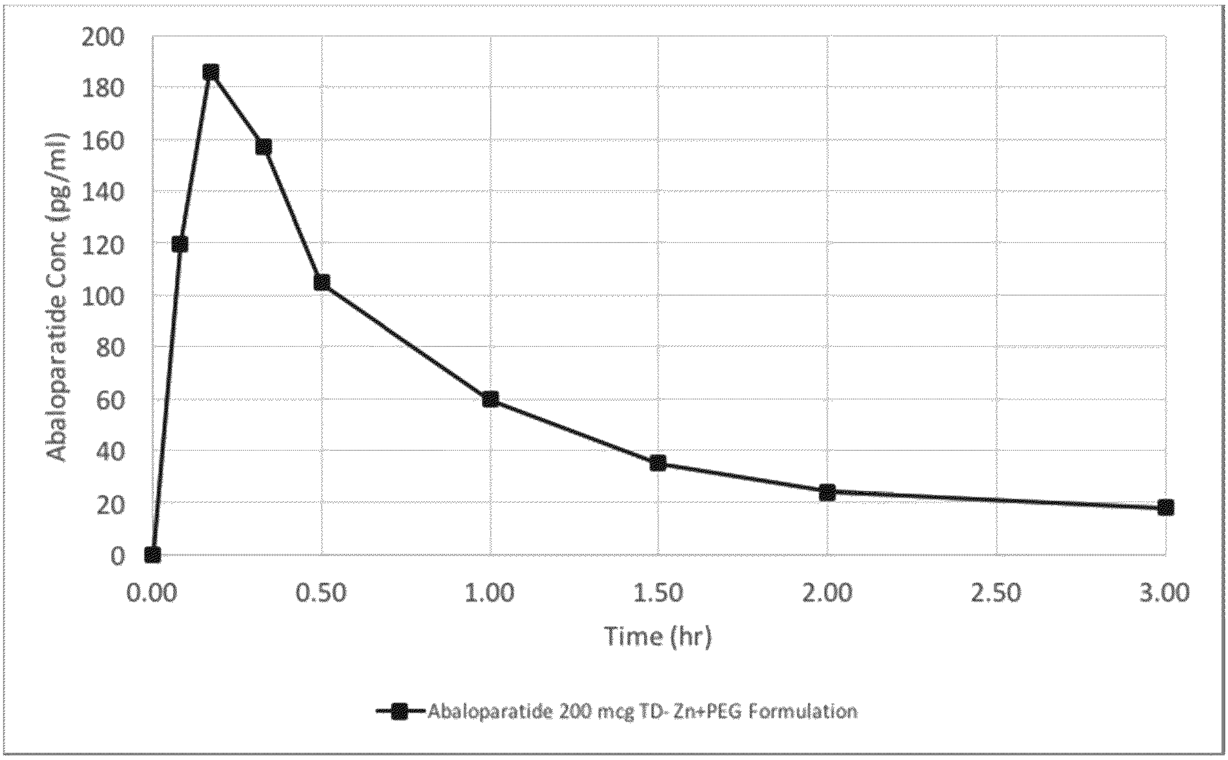
200
180
160
140
120
100
80
60
40
20
0
Abaloparatide Conc (pg/ml)
0.00
0.50
1.00
1.50
2.00
2.50
3.00
Time (hr)
Abaloparatide 200 mcg TD- Zn+PEG Formulation

Figure 39

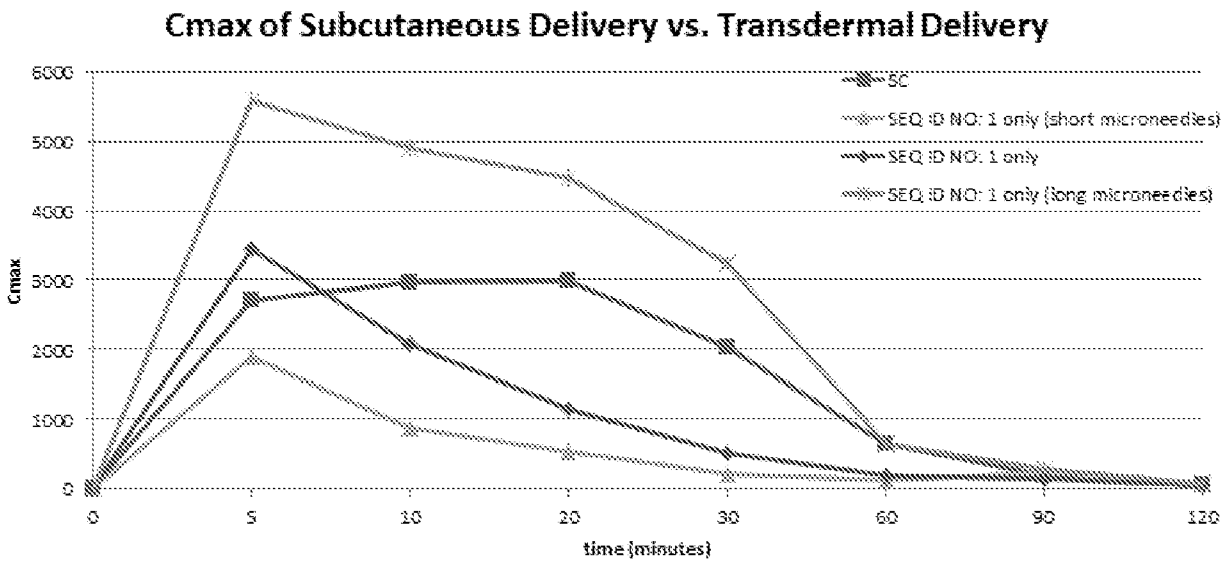
Cmax of Subcutaneous Delivery vs. Transdermal Delivery
6000
5000
4000
3000
2000
1000
0
Cmax
0
5
10
20
30
60
90
120
time (minutes)
SC
SEQ ID NO: 1 only (short microneedles)
SEQ ID NO: 1 only
SEQ ID NO: 1 only (long microneedles)

Figure 40

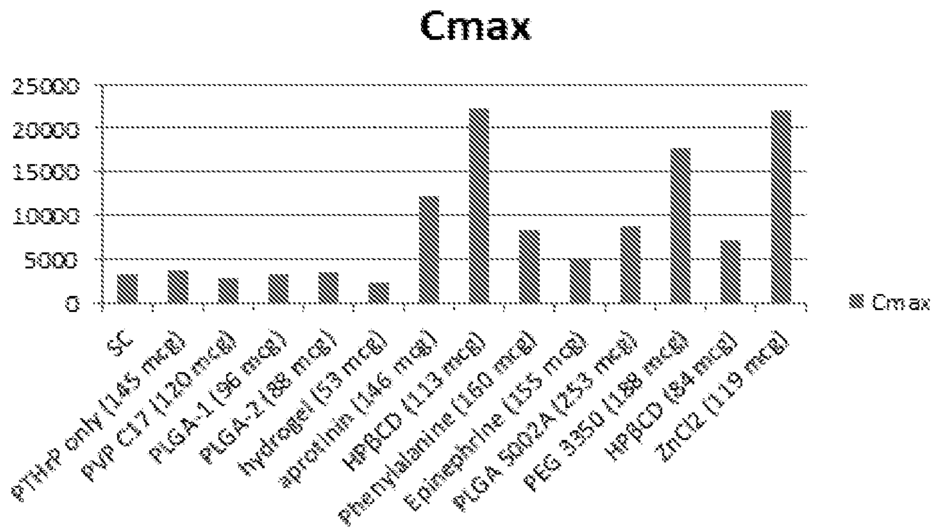


Figure 41

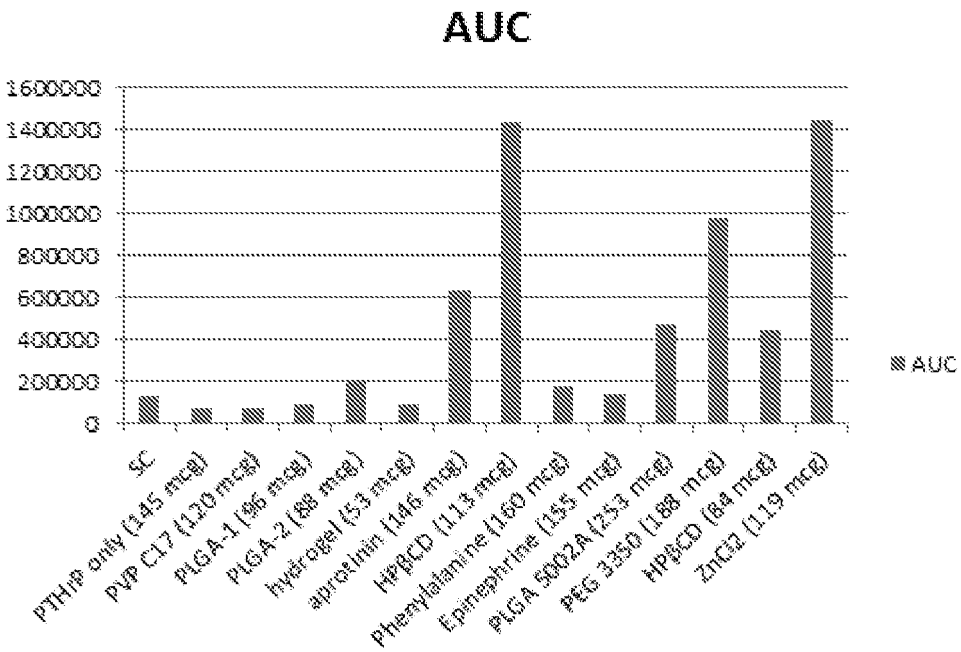

# Figure 42

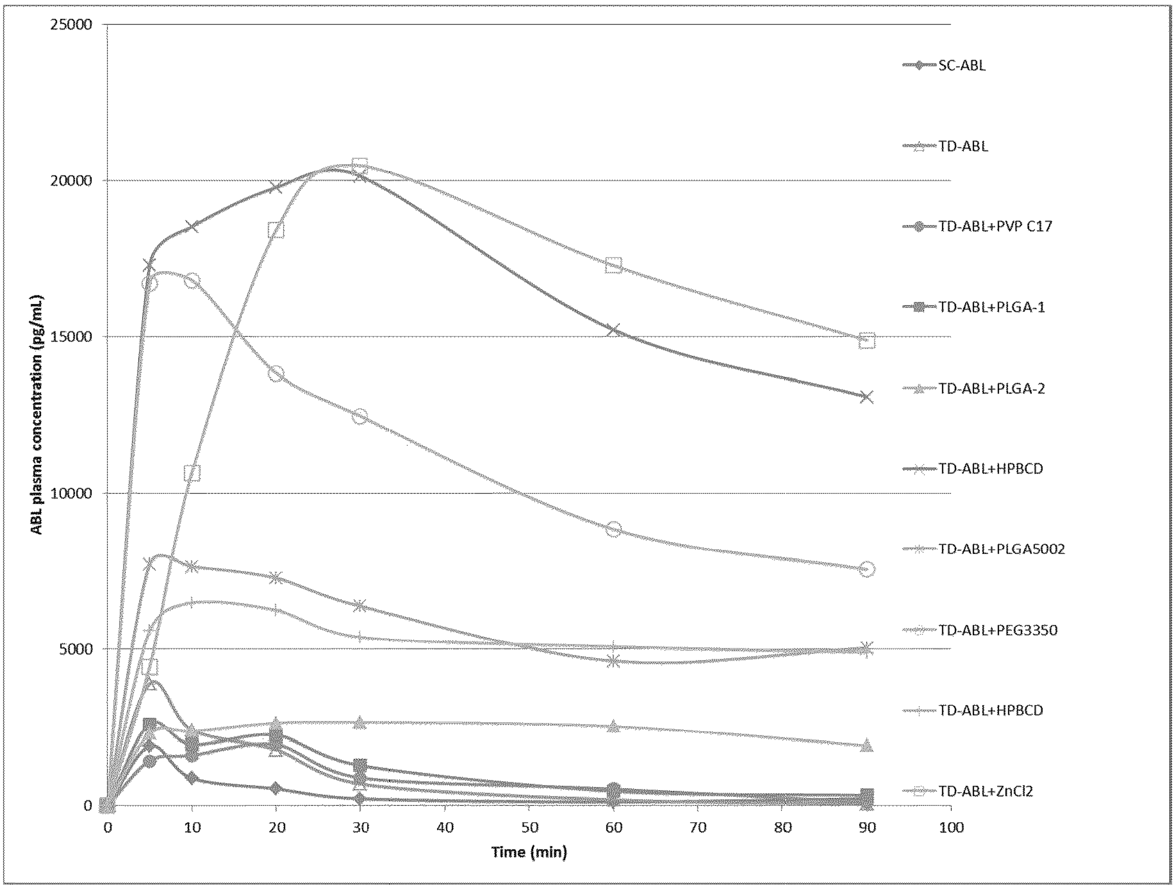

25000
20000
15000
10000
5000
0
0
10
20
30
40
50
60
70
80
90
100
ABL plasma concentration (pg/mL)
Time (min)
SC-ABL
TD-ABL
TD-ABL+PVP C17
TD-ABL+PLGA-1
TD-ABL+PLGA-2
TD-ABL+HPBCD
TD-ABL+PLGA5002
TD-ABL+PEG3350
TD-ABL+HPBCD
TD-ABL+ZnCl2

Europäisches Patentamt
European Patent Office
Office européen des brevets

# EUROPEAN SEARCH REPORT

Application Number
EP 21 16 7699

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GARY HATTERSLEY ET AL: "PP450: Transdermal delivery of BA058, a novel analog of hPTHrP (1-34), with a short wear time patch in preclinical and clinical studies", BONE ABSTRACTS, vol. 1, 1 May 2013 (2013-05-01), - 21 May 2013 (2013-05-21), pages 177-178, XP055590258, UK ISSN: 2052-1219, DOI: 10.1530/boneabs.1.PP450 Retrieved from the Internet: URL:http://www.bone-abstracts.org/ba/0001/ECTS2013AbstractBook.pdf> * abstract PP450 * ----- | 1-15 | INV. A61K9/00 A61K38/00 A61K47/02 A61K9/70 A61K33/06 A61K33/30 A61K38/29 A61P19/02 A61P19/10 A61K47/10 A61K47/40 A61P29/00 A61P43/00 C07K14/635 A61P19/00 A61K9/08 A61P19/08 |
| X | US 2013/041330 A1 (MATSUDO TOSHIYUKI [JP] ET AL) 14 February 2013 (2013-02-14) * table 8 * ----- | 1,2,5-8, 10 | |
| X | US 2011/281790 A1 (POHL RODERIKE [US] ET AL) 17 November 2011 (2011-11-17) * example 2 * ----- | 1-5 | TECHNICAL FIELDS SEARCHED (IPC) A61K |
| X | SHIRKHANZADEH M: "Microneedles coated with porous calcium phosphate ceramics: Effective vehicles for transdermal delivery of solid trehalose", JOURNAL OF MATERIALS SCIENCE: MATERIALS IN MEDICINE, KLUWER ACADEMIC PUBLISHERS, BO, vol. 16, no. 1, 1 January 2005 (2005-01-01), pages 37-45, XP019212081, ISSN: 1573-4838, DOI: 10.1007/S10856-005-6444-2 * Materials and methods * ----- -/-- | 1,4-7,9, 10 | |

1

The present search report has been drawn up for all claims

| Place of search | Date of completion of the search | Examiner |
|---|---|---|
| The Hague | 5 October 2021 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 16 7699

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/046293 A1 (HATTERSLEY GARY [US] ET AL) 13 February 2014 (2014-02-13)<br>* paragraph [0261] *<br>----- | 1-15 | |

TECHNICAL FIELDS SEARCHED (IPC)

1 The present search report has been drawn up for all claims

| Place of search | Date of completion of the search | Examiner |
|---|---|---|
| The Hague | 5 October 2021 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 21 16 7699

This annex lists the patent family members relating to the patent documents cited in the above-mentioned European search report. The members are as contained in the European Patent Office EDP file on The European Patent Office is in no way liable for these particulars which are merely given for the purpose of information.

05-10-2021

| Patent document cited in search report | Publication date | Patent family member(s) | Publication date |
|---|---|---|---|
| US 2013041330 A1 | 14-02-2013 | CA 2790923 A1 | 01-09-2011 |
| | | CN 102770176 A | 07-11-2012 |
| | | EP 2540337 A1 | 02-01-2013 |
| | | JP 5715617 B2 | 07-05-2015 |
| | | JP WO2011105508 A1 | 20-06-2013 |
| | | KR 20120138235 A | 24-12-2012 |
| | | MY 158687 A | 31-10-2016 |
| | | SG 183421 A1 | 27-09-2012 |
| | | US 2013041330 A1 | 14-02-2013 |
| | | WO 2011105508 A1 | 01-09-2011 |
| US 2011281790 A1 | 17-11-2011 | US 2011281790 A1 | 17-11-2011 |
| | | WO 2011143421 A1 | 17-11-2011 |
| US 2014046293 A1 | 13-02-2014 | AU 2012245301 B2 | 19-05-2016 |
| | | AU 2016216584 A1 | 01-09-2016 |
| | | BR 112013027153 A2 | 29-11-2016 |
| | | CA 2833571 A1 | 26-10-2012 |
| | | CN 103917242 A | 09-07-2014 |
| | | EP 2699252 A2 | 26-02-2014 |
| | | EP 3332799 A1 | 13-06-2018 |
| | | ES 2648864 T3 | 08-01-2018 |
| | | JP 6033280 B2 | 30-11-2016 |
| | | JP 2014517825 A | 24-07-2014 |
| | | JP 2017036323 A | 16-02-2017 |
| | | KR 20140047043 A | 21-04-2014 |
| | | NZ 618003 A | 27-05-2016 |
| | | RU 2013151314 A | 27-05-2015 |
| | | SG 194570 A1 | 30-12-2013 |
| | | US 2013006217 A1 | 03-01-2013 |
| | | US 2014046292 A1 | 13-02-2014 |
| | | US 2014046293 A1 | 13-02-2014 |
| | | US 2017189493 A1 | 06-07-2017 |
| | | US 2019290738 A1 | 26-09-2019 |
| | | US 2021052705 A1 | 25-02-2021 |
| | | WO 2012145665 A2 | 26-10-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62239773
- US 62239774
- US 62239801
- US 62324336
- US 62353249
- US 62396196
- US 14361787 B
- US 14361802 B
- US 13452412 B
- US 13791170 B
- US 13791360 B


### Non-patent literature cited in the description


- **AUGUSTINE et al.** Parathyroid hormone and parathyroid hormone-related protein analogs as therapies for osteoporosis. *Curr Osteoporos Rep,* 2013, vol. 11, 400-406
- **BOSTROM et al.** Parathyroid hormone-related protein analog RS-66271 is an effective therapy for impaired bone healing in rabbits on corticosteroid therapy. *Bone,* 2000, vol. 26, 437-442
- **DEAN et al.** Altered selectivity of parathyroid hormone (PTH) and PTH-related protein (PTHrP) for distinct conformations of the PTH/PTHrP receptor. *Mol Endocrinol,* 2008, vol. 22, 156-166
- **DEMPSTER et al.** Skeletal histomorphometry in subjects on teriparatide or zoledronic acid therapy (SHOTZ) study: a randomized controlled trial. *J Clin Endocrinol Metab,* 2012, vol. 97, 2799-2808
- **DHAINAUT et al.** Cortical hand bone porosity and its association with distal radius fracture in middle aged and elderly women. *PLoS One,* 2013, vol. 8, e68405
- **DOYLE et al.** BA058, a novel human PTHrP analog: reverses ovariectomy-induced bone loss and strength at the lumbar spine in aged cynomolgus monkeys. *J Bone Miner Res,* 2013, vol. 28 (1
- **DOYLE et al.** Long term effect of BA058, a hovel human PTHrP analog, restores bone mass in the aged osteopenic ovariectomized cynomolgus monkey. *J Bone Miner Res,* 2013, vol. 28 (1
- **HATTERSLEY G ; LESAGE E ; VARELA A ; MITH SY.** BA058, a Novel Human PTHrP Analog, Restores Bone Density and Increases Bone Strength At the Spine and Femur in Osteopenic Rats. *Endocr Rev.,* 2013, 34
- **HORWITZ et al.** Safety and tolerability of subcutaneous PTHrP(1-36) in healthy human volunteers: a dose escalation study. *Osteoporos Int,* 2006, vol. 17, 225-230
- **HORWITZ et al.** Parathyroid hormone-related protein for the treatment of postmenopausal osteoporosis: defining the maximal tolerable dose. *J Clin Endocrinol Metab,* 2010, vol. 95, 1279-1287
- **KRONENBERG.** PTHrP and skeletal development. *Ann N Y Acad Sci,* 2006, vol. 1068, 1-13
- **LEDER et al.** Two years of Denosumab and teriparatide administration in postmenopausal women with osteoporosis (The DATA Extension Study): a randomized controlled trial. *J Clin Endocrinol Metab,* 2014, vol. 99, 1694-1700
- **MA et al.** Comparative effects of teriparatide and strontium ranelate in the periosteum of iliac crest biopsies in postmenopausal women with osteoporosis. *Bone,* 2011, vol. 48, 972-978
- **MACLEAN et al.** Systematic review: comparative effectiveness of treatments to prevent fractures in men and women with low bone density or osteoporosis. *Ann Intern Med,* 2008, vol. 148, 197-213
- **NEER et al.** Effect of parathyroid hormone (1-34) on fractures and bone mineral density in postmenopausal women with osteoporosis. *N Engl J Med,* 2001, vol. 344, 1434-1441
- **OBAIDI et al.** Pharmacokinetics and Pharmacokinetics and pharmacodynamic of subcutaneously (SC) administered doses of BA058, a bone mass density restoring agent in healthy postmenopausal women. *AAPS Abstract W5385,* 2010
- **OEI et al.** High bone mineral density and fracture risk in type 2 diabetes as skeletal complications of inadequate glucose control: the Rotterdam Study. *Diabetes Care,* 2013, vol. 36
- **OKAZAKI et al.** Prolonged signaling at the parathyroid hormone receptor by peptide ligands targeted to a specific receptor conformation. *Proc Natl Acad Sci USA,* 2008, vol. 105, 16525-16530

- **PIOSZAK et al.** Structural basis for parathyroid hormone-related protein binding to the parathyroid hormone receptor and design of conformation-selective peptides. *J Biol Chem,* 2009, vol. 284, 28382-28391
- **RECKER et al.** Comparative effects of teriparatide and strontium ranelate on bone biopsies and biochemical markers of bone turnover in postmenopausal women with osteoporosis. *J Bone Miner Res,* 2009, vol. 24, 1358-1368